(19) Europäisches Patentamt
European Patent Office
Office européen des brevets

(11) Numéro de publication: **0 231 709 B1**

(12) # FASCICULE DE BREVET EUROPEEN

(45) Date de publication de fascicule du brevet: **22.07.92**

(51) Int. Cl.[5]: **C07D 471/04**, C07F 9/64, A61K 31/435, A61K 31/675, //(C07D471/04,221:00,221:00)

(21) Numéro de dépôt: **86402949.1**

(22) Date de dépôt: **29.12.86**

Le dossier contient des informations techniques présentées postérieurement au dépôt de la demande et ne figurant pas dans le présent fascicule.

(54) **Nouvelles phényl-naphtyridines fonctionnalisées en position-3, leur procédé de préparation, médicaments les contenant, notamment anti-ulcères.**

(30) Priorité: **06.01.86 FR 8600102**
**06.01.86 FR 8600103**
**06.01.86 FR 8600105**

(43) Date de publication de la demande:
**12.08.87 Bulletin 87/33**

(45) Mention de la délivrance du brevet:
**22.07.92 Bulletin 92/30**

(84) Etats contractants désignés:
**AT BE CH DE ES FR GB GR IT LI LU NL SE**

(56) Documents cités:
**EP-A- 0 000 490**
**EP-A- 0 018 735**
**EP-A- 0 060 012**
**EP-A- 0 172 058**
**US-A- 4 215 123**

(73) Titulaire: **LABORATOIRES UPSA**
**1 bis, rue du Docteur Camille Bru**
**F-47000 Agen(FR)**

(72) Inventeur: **Teulon, Jean-Marie**
**13, Avenue Guibert**
**F-78170 La Celle St Cloud(FR)**

(74) Mandataire: **Portal, Gérard et al**
**Cabinet Beau de Loménie 55, rue d'Amsterdam**
**F-75008 Paris(FR)**

EP 0 231 709 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne de nouvelles phénylnaphthyridines fonctionnalisées en position 3 de formule (I). Elle concerne également le procédé de préparation desdits produits et leurs applications notamment en thérapeutique.

Le document CARPIBEM EP-A-0 172 058 est un document publié le 19/02/86, postérieurement à la date de priorité revendiquée. Il décrit des composés à activité anti-ulcères qui sont proches de ceux présentement revendiqués dans le cas où $R_1$ représente l'atome d'hydrogène et $R_2$ représente un radical alkyl inférieur linéaire ou ramifié, $R_2$ étant de préférence $CH_3$.

La présente invention décrite plus loin diffère ainsi fondamentalement des phénylnaphthyridines décrites dans ce document antérieur en ce que la substitution en position 3 (groupement Z dans la formule selon la présente invention) est constituée par un groupe fonctionnel pouvant être une insaturation, une fonction alkylthio, une fonction alkylsulfinyl, une fonction éther, une fonction alcool estérifiée ou non, une fonction cétone, une fonction acide estérifié ou non, un halogène, une fonction cyanée, une fonction amine, une fonction aminoacide ou amidoacide, une fonction phosphorilée, ou un noyau aromatique ou hétéroaromatique.

Le document européen AMERICAN HOME PRODUCTS (HAP) EP-A-0 018 735 décrit des 1,8-naphthyridines ayant une activité anti-ulcère, anti-sécrétoire, et dans certains cas diurétique, et sont ainsi utiles pour le traitement d'ulcère peptique.

Ces composés ont une structure très différente de celle des composés de l'invention en comportant obligatoirement une substitution $NR_3R_4$ en position 4 de la naphthyridine, alors que selon l'invention la position 4 est non substituée, tandis que la position 3 est substituée par un groupe fonctionnalisé.

Le document EP-A-0 060 012 AMERICAN HOME PRODUCTS (AHP) est une division de EP-A-18735 et vise à couvrir des intermédiaires de synthèse. Ces composés intermédiaires ne présentent pas d'activité en eux-mêmes. Au contraire, l'existence de ces intermédiaires non actifs démontre qu'il n'y a pas de relation directe structure-activité, et que l'homme du métier doit normalement s'attendre à ce qu'un changement de structure fasse perdre l'activité.

Le document EP-A-0 000 490 MERCK prévoit des composés naphthyridinones selon lesquels l'azote est substitué par un radical alkylamino, ce qui distingue fondamentalement ces composés de ceux de l'invention.

En outre, de nombreuses possibilités de substitution sur les deux cycles accolés de la naphthyridine ne peuvent que détourner l'homme de l'art d'une structure chimique conforme à l'invention.

Le document US 4 215 123 AMERICAN HOME PRODUCTS décrit des composés obligatoirement substitués en position 4 par un radical $OR_4$, c'est-à-dire qu'il s'agit de dérivé 4-hydroxy substitué de 1,8-naphthyridine.

Les nouveaux composés selon l'invention sont choisis parmi l'ensemble constitué par les composés de formule générale (I) :

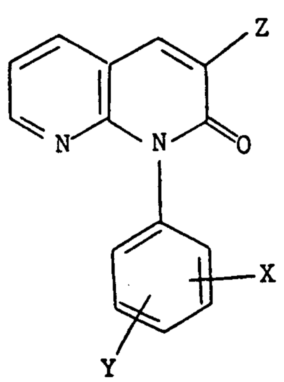

(I)

dans laquelle :

X et Y représentent l'atome d'hydrogène, un halogène, un groupement alkyle inférieur de 1 à 5 atomes de carbone, un trifluorométhyle, un alcoxy, un méthylthio, un nitro, un cyano et peuvent être en position ortho, méta, para sur le cycle aromatique préférentiellement en position méta ou para,

Z est un groupement fonctionnel choisi parmi :

a) un propényle ou propynyle,

b) une chaîne $-(CH_2)_n-S-(CH_2)_m-R$ ou $-(CH_2)_n-SO-(CH_2)_m-R$, dans laquelle n et m sont identiques ou différents et sont des nombres compris entre 0 et 5, et R est un méthyle, ou une fonction du type

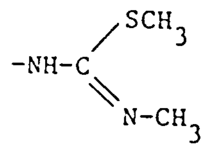 ,

du type

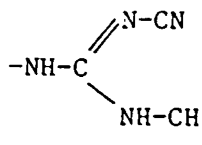

ou du type

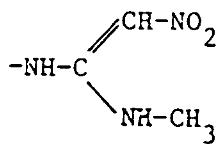 ,

c) une chaîne $-(CH_2)_n-O-(CH_2)_m-CH_3$ (n et m étant définis comme ci-dessus)

d) une chaîne $-(CH_2)_n-OH$ ou un de ses esters choisis parmi le groupe consistant d'un acétate, propionate, nicotinate, furanne-2-carboxylate, et phényl acétate, n étant défini comme ci-dessus ;

e) une chaîne $-(CH_2)_n-CHOH-(CH_2)_m-R'$ ou un de ses esters choisis parmi le groupe consistant d'un acétate, propionate, nicotinate, furanne-2-carboxylate, et phényl acétate, n et m étant définis comme ci-dessus, R' étant un méthyle ou un groupe phényle ou pyridyle;

f) une chaîne $-(CH_2)_n-CO-(CH_2)_m-CH_3$ (n et m étant définis comme ci-dessus) ;

g) une chaîne $-(CH_2)_n-COOH$ (n étant défini comme ci-dessus), principalement n étant au moins égal à 3 et optimalement n = 4 ;

h) une chaîne $-(CH_2)_n-Hal$ (n étant défini comme ci-dessus et Hal étant un halogène) ;

i) une chaîne $-(CH_2)_n-CN$ (n étant défini comme ci-dessus) ;

j) une chaîne

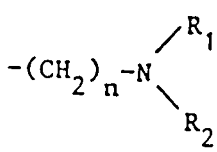

n étant défini comme ci-dessus, et $NR_1R_2$ forme un cycle pypérazine substitué par un groupe méthyle, un groupe phényle ou un groupe phényle substitué par un méthoxy ou alternativement $NR_1R_2$ forme un cycle imidazole ;

k) une chaîne

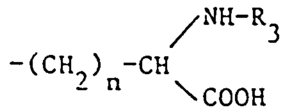

n étant défini comme ci-dessus,
$R_3$ étant un hydrogène ou un groupe para-chlorobenzoyl ;
l) une chaîne

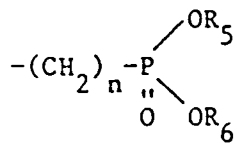

n étant défini comme ci-dessus,
$R_5$ et $R_6$ étant un groupe éthyle, et
m) un noyau phényle, pyridyle, thiophène ou furanne, ainsi qu'éventuellement les sels d'addition d'acide non toxiques.

Les composés de formule (I) selon l'invention peuvent être synthétisés par des méthodes classiques de réaction de condensation de Stobbe, Perkin, Claisen ou Knoevenagel sur un aldéhyde de formule (II) :

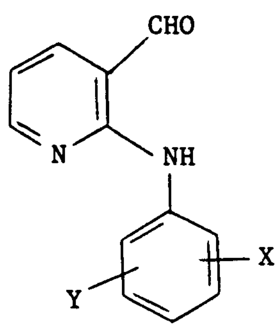

(II)

dans la formule (II), X et Y sont définis comme ci-dessus.

D'une façon générale, les aldéhydes de formule (II) peuvent être obtenus par oxydation, à l'aide d'un oxydant doux tel que par exemple $MnO_2$, dans un solvant organique comme le dichlorométhane ou le chloroforme, à une température comprise entre 20 et 50 ° C d'un alcool de formule (III) :

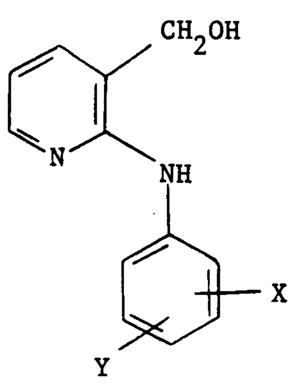

(III)

dans la formule (III), X et Y sont définis comme ci-dessus.

Les alcools de formule (III) sont obtenus par réduction à l'aide d'un réducteur classique tel que par

exemple l'hydrure double d'aluminium et de lithium dans un solvant organique tel que par exemple le tétrahydrofuranne ou l'éther éthylique d'un acide ou d'un de ses esters de formule (IV) ; dans le cas où le noyau phényle sera porteur d'une substitution sensible à certains réducteurs tels que par exemple nitro ou cyano, on choisira la réduction de l'ester par un réducteur respectant cette substitution par exemple le borohydrure de lithium préparé "in situ" à partir du borohydrure de potassium et du chlorure de lithium :

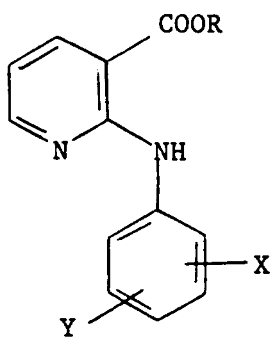

(IV)

dans la formule (IV), X et Y sont définis comme ci-dessus, R est l'atome d'hydrogène ou un alkyle.

Les procédés pour la synthèse des produits de formule (I) consistent donc à faire réagir les aldéhydes de formule (II) avec :

- des anhydrides d'acides de formule

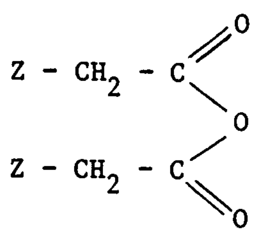

Z étant défini comme ci-dessus en présence du sel de sodium de l'acide Z-$CH_2$-COOH

- des anhydrides d'acides de formule

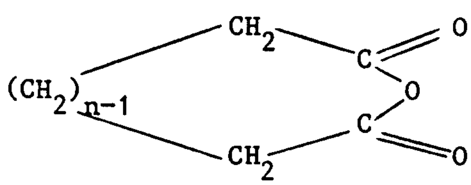

ou son chlorure

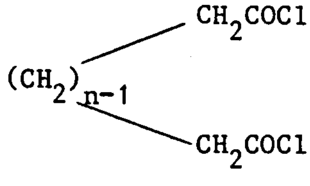

en présence du sel de sodium de l'acide correspondant, n étant défini comme ci-dessus,

- des esters d'acides de formule

Z - $CH_2$ - COOR"

Z étant défini comme ci-dessus, R" étant un alkyle, en présence d'alcoolate de sodium ou de potassium ou d'hydrure de sodium ou de sodium métal,

- des lactones de formule :

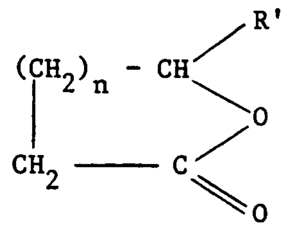

n étant défini comme ci-dessus, R' étant un méthyle ou un noyau aromatique ou hétéroaromatique en présence d'alcoolate de sodium ou de potassium ou d'hydrure de sodium ou de sodium métal.

Ces réactions peuvent être réalisées sans solvant ou avantageusement dans un solvant organique tel qu'un alcool, le benzène, le toluène ou la N-méthylpyrrolidone à une température comprise entre 20 et 200 ° C environ.

Les dérivés S → O s'obtiennent par oxydation de composés -S- à l'aide par exemple d'un peracide, l'acide métachloroperbenzoïque pouvant être utilisé dans un solvant tel que le chloroforme ou le dichlorométhane.

Les dérivés ester d'alcool seront obtenus de façon classique par estérification des alcools correspondants par un chlorure d'acide ou un anhydride d'acide.

Les dérivés cétoniques sont synthétisés par oxydation des alcools secondaires à l'aide d'oxydants connus tels que le réactif de Jones ou le réactif de Sarett dans un solvant organique tel que l'acétone, le chloroforme ou le dichlorométhane.

Les dérivés halogénés sont préparés par action du chlorure de thionyle ou de l'acide bromhydrique sur les alcools primaires correspondants, ces dérivés peuvent être utilisés en particulier pour la synthèse des dérivés aminés par action de l'amine correspondante, imidazolyle par action de l'imiadazole métallé, pour préparer certains dérivés cyanés, alcoxy ou alkylthio par action du sel de sodium ou de potassium du composé correspondant, pour la préparation de certains amino et amidoacides par synthèse malonique en condensant l'acétamido malonate de dialkyle préalablement métallé ou encore par la synthèse de composés phosphorés par action de trialkylphosphites.

Des sels d'addition de certains composés de formule (I) peuvent s'obtenir par réaction de ces composés avec un acide minéral ou organique suivant une méthode connue en soi. Parmi les acides utilisables à cet effet, on citera les acides chlorhydrique, bromhydrique, sulfurique, phosphorique, 4-toluènesulfonique, méthanesulfonique, cyclohexylsulfonique, oxalique, succinique, formique, fumarique, maléique, citrique, aspartique, cinnamique, lactique, glutamique, N-acétylaspartique, N-acétylglutamique, ascorbique, malique, benzoïque et acétique.

Selon l'invention, on propose des compositions thérapeutiques utiles notamment pour le traitement des ulcères gastro-intestinaux caractérisées en ce qu'elles renferment, en association avec un excipient physiologiquement acceptable, au moins un composé de formule (I).

D'autres caractéristiques et avantages de l'invention seront mieux compris à la lecture qui va suivre de quelques exemples de préparation nullement limitatifs, mais donnés à titre d'illustration.

Le tableau I ci-après donne la formule développée de certains produits.

Exemple 1

(trifluorométhyl-3 phényl)amino-2 hydroxyméthyl-3 pyridine

Formule III X = 3-$CF_3$ ; Y = H

A une suspension de 52 g d'hydrure double d'aluminium et de lithium dans 1 000 ml d'éther éthylique anhydre, on ajoute goutte à goutte une solution de 200 g d'acide (trifluorométhyl-3 phényl)amino-2 nicotinique dans 500 ml de tétrahydrofuranne anhydre. Après la fin de l'addition, le mélange réactionnel est porté 3 h au reflux. Après refroidissement, l'excès d'hydrure double est détruit par addition d'acétate d'éthyle, puis d'une solution aqueuse saturée de sulfate de sodium. Le précipité formé est essoré et lavé à l'éther. Les filtrats rassemblés sont évaporés sous vide et on récupère 185,2 g de (trifluorométhyl-3 phényl)-

amino-2 hydroxyméthyl-3 pyridine sous forme de cristaux de point de fusion 103-105 ° C.

Selon cette méthode sont préparés les dérivés suivants :

- phénylamino-2 hydroxyméthyl-3 pyridine

  Formule III : X = Y = H

  Huile ; rendement 95 %
- (fluoro-4 phényl)amino-2 hydroxyméthyl-3 pyridine

  Formule III : X = 4-F ; Y = H

  Cristaux F = 89-90 ° C ; rendement 88 %
- (méthylthio-3 phényl)amino-2 hydroxyméthyl-3 pyridine

  Formule III : X = 3-$SCH_3$ ; Y = H

  Huile ; rendement 95 %
- (difluoro-2,5 phényl)amino-2 hydroxyméthyl-3 pyridine

  Formule III : X = 2-F ; Y = 5-F

  Cristaux F = 71-4 ° C ; rendement 98 %
- (méthoxy-3 phényl)amino-2 hydroxyméthyl-3 pyridine

  Formule III : X = 3-$OCH_3$ ; Y = H

  Cristaux F = 94-95 ° C ; rendement 98 %
- (chloro-3 phényl)amino-2 hydroxyméthyl-3 pyridine

  Formule III : X = 3-Cl ; Y = H

  Cristaux F = 114-115 ° C ; rendement 94 %
- (chloro-4 phényl)amino-2 hydroxyméthyl-3 pyridine

  Formule III : X = 4-Cl ; Y = H

  Cristaux F = 124-126 ° C ; rendement 95 %

<u>Exemple 2</u>

(cyano-3 phényl)amino-2 hydroxyméthyl-3 pyridine

Formule III X = 3-CN ; Y = H

A une solution de 39,3 g de (cyano-3 phényl)amino-2 nicotinate de méthyle dans 600 ml de tétrahydrofuranne contenant 10 g de borohydrure de potassium, on ajoute par petites quantités, sous agitation, 8 g de chlorure de lithium. Après la fin de l'addition, le mélange est porté au reflux durant 4 h puis concentré sous vide. Après addition d'eau et de glace au résidu obtenu, on extrait à l'éther et la phase éthérée est lavée à l'eau puis séchée sur sulfate de sodium.

Après évaporation de l'éther, on obtient 31,6 g de (cyano-3 phényl)amino-2 hydroxyméthyl-3 pyridine sous forme de cristaux de point de fusion 126 ° C.

Selon ce procédé, sont préparés les dérivés suivants :

- (nitro-3 phényl)amino-2 hydroxyméthyl-3 pyridine

  Formule III : X = 3-$NO_2$ ; Y = H

  Cristaux F = 150-155 ° C ; rendement 84 %
- (cyano-3 chloro-4 phényl)amino-2 hydroxyméthyl-3 pyridine

Formule III : X = 3-CN ; Y = 4-Cl

Cristaux F = 147 ° C ; rendement 90 %

- (cyano-3 fluoro-4 phényl)amino-2 hydroxyméthyl-3 pyridine

Formule III : X = 3-CN ; Y = 4-F

Cristaux F = 126 ° C ; rendement 90 %

Exemple 3

(trifluorométhyl-3 phényl)amino-2 nicotinaldéhyde

Formule II X = 3-$CF_3$ ; Y = H

A une solution de 185 g de (trifluorométhyl-3 phényl)amino-2 hydroxyméthyl-3 pyridine, préparés à l'exemple 1 dans 2 300 ml de chloroforme, on ajoute par petites fractions 690 g de $MnO_2$. Après la fin de l'addition, on agite à température ambiante durant 6 h. Le milieu réactionnel est ensuite filtré sur Celite et le filtrat est évaporé à sec. Les cristaux obtenus alors, 175 g, sont recristallisés dans l'heptane. On récupère ainsi 160 g de (trifluorométhyl-3 phényl)amino-2 nicotinaldéhyde sous forme de cristaux de point de fusion 80-81 ° C.

Selon cette méthode, sont préparés les dérivés suivants :

- phénylamino-2 nicotinaldéhyde

Formule II : X = Y = H

Cristaux (éther isopropylique) F = 77-78 ° C ; rendement 80 %

- (cyano-3 phényl)amino-2 nicotinaldéhyde

Formule II : X = 3-CN ; Y = H

Cristaux (acétonitrile) F = 153-154 ° C ; rendement 60 %

- (fluoro-4 phényl)amino-2 nicotinaldéhyde

Formule II : X = 4-F ; Y = H

Cristaux F = 67-68 ° C ; rendement 71 %

- (méthylthio-3 phényl)amino-2 nicotinaldéhyde

Formule II : X = 3-$SCH_3$ ; Y = H

Cristaux F = 63-64 ° C ; rendement 70 %

- (difluoro-2,5 phényl)amino-2 nicotinaldéhyde

Formule II : X = 2-F ; Y = 5-F

Cristaux (isopropanol) F = 129-130 ° C ; rendement 76 %

- (méthoxy-3 phényl)amino-2 nicotinaldéhyde

Formule II : X = 3-$OCH_3$ ; Y = H

Cristaux (éther isopropylique) F - 65-66 ° C ; rendement 75 %

- (chloro-3 phényl)amino-2 nicotinaldéhyde

Formule II : X = 3-Cl ; Y = H

Cristaux F = 99-100 ° C ; rendement 78 %

- (nitro-3 phényl)amino-2 nicotinaldéhyde

Formule II : X = 3-$NO_2$ ; Y = H

Cristaux (acétonitrile) F = 160-162 °C ; rendement 70 %

- (cyano-3 chloro-4 phényl)amino-2 nicotinaldéhyde

Formule II : X = 3-CN ; Y = 4-Cl

Cristaux (acétonitrile) F = 203 °C ; rendement 60 %

- (cyano-3 fluoro-4 phényl)amino-2 nicotinaldéhyde

Formule II : X = 3-CN ; Y = 4-F

Cristaux (acétonitrile) F = 193 °C ; rendement 80 %

- (chloro-4 phényl)amino-2 nicotinaldéhyde

Formule II : X = 4-Cl ; Y = H

Cristaux (acétate d'isopropyle) F = 101-102 °C ; rendement 60 %

Exemple 4

[(trifluorométhyl-3 phényl)-1 dihydro-1,2 oxo-2 naphtyridine-1,8 yl-3]propène-1,2 et -2,3

Formule I : X = 3-$CF_3$, Y = H, Z = propène-1,2 et propène-2,3

On porte à 170-180 °C durant 1 h 15 min un mélange de 31 g de (trifluorométhyl-3 phényl)amino-2 nicotinaldéhyde préparés à l'exemple 3, 14 g du sel de sodium de l'acide 4-penténoïque et de 31 g de l'anhydride de l'acide 4-penténoïque dans 40 ml de N-méthylpyrrolidone.

Le mélange réactionnel est ensuite refroidi, additionné d'eau et de glace et extrait au dichlorométhane.

La phase organique est lavée à l'eau, avec une solution de soude 10 %, puis encore à l'eau et séchée sur sulfate de sodium. Le dichlorométhane évaporé, le résidu obtenu est filtré sur gel de silice. A l'éluant éther isopropylique, on récupère des cristaux qui, recristallisés dans l'isopropanol, donnent 8 g d'un mélange constitué par les [(trifluorométhyl-3 phényl)-1 dihydro-1,2 oxo-2 naphtyridine-1,8 yl-3]propène-1,2 et -2,3 sous forme de cristaux de point de fusion 151-154 °C.

Exemple 5

(trifluorométhyl-3 phényl)-1 dihydro-1,2 oxo-2 naphtyridine-1,8 méthylthio-3

Formule I X = 3-$CF_3$, Y = H, Z = $SCH_3$

On porte à 110-120 °C durant 1 h 15 min un mélange de 9,7 g de (trifluorométhyl-3 phényl)amino-2 nicotinaldéhyde préparés à l'exemple 3, 6,5 g de méthylthioacétate de sodium et de 14,5 g d'anhydride méthylthioacétique dans 10 ml de N-méthylpyrrolidone.

Le mélange réactionnel est ensuite refroidi, additionné d'eau et de glace et extrait au dichlorométhane. La phase organique est lavée à l'eau, avec une solution de soude 10 %, puis encore à l'eau et séchée sur sulfate de sodium. Le dichlorométhane évaporé, on obtient un résidu qui cristallise dans l'éther. Après recristallisation dans l'éthanol, on obtient 2,6 g de (trifluorométhyl-3 phényl)-1 dihydro-1,2 oxo-2 naphtyridine-1,8 méthylthio-3 sous forme de cristaux de point de fusion 202-203 °C.

Exemple 6

(trifluorométhyl-3 phényl)-1 dihydro-1,2 oxo-2 naphtyridine-1,8 méthylthiométhyl-3

Formule I X = 3-$CF_3$, Y = H, Z = $CH_2$-S-$CH_3$

Préparé selon le mode opératoire de l'exemple 5

Cristaux (isopropanol) F = 144-147 °C ; rendement 20 %

Exemple 7

[(trifluorométhyl-3 phényl)-1 dihydro-1,2 oxo-2 naphtyridine-1,8 yl-3] éthanol-2

Formule I X = 3-$CF_3$, Y = H, Z = $CH_2$-$CH_2$-OH

Une solution de 30 g de (trifluorométhyl-3 phényl)amino-2 nicotinaldéhyde préparés à l'exemple 3 et de 30 g de butyrolactone dans 150 ml de benzène est ajoutée goutte à goutte à une suspension de 8,2 g d'hydrure de sodium dans 150 ml de benzène. La réaction est amorcée avec quelques gouttes d'éthanol puis agitée durant 3 h. Après addition d'eau, on ajoute du chloroforme et la phase organique séparée est lavée à l'eau puis séchée sur sulfate de sodium. Le solvant est évaporé et le résidu obtenu cristallise dans un mélange d'éther isopropylique et d'acétone. Les cristaux obtenus sont essorés, lavés à l'éther et séchés. Les 24 g obtenus alors sont recristallisés dans l'acétonitrile. On obtient ainsi 17 g de [(trifluorométhyl-3 phényl)-1 dihydro-1,2 oxo-2 naphtyridine-1,8 yl-3] éthanol-2 sous forme de cristaux de point de fusion 179-180 ° C.

Exemple 8
(trifluorométhyl-3 phényl)-1 dihydro-1,2 oxo-2 naphtyridine-1,8 méthylsulfinyl-3

Formule I X = 3-$CF_3$, Y = H, Z = $SOCH_3$

A une solution de 5 g de (trifluorométhyl-3 phényl)-1 dihydro-1,2 oxo-2 naphtyridine-1,8 méthylthio-3 préparés à l'exemple 5 dans 500 ml de chloroforme refroidi à 0 ° C, on ajoute par petites fractions 2,8 g d'acide métachloroperbenzoïque 80-90 % sous bonne agitation. On laisse ensuite remonter à température ambiante puis on laisse au contact une nuit.

Le phase chloroformique est ensuite lavée à l'eau, avec une solution d'ammoniaque puis encore à l'eau et sechée sur sulfate de sodium. Après évaporation du solvant, les cristaux obtenus sont recristallisés dans l'isopropanol conduisant à 3,3 g de (trifluorométhyl-3 phényl)-1 dihydro-1,2 oxo-2 naphtyridine-1,8 méthylsulfinyl-3 sous forme de cristaux de point de fusion 227-228 ° C.

Exemple 9
(trifluorométhyl-3 phényl)-1 dihydro-1,2 oxo-2 naphtyridine-1,8 méthylsufinylméthyl-3

Formule I X = 3-$CF_3$, Y = H, Z = $CH_2$-SO-$CH_3$

Préparé selon le mode opératoire de l'exemple 8
Cristaux (isopropanol) F = 194-195 ° C ; rendement 66 %

Exemple 10
[(trifluorométhyl-3 phényl)-1 dihydro-1,2 oxo-2 naphtyridine-1,8 yl-3] propanol-3

Formule I X = 3-$CF_3$, Y = H, Z = $CH_2$-$CH_2$-$CH_2$-OH

Préparé selon le mode opératoire de l'exemple 7 avec la $\delta$-valérolactone
Cristaux ($CCl_4$) F = 121-122 ° C ; rendement 50 %

Exemple 11
Acétate de [(trifluorométhyl-3 phényl)-1 dihydro-1,2 oxo-2-naphtyridine-1,8 yl-3] propanol-3

Formule I X = 3-$CF_3$, Y = H, Z = $CH_2$-$CH_2$-$CH_2$-O-CO-$CH_3$

On porte au reflux durant 3 h une solution de 7 g de [(trifluorométhyl-3 phényl)-1 dihydro-1,2 oxo-2 naphtyridine-1,8 yl-3] propanol-3 préparés à l'exemple 10 dans 20 ml d'anhydride acétique. Après évaporation de l'anhydride acétique, le résidu obtenu cristallisé est soigneusement lavé au pentane puis séché. On obtient ainsi 7 g d'acétate de [(trifluorométhyl-3 phényl)-1 dihydro-1,2 oxo-2 naphtyridine-1,8 yl-3] propanol-3 sous forme de cristaux de point de fusion 93-95 ° C.

Exemple 12
(phényl-1 dihydro-1,2 oxo-2 naphtyridine-1,8 yl-3) propanol-3

Formule I X = Y = H, Z = $CH_2$-$CH_2$-$CH_2$-OH

Préparé selon le mode opératoire de l'exemple 10
Cristaux (toluène-isopropanol) F = 166-168° C ; rendement 50 %

Exemple 13
Acétate de (phényl-1 dihydro-1,2 oxo-2 naphtyridine-1,8 yl-3) propanol-3

Formule I  X = Y = H, Z = $CH_2$-$CH_2$-$CH_2$-O-CO-$CH_3$

Préparé selon le mode opératoire de l'exemple 11
Cristaux F = 156-158° C ; rendement 93 %

Exemple 14
(phényl-1 dihydro-1,2 oxo-2 naphtyridine-1,8 yl-3) éthanol-2

Formule I  X = Y = H, Z = $CH_2$-$CH_2$-OH

Préparé selon le mode opératoire de l'exemple 7
Cristaux (éthanol) F = 216-217° C ; rendement 51 %

Exemple 15
Acétate de (phényl-1 dihydro-1,2 oxo-2 naphtyridine-1,8 yl-3) éthanol-2

Formule I  X = Y = H, Z = $CH_2$-$CH_2$-O-CO-$CH_3$

Préparé selon le mode opératoire de l'exemple 11
Cristaux F = 190-191° C ; rendement 95 %

Exemple 16
Acétate de [(trifluorométhyl-3 phényl)-1 dihydro-1,2 oxo-2 naphtyridine-1,8 yl-3] éthanol-2

Formule I  X = 3-$CF_3$, Y = H, Z = $CH_2$-$CH_2$-O-CO-$CH_3$

Préparé selon le mode opératoire de l'exemple 11
Cristaux F = 100-102° C ; rendement 93 %

Exemple 17
(phényl-1 dihydro-1,2 oxo-2 naphtyridine-1,8 yl-3) butanol-4

Formule I  X = Y = H, Z = $CH_2$-$CH_2$-$CH_2$-$CH_2$-OH

Préparé selon le mode opératoire de l'exemple 7 avec la $\epsilon$-caprolactone
Cristaux (acétonitrile) F = 176-177° C ; rendement 42 %

Exemple 18
[(trifluorométhyl-3 phényl)-1 dihydro-1,2 oxo-2 naphtyridine-1,8 yl-3] pyridyl-3

Formule I  X = 3-$CF_3$, Y = H, Z = 3-pyridyle

Une solution de 10,6 g de (trifluorométhyl-3 phényl)amino-2 nicotinaldéhyde préparés à l'exemple 3 et de 10 g d'acétate d'éthyle-3 pyridine dans 75 ml de toluène est ajoutée goutte à goutte à une suspension de 2,9 g d'hydrure de sodium dans 50 ml de toluène. La réaction est amorcée avec quelques gouttes d'éthanol puis agitée 1 h à température ambiante et 20 min à 80° C. Après refroidissement, on verse sur un mélange d'eau et de glace et on ajoute du chloroforme. La phase organique séparée est lavée à l'eau, puis séchée sur sulfate de sodium. Le solvant est évaporé et le résidu cristallise dans un mélange éther isopropylique-acétone.Les cristaux obtenus sont essorés, lavés à l'éther et séchés. On récupère ainsi 5,5 g de [(trifluorométhyl-3 phényl)-1 dihydro-1,2 oxo-2 naphtyridine-1,8 yl-3] pyridyl-3 sous forme de cristaux de point de fusion 208-209° C.

Exemple 19
[(trifulorométhyl-3 phényl)-1 dihydro-1,2 oxo-2 naphtyridine-1,8 yl-3] butanol-4

Formule I X = 3-$CF_3$, Y = H, Z = $CH_2$-$CH_2$-$CH_2$-$CH_2$-OH

Préparé selon le mode opératoire de l'exemple 7 avec la $\epsilon$-caprolactone
Cristaux ($CCl_4$) F = 117-118 ° C ; rendement 40 %

Exemple 20
[(trifluorométhyl-3 phényl)-1 dihydro-1,2 oxo-2 naphtyridine-1,8 yl-3] propanol-2

Formule I X = 3-$CF_3$, Y = H, Z = $CH_2$-CHOH-$CH_3$

Préparé selon le mode opératoire de l'exemple 7 avec la $\gamma$-valérolactone
Cristaux F = 121-122 ° C ; rendement 22 %

Exemple 21
Acétate de (phényl-1 dihydro-1,2 oxo-2 naphtyridine-1,8 yl-3) butanol-4

Formule I X = Y = H, Z = $CH_2$-$CH_2$-$CH_2$-$CH_2$-O-CO-$CH_3$

Préparé selon le mode opératoire de l'exemple 11
Cristaux (isopropanol) F = 148-150 ° C ; rendement 92 %

Exemple 22
(phényl-1 dihydro-1,2 oxo-2 naphtyridine-1,8 yl-3) propanol-2

Formule I X = Y = H, Z = $CH_2$-CHOH-$CH_3$

Préparé selon le mode opératoire de l'exemple 7 avec la $\gamma$-valérolactone
Cristaux (xylène) F = 165 ° C ; rendement 34 %

Exemple 23
(phényl-1 dihydro-1,2-oxo-2 naphtyridine-1,8 yl-3) propanone-2

Formule I X = Y = H, Z = $CH_2$-CO-$CH_3$

A une solution de 8,5 g de (phényl-1 dihydro-1,2 oxo-2 naphtyridine-1,8 yl-3) propanol-2 préparés à l'exemple 22 dans 300 ml d'acétone on ajoute goutte à goutte 25 ml du réactif de Jones sous bonne agitation. Après la fin de l'addition, on agite encore 10 min, puis on additionne 50 ml d'eau et on agite encore 15 min. On additionne encore de l'eau et on extrait au chloroforme qu'on lave à l'eau, avec une solution de soude diluée puis encore à l'eau et qu'on sèche sur sulfate de sodium. Après évaporation du solvant, le résidu obtenu est filtré sur gel de silice et les cristaux formés sont recristallisés dans l'acétonitrile. On obtient ainsi 3,8 g de (phényl-1 dihydro-1,2 oxo-2 naphtyridine-1,8 yl-3) propanone-2 sous forme de cristaux de point de fusion 218-219 ° C.

Exemple 24
Diphényl-1,3 dihydro-1,2 oxo-2 naphtyridine-1,8

Formule I X = Y = H, Z = phényle

Préparé selon le mode opératoire de l'exemple 18 avec le phénylacétate d'éthyle.
Cristaux (isopropanol) F = 197-198 ° C ; rendement 40 %

Exemple 25
(phényl-1 dihydro-1,2 oxo-2 naphtyridine-1,8 yl-3) pyridyl-3

Formule I X = Y = H, Z = 3-pyridyle

Préparé selon le mode opératoire de l'exemple 18
Cristaux (acétonitrile) F = 236-238 °C ; rendement 30 %

Exemple 26
(phényl-1 dihydro-1,2 oxo-2 naphtyridine-1,8 yl-3) pyridyl-4

Formule I X = Y = H, Z = 4-pyridyle

Préparé selon le mode opératoire de l'exemple 18 avec l'acétate d'éthyle-4 pyridine
Cristaux (méthanol) F = 280-283 °C ; rendement 20 %

Exemple 27
[(trifluorométhyl-3 phényl)-1 dihydro-1,2 oxo-2 naphtyridine-1,8 yl-3] thiophène-3

Formule I X = 3-$CF_3$, Y = H, Z = 3-thiophène

Préparé selon le mode opératoire de l'exemple 18 avec l'acétate d'éthyle 3-thiophène
Cristaux F = 205-206 °C ; rendement 41 %.

Exemple 28
Phényl-1 dihydro-1,2 oxo-2 naphtyridine-1,8 méthoxy-3

Formule I X = Y = H, Z = $OCH_3$

On chauffe entre 150 et 160 °C durant 1 h un mélange de 9,9 g de phénylamino-2 nicotinaldéhyde préparés précédemment, 16 g d'anhydride méthoxyacétique et 7,8 g de méthoxyacétate de sodium.

Le mélange réactionnel est ensuite refroidi, additionné d'eau et de glace et extrait au chloroforme. La phase chloroformique est lavée à l'eau, avec une solution de soude diluée puis encore à l'eau et séchée sur sulfate de sodium. Le solvant évaporé, le résidu cristallise dans l'éther. Après recristallisation dans le diméthylformamide, on obtient 4 g de phényl-1 dihydro-1,2 oxo-2 naphtyridine-1,8 méthoxy-3 sous forme de cristaux de point de fusion 272-274 °C.

Exemple 29
(trifluorométhyl-3 phényl)-1 dihydro-1,2 oxo-2 naphtyridine-1,8 méthoxy-3

Formule I X = 3-$CF_3$, Y = H, Z = $OCH_3$

Préparé selon le mode opératoire de l'exemple 28
Cristaux (acétate d'éthyle) F = 221-223 °C ; rendement 30 %

Exemple 30
[(trifluorométhyl-3 phényl)-1 dihydro-1,2 oxo-2 naphtyridine-1,8 yl-3] furanne-2

Formule I X = 3-$CF_3$, Y = H, Z = 2-furanne

Préparé selon le mode opératoire de l'exemple 18 avec l'acétate d'éthyle-2 furanne
Cristaux (isopropanol) F = 205-206 °C ; rendement 20 %

Exemple 31
[(cyano-3 phényl)-1 dihydro-1,2 oxo-2 naphtyridine-1,8 yl-3] éthanol-2

Formule I X = 3-CN, Y = H, Z = $CH_2$-$CH_2$-OH

Préparé selon le mode opératoire de l'exemple 7
Cristaux F = 189 °C ; rendement 57 %.

Exemple 32

Acétate de [(cyano-3 phényl)-1 dihydro-1,2 oxo-2 naphtyridine-1,8 yl-3] éthanol-2

Formule I X = 3-CN, Y = H, Z = $CH_2$-$CH_2$-O-CO-$CH_3$

Préparé selon le mode opératoire de l'exemple 11
Cristaux (éthanol) F = 176-177 ° C ; rendement 80 %

Exemple 33
[(cyano-3 phényl)-1 dihydro-1,2 oxo-2 naphtyridine-1,8 yl-3] propanol-2

Formule I X = 3-CN, Y = H, Z = $CH_2$-CHOH-$CH_3$

Préparé selon le mode opératoire de l'exemple 7 avec la $\gamma$-valérolactone
Cristaux F = 124-127 ° C ; rendement 44 %

Exemple 34
[(cyano-3 phényl)-1 dihydro-1,2 oxo-2 naphtyridine-1,8 yl-3] propanone-2

Formule I X = 3-CN, Y = H, Z = $CH_2$-CO-$CH_3$

Préparé selon le mode operatoire de l'exemple 23
Cristaux F = 184-186 ° C ; rendement 62 %

Exemple 35
(cyano-3 phényl)-1 dihydro-1,2 oxo-2 naphtyridine-1,8 méthylthiométhyl-3

Formule I X = 3-CN, Y = H, Z = $CH_2$-S-$CH_3$

Préparé selon le mode opératoire de l'exemple 5
Cristaux (acétonitrile) F = 204-205 ° C ; rendement 20 %

Exemple 36
(cyano-3 phényl)-1 dihydro-1,2 oxo-2 naphtyridine-1,8 méthylsulfinylméthyl-3

Formule I X = 3-CN, Y = H, Z = $CH_2$-SO-$CH_3$

Préparé selon le mode opératoire de l'exemple 8
Cristaux F = 217-218 ° C ; rendement 70 %

Exemple 37
[(fluoro-4 phényl)-1 dihydro-1,2 oxo-2 naphtyridine-1,8 yl-3] éthanol-2

Formule I X = 4-F, Y = H, Z = $CH_2$-$CH_2$-OH

Préparé selon le mode opératoire de l'exemple 7
Cristaux (méthanol) F = 199-200 ° C ; rendement 48 %

Exemple 38
(méthylthio-3 phényl)-1 dihydro-1,2 oxo-2 naphtyridine-1,8 méthylthiométhyl-3

Formule I X = 3-$SCH_3$, Y = H, Z = $CH_2$-S-$CH_3$

A une solution de 9,8 g de (méthylthio-3 phényl) amino-2 nicotinaldéhyde préparés précédemment et de 7,4 g de $\beta$-méthylthiopropionate d'éthyle dans 30 ml d'éthanol, on ajoute goutte à goutte une solution d'éthylate de sodium préparée à partir de 40 ml d'éthanol et 1,2 g de sodium. Après la fin de l'addition, on porte durant 10 min à 50-60 ° C sous agitation. On observe un passage complet en solution puis formation d'un précipité. On laisse alors revenir à température ambiante et on continue l'agitation durant 30 min. Le précipité est essoré, lavé avec un peu d'éthanol, de l'eau puis à l'éther et séché. Par recristallisation dans le

méthanol, on obtient 3,8 g de (méthylthio-3 phényl)-1 dihydro-1,2 oxo-2 naphtyridine-1,8 méthylthiométhyl-3 sous forme de cristaux de point de fusion 132-133 ° C.

Exemple 39
[(trifluorométhyl-3 phényl)-1 dihydro-1,2 oxo-2 naphtyridine-1,8 yl-3] propanone-2

Formule I X = 3-$CF_3$, Y = H, Z = $CH_2$-CO-$CH_3$

préparé selon le mode opératoire de l'exemple 23
Cristaux F = 124-125 ° C ; rendement 60 %

Exemple 40
[(méthylthio-3 phényl)-1 dihydro-1,2 oxo-2 naphtyridine-1,8 yl-3] propanol-2

Formule I X = 3-$SCH_3$, Y = H, Z = $CH_2$-CHOH-$CH_3$

A une solution de 12 g de (méthylthio-3 phényl)amino-2 nicotinaldéhyde preparés précédemment et 6,4 g de $\gamma$-valérolactone dans 50 ml d'éthanol, on ajoute goutte à goutte sous bonne agitation une solution d'éthylate de sodium préparée à partir de 1,5 g de sodium dans 50 ml d'éthanol à une température de 40 ° C. Après la fin de l'addition, on laisse le mélange réactionnel revenir à température ambiante puis on agite durant 3 h. Le précipité formé est essoré, lavé à l'eau puis avec un peu d'éthanol et séché. Après recristallisation dans l'acétate d'éthyle, on obtient 7 g de [(méthylthio-3 phényl)-1 dihydro-1,2 oxo-2 naphtyridine-1,8 yl-3] propanol-2 sous forme de cristaux de point de fusion 116-117 ° C.

Exemple 41
[(difluoro-2,5 phényl)-1 dihydro-1,2 oxo-2 naphtyridine-1,8 yl-3] propanol-2

Formule I X = 2-F, Y = 5-F, Z = $CH_2$-CHOH-$CH_3$

Préparé selon le mode opératoire de l'exemple 40
Cristaux (éther isopropylique) F = 111-112 ° C ; rendement 52 %

Exemple 42
[(difluoro-2,5 phényl)-1 dihydro-1,2 oxo-2 naphtyridine-1,8 yl-3] propanone-2

Formule I X = 2-F, Y = 5-F, Z = $CH_2$-CO-$CH_3$

A une solution de 5 g de [(difluoro-2,5 phényl)-1 dihydro-1,2 oxo-2 naphtyridine-1,8 yl-3] propanol-2 préparés à l'exemple 41 dans 50 ml de dichlorométhane, on ajoute par petites quantités sous bonne agitation 10 g de réactif de Sarett. On continue après la fin de l'addition l'agitation durant 4 h puis on rajoute 10 g de réactif de Sarett et on agite encore 5 h en portant au reflux. Après un repos d'une nuit à température ambiante, on filtre sur Celite et la phase dichlorométhane est lavée soigneusement à l'eau et séchée. Après évaporation du solvant, le résidu obtenu est filtré sur gel de silice. Dans le mélange isopropanoléther isopropylique, on obtient des cristaux qui sont essorés et lavés à l'éther isopropylique puis séchés. On obtient ainsi 2,5 g de [(difluoro-2,5 phényl)-1 dihydro-1,2 oxo-2 naphtyridine-1,8 yl-3] propanone-2 sous forme de cristaux de point de fusion 141-142 ° C.

Exemple 43
(fluoro-4 phényl)-1 dihydro-1,2 oxo-2 naphtyridine-1,8 méthylthiométhyl-3

Formule I X = 4-F, Y = H, Z = $CH_2$-S-$CH_3$

Préparé selon le mode opératoire de l'exemple 38
Cristaux (diméthylformamide) F = 225-226 ° C ; rendement 60 %

Exemple 44
(chloro-3 phényl)-1 dihydro-1,2 oxo-2 naphtyridine-1,8 méthylthiométhyl-3

Formule I X = 3-Cl, Y = H, Z = $CH_2$-S-$CH_3$

Préparé selon le mode opératoire de l'exemple 38
Cristaux (acétate d'éthyle) F = 167-168° C ; rendement 40 %

Exemple 45
(méthoxy)3 phényl)-1 dihydro-1,2 oxo-2 naphtyridine-1,8 méthylthiométhyl-3

Formule I X = 3-$OCH_3$, Y = H, Z = $CH_2$-S-$CH_3$

Préparé selon le mode opératoire de l'exemple 38
Cristaux (méthanol) F = 181-182° C ; rendement 55 %

Exemple 46
[(chloro-3 phényl)-1 dihydro-1,2 oxo-2 naphtyridine-1,8 yl-3] propanol-2

Formule I X = 3-Cl, Y = H, Z = $CH_2$-CHOH-$CH_3$

Préparé selon le mode opératoire de l'exemple 40
Cristaux F = 132° C ; rendement 40 %

Exemple 47
[(chloro-3 phényl)-1 dihydro-1,2 oxo-2 naphtyridine-1,8 yl-3] propanone-2

Formule I X = 3-Cl, Y = H, Z = $CH_2$-CO-$CH_3$

Préparé selon le mode opératoire de l'exemple 42
Cristaux F = 166° C ; rendement 50 %

Exemple 48
[(cyano-3 phényl)-1 dihydro-1,2 oxo-2 naphtyridine-1,8 yl-3] propanol-3

Formule I X = 3-CN, Y = H, Z = $CH_2$-$CH_2$-$CH_2$-OH

Préparé selon le mode opératoire de l'exemple 40 avec la δ-valérolactone
Cristaux (éthanol) F = 166-167° C ; rendement 37 %

Exemple 49
[(chloro-3 phényl)-1 dihydro-1,2 oxo-2 naphtyridine-1,8 yl-3] éthanol-2

Formule I X = 3-Cl, Y = H, Z = $CH_2$-$CH_2$-OH

Préparé selon le mode opératoire de l'exemple 7
Cristaux (acétonitrile) F = 175° C ; rendement 40 %

Exemple 50
Acétate de [(chloro-3 phényl)-1 dihydro-1,2 oxo-2 naphtyridine-1,8 yl-3] éthanol-2

Formule I X = 3-Cl, Y = H, Z = $CH_2$-$CH_2$-O-CO-$CH_3$

Préparé selon le mode opératoire de l'exemple 11
Cristaux (isopropanol) F = 138-139° C ; rendement 90 %

Exemple 51
(cyano-3 phényl)-1 dihydro-1,2 oxo-2 naphtyridine-1,8 méthoxyméthyl-3

Formule I X = 3-CN, Y = H, Z = $CH_2$-$OCH_3$

Préparé selon le mode opératoire de l'exemple 38 en utilisant le $\beta$-méthoxypropionate d'éthyle
Cristaux (méthanol) F = 192-193 ° C ; rendement 27 %

Exemple 52
(cyano-3 phényl)-1 dihydro-1,2 oxo-2 naphtyridine-1,8 méthoxy-3

Formule I  X = 3-CN, Y = H, Z = $OCH_3$

Préparé selon le mode opératoire de l'exemple 38 en utilisant le méthoxyacétate d'éthyle
Cristaux (diméthylformamide) F = 284 ° C ; rendement 29 %

Exemple 53
[(méthoxy-3 phényl)-1 dihydro-1,2 oxo-2 naphtyridine-1,8 yl-3] propanol-2

Formule I  X = 3-$OCH_3$, Y = H, Z = $CH_2$-CHOH-$CH_3$

Préparé selon le mode opératoire de l'exemple 40
Cristaux F = 171 ° C ; rendement 53 %

Exemple 54
[(méthoxy-3 phényl)-1 dihydro-1,2 oxo-2 naphtyridine-1,8 yl-3] propanone-2

Formule I  X = 3-$OCH_3$, Y = H, Z = $CH_2$-CO-$CH_3$

Préparé selon le mode opératoire de l'exemple 23
Cristaux (acétate d'éthyle) F = 161-164 ° C ; rendement 43 %

Exemple 55
(nitro-3 phényl)-1 dihydro-1,2 oxo-2 naphtyridine-1,8 méthylthiométhyl-3

Formule I  X = 3-$NO_2$, Y = H, Z = $CH_2$-S-$CH_3$

Préparé selon le mode opératoire de l'exemple 38
Cristaux (diméthylformamide) F = 190-191 ° C ; rendement 22 %

Exemple 56
[(méthylthio-3 phényl)-1 dihydro-1,2 oxo-2 naphtyridine-1,8 yl-3] propanone-2

Formule I  X = 3-$SCH_3$, Y = H, Z = $CH_2$-CO-$CH_3$

Préparé selon le mode opératoire de l'exemple 23
Cristaux (filtration sur gel de silice, éluant dichlorométhaneéther 9-1) F = 146 ° C ; rendement 5 %

Exemple 57
[(fluoro-4 phényl)-1 dihydro-1,2 oxo-2 naphtyridine-1,8 yl-3] propanol-2

Formule I  X = 4-F, Y = H, Z = $CH_2$-CHOH-$CH_3$

Préparé selon le mode opératoire de l'exemple 40
Cristaux F = 171-172 ° C ; rendement 41 %

Exemple 58
[(fluoro-4 phényl)-1 dihydro-1,2 oxo-2 naphtyridine-1,8 yl-3] propanone-2

Formule I  X = 4-F, Y = H, Z = $CH_2$-CO-$CH_3$

Préparé selon le mode opératoire de l'exemple 23
Cristaux (acétate d'éthyle) F = 203 ° C ; rendement 60 %

Exemple 59
[(nitro-3 phényl)-1 dihydro-1,2 oxo-2 naphtyridine-1,8 yl-3] propanol-3

Formule I X = 3-$NO_2$, Y = H, Z = $CH_2$-$CH_2$-$CH_2$-OH

Préparé selon le mode opératoire de l'exemple 40 avec la δ-valérolactone
Cristaux (toluène) F = 160-162 ° C ; rendement 20 %

Exemple 60
Phényl-1 dihydro-1,2 oxo-2 naphtyridine-1,8 méthoxyméthyl-3

Formule I X = Y = H, Z = $CH_2$-$OCH_3$

Préparé selon le mode opératoire de l'exemple 38 en utilisant le β-méthoxypropionate de méthyle
Cristaux (acétonitrile) F = 206-207 ° C ; rendement 65 %

Exemple 61
Phényl-1 dihydro-1,2 oxo-2 naphtyridine-1,8 méthylthiométhyl-3

Formule I X = Y = H, Z = $CH_2$-$SCH_3$

Préparé selon le mode opératoire de l'exemple 38
Cristaux (acétonitrile) F = 207-208 ° C ; rendement 61 %

Exemple 62
(cyano-3 chloro-4 phényl)-1 dihydro-1,2 oxo-2 naphtyridine-1,8 méthylthiométhyl-3

Formule I X = 3-CN, Y = 4-Cl, Z = $CH_2$-$SCH_3$

Préparé selon le mode opératoire de l'exemple 38
Cristaux (acétonitrile) F = 218-219 ° C ; rendement 60 %

Exemple 63
[(cyano-3 chloro-4 phényl)-1 dihydro-1,2 oxo-2 naphtyridine-1,8 yl-3] propanol-2

Formule I X = 3-CN, Y = 4-Cl, Z = $CH_2$-CHOH-$CH_3$

Préparé selon le mode opératoire de l'exemple 40
Cristaux (acétonitrile) F = 201-202 ° C ; rendement 52 %

Exemple 64
[(cyano-3 chloro-4 phényl)-1 dihydro-1,2 oxo-2 naphtyridine-1,8 yl-3] propanone-2

Formule I X = 3-CN, Y = 4-Cl, Z = $CH_2$-CO-$CH_3$

Préparé selon le mode opératoire de l'exemple 23
Cristaux (acétonitrile) F = 202-2o4 ° C ; rendement 43 %

Exemple 65
(phényl-1-dihydro-1,2 oxo-2 naphtyridine-1,8 yl-3) propène-1,2

Formule I X = Y = H, Z = CH=CH-$CH_3$

Préparé selon le mode opératoire de l'exemple 38 avec l'ester éthylique de l'acide 4-penténoïque
Cristaux après filtration sur silicagel et recristallisation dans l'isopropanol F = 201-202 ° C ; rendement 10 %

Exemple 66
Phényldihydro-1,2 oxo-2 naphtyridine-1,8 cyano-3

Formule I   X = Y = H, Z = CN

On porte au reflux durant 3 h une solution de 10 g de phénylamino-2 nicotinaldéhyde préparés précédemment, 6,8 g de cyanacétate d'éthyle dans 100 ml de toluène contenant 1 ml de pipéridine et 0,8 ml d'acide acétique. Après avoir laissé le mélange réactionnel revenir à température ambiante, les cristaux formés sont essorés, lavés à l'eau puis avec un peu d'éthanol et séchés. Après recristallisation dans l'acétonitrile, on récupère 4,8 g de phényl-1 dihydro-1,2 oxo-2 naphtyridine-1,8 cyano-3 sous forme de cristaux de point de fusion 289 °C.

Exemple 67

(cyano-3 fluoro-4 phényl)-1 dihydro-1,2 oxo-2 naphtyridine-1,8 méthylthiométhyl-3

Formule I   X = 3-CN, Y = 4-F, Z = $CH_2$-$SCH_3$

Préparé selon le mode opératoire de l'exemple 38
Cristaux après séparation par HPLC préparative F = 223 °C ; rendement 17 %

Exemple 68

[(cyano-3 éthoxy-4 phényl)-1 dihydro-1,2 oxo-2 naphtyridine-1,8 yl-3] propanone-2

Formule I   X = 3-CN, Y = 4-$OC_2H_5$, Z = $CH_2$-CO-$CH_3$

En utilisant le mode opératoire de l'exemple 40 pour faire réagir la γ-valérolactone avec le (cyano-3 fluoro-4 phényl)amino-nicotinaldéhyde on observe, en plus de la réaction de condensation, la substitution de l'atome F par le groupement éthoxy, l'éthylate de sodium ayant réagi sur cet halogène activé par le groupement nitrile. Une HPLC préparative permet donc d'isoler le [(cyano-3 éthoxy-4 phényl)-1 dihydro-1,2 oxo-2 naphtyridine-1,8 yl-3] propanol-2 sous forme de cristaux de point de fusion 145 °C qui sont oxydés par le réactif de Jones selon le mode opératoire de l'exemple 23 en [(cyano-3 éthoxy-4 phényl)-1 dihydro-1,2 oxo-2 naphtyridine-1,8 yl-3] propanone-2 sous forme de cristaux de point de fusion 188-189 °C avec un rendement global pour les deux réactions de 5 %.

Exemple 69

Acétate de [(cyano-3 phényl)-1 dihydro-1,2 oxo-2 naphtyridine-1,8 yl-3] propanol-2

Formule I   X = 3-CN, Y = H, Z = $CH_2$-CH(O-CO-$CH_3$)-$CH_3$

Préparé selon le mode opératoire de l'exemple 11
Cristaux (éthanol F = 160-162 °C ; rendement 65 %

Exemple 70

Nicotinate de [(cyano-3 phényl)-1 dihydro-1,2 oxo-2 naphtyridine-1,8 yl-3] propanol-2

Formule I   X = 3-CN, Y = H, Z = $CH_2$-CH(O-CO 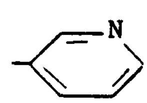 )-$CH_3$

On porte au reflux durant 5 h une solution de 5 g de [(cyano-3 phényl)-1 dihydro-1,2 oxo-2 naphtyridine-1,8 yl-3] propanol-2 préparés à l'exemple 33 et de 4 g du chlorure de l'acide nicotinique dans 150 ml de tétrahydrofuranne contenant 2,5 ml de triéthylamine. Après avoir laissé revenir à température ambiante, on laisse au repos une nuit puis on ajoute de l'eau additionnée de soude et on extrait au chloroforme. La phase organique est lavée à l'eau puis séchée sur sulfate de sodium et le chloroforme évaporé, le résidu obtenu cristallise dans l'éther. Après recristallisation dans l'acétonitrile, on récupère 3,8 g de nicotinate de [(cyano-3 phényl)-1 dihydro-1,2 oxo-2 naphtyridine-1,8 yl-3] propanol-2 sous forme de cristaux de point de fusion 179-180 °C.

Exemple 71
Nicotinate de (phényl-1 dihydro-1,2 oxo-2 naphtyridine-1,8 yl-3) propanol-2

Formule I X = Y = H, Z = $CH_2$-CH(O-CO 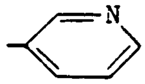 )-$CH_3$

Préparé selon le mode opératoire de l'exemple 70
Cristaux (acétonitrile) F = 154-155 °C ; rendement 60 %

Exemple 72
Acétate de [(cyano-3 phényl)-1 dihydro-1,2 oxo-2 naphtyridine-1,8 yl-3] propanol-3

Formule I X = 3-CN, Y = H, Z = $CH_2$-$CH_2$-$CH_2$-O-CO-$CH_3$

Préparé selon le mode opératoire de l'exemple 11
Cristaux (éthanol) F = 151-153 °C ; rendement 80 %

Exemple 73
Acétate de (phényl-1 dihydro-1,2 oxo-2 naphtyridine-1,8 yl-3) propanol-2

Formule I X = Y = H, Z = $CH_2$-CH(O-CO-$CH_3$)-$CH_3$

Préparé selon le mode opératoire de l'exemple 11
Cristaux (isopropanol) F = 114-116 °C ; rendement 80 %

Exemple 74
Acétate de [(méthylthio-3 phényl)-1 dihydro-1,2 oxo-2 naphtyridine-1,8 yl-3] propanol-2

Formule I X = 3-$SCH_3$, Y = H, Z = $CH_2$-CH(O-CO-$CH_3$)-$CH_3$

Préparé selon le mode opératoire de l'exemple 11
Cristaux F = 138 °C ; rendement 88 %

Exemple 75
Acétate de [(chloro-3 phényl)-1 dihydro-1,2 oxo-2 naphtyridine-1,8 yl-3] propanol-2

Formule I X = 3-Cl, Y = H, Z = $CH_2$-CH(O-CO-$CH_3$)-$CH_3$

Préparé selon le mode opératoire de l'exemple 11
Cristaux F = 119-120 °C ; rendement 90 %

Exemple 76
Nicotinate de [(chloro-3 phényl)-1 dihydro-1,2 oxo-2 naphtyridine-1,8 yl-3] propanol-2

Formule I X = 3-Cl, Y = H, Z = $CH_2$-CH(O-CO 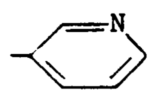 )-$CH_3$

Préparé selon le mode opératoire de l'exemple 70
Cristaux (isopropanol-éther isopropylique) F = 130 °C ; rendement 62 %

Exemple 77
Propionate de (phényl-1 dihydro-1,2 oxo-2 naphtyridine-1,8 yl-3) propanol-2

Formule I X = Y = H, Z = $CH_2$-CH(O-CO-$C_2H_5$)-$CH_3$

Préparé selon le mode opératoire de l'exemple 11 avec l'anhydride propionique
Cristaux (éther isopropylique) F = 90-92 °C ; rendement 68 %

Exemple 78
Acétate de [(trifluorométhyl-3 phényl)-1 dihydro-1,2 oxo-2 naphtyridine-1,8 yl-3] propanol-2

Formule I X = $3\text{-}CF_3$, Y = H, Z = $CH_2\text{-}CH(O\text{-}CO\text{-}CH_3)\text{-}CH_3$

Préparé selon le mode opératoire de l'exemple 11
Cristaux (isopropanol) F = 142-143 °C ; rendement 90 %

Exemple 79
[(méthylthio-3 phényl)-1 dihydro-1,2 oxo-2 naphtyridine-1,8 yl-3] propanol-3

Formule I X = $3\text{-}SCH_3$, Y = H, Z = $CH_2\text{-}CH_2\text{-}CH_2\text{-}OH$

Préparé selon le mode opératoire de l'exemple 40 avec la δ-valérolactone
Cristaux (acétonitrile) avec une molécule d'eau d'hydratation F = 114-116 °C ; rendement 44 %

Exemple 80
Maléate de S-méthyl N-méthyl N′-[[(phényl-1 dihydro-1,2 oxo-2 naphtyridine-1,8 yl-3) méthylthio]-2-éthyl] isothiourée

Formule I X = Y = H, Z = $CH_2\text{-}S\text{-}CH_2\text{-}CH_2\text{-}NH\text{-}C(\text{-}S\text{-}CH_3)\text{=}N\text{-}CH_3$

Préparé selon le mode opératoire de l'exemple 38 en utilisant l'ester éthylique de l'acide S-méthyl N-méthyl N′-éthylthio propionique isothiourée. Sur les cristaux obtenus, F = 164 °C par addition d'acide maléique dans un mélange acétone éther, on obtient le maléate sous forme de cristaux de point de fusion 181-183 °C ; rendement 15 %.

Exemple 81
[(chloro-4 phényl)-1 dihydro-1,2 oxo-2 naphtyridine-1,8 yl-3] propanol-2

Formule I X = 4-Cl, Y = H, Z = $CH_2\text{-}CHOH\text{-}CH_3$

Préparé selon le mode opératoire de l'exemple 40
Cristaux (xylène) F = 166 °C ; rendement 41 %

Exemple 82
Acétate de [(chloro-4 phényl)-1 dihydro-1,2 oxo-2 naphtyridine-1,8 yl-3] propanol-2

Formule I X = 4-Cl, Y = H, Z = $CH_2\text{-}CH(O\text{-}CO\text{-}CH_3)\text{-}CH_3$

Préparé selon le mode opératoire de l'exemple 11
Cristaux F = 167 °C ; rendement 92 %

Exemple 83
Acétate de [(méthylthio-3 phényl)-1 dihydro-1,2 oxo-2 naphtyridine-1,8 yl-3] propanol-3

Formule I X = $3\text{-}SCH_3$, Y = H, Z = $CH_2\text{-}CH_2\text{-}CH_2\text{-}O\text{-}CO\text{-}CH_3$

Préparé selon le mode opératoire de l'exemple 11
Cristaux (isopropanol) F = 93-94 °C ; rendement 76 %

Exemple 84
[(fluoro-4 phényl)-1 dihydro-1,2 oxo-2 naphtyridine-1,8 yl-3] propanol-3

Formule I X = 4-F, Y = H, Z = $CH_2$-$CH_2$-$CH_2$-OH

Préparé selon le mode opératoire de l'exemple 40 avec la δ-valérolactone
Cristaux (éthanol) F = 168-169 °C ; rendement 35 %

Exemple 85
[(chloro-4 phényl)-1 dihydro-1,2 oxo-2 naphtyridine-1,8 yl-3] propanol-3

Formule I X = 4-Cl, Y = H, Z = $CH_2$-$CH_2$-$CH_2$-OH

Préparé selon le mode opératoire de l'exemple 40 avec la δ-valérolactone
Cristaux (isopropanol) F = 165 °C ; rendement 42 %

Exemple 86
Acétate de [(chloro-4 phényl)-1 dihydro-1,2 oxo-2 naphtyridine-1,8 yl-3] propanol-3

Formule I X = 4-Cl, Y = H, Z = $CH_2$-$CH_2$-$CH_2$-O-CO-$CH_3$

Préparé selon le mode opératoire de l'exemple 11
Cristaux F = 143-145 °C ; rendement 90 %

Exemple 87
[(chloro-4 phényl)-1 dihydro-1,2 oxo-2 naphtyridine-1,8 yl-3] propanone-2
Formule I X = 4-Cl, Y = H, Z = $CH_2$-CO-$CH_3$

Préparé selon le mode opératoire de l'exemple 23
Cristaux (isopropanol) F = 192-193 °C ; rendement 45 %

Exemple 88
(phényl-1 dihydro-1,2 oxo-2 naphtyridine-1,8 yl-3)-2 phényl-1 éthanol

Formule I X = Y = H, Z = $CH_2$-CH(OH) 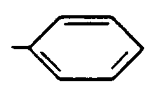

Préparé selon le mode opératoire de l'exemple 40 avec la 5-phénylbutyrolactone
Cristaux (isopropanol) F = 149-151 °C ; rendement 32 %

Exemple 89
Furanne-2 carboxylate de (phényl-1 dihydro-1,2 oxo-2 naphtyridine-1,8 yl-3) propanol-2

Formule I X = Y = H, Z = $CH_2$-CH(O-CO 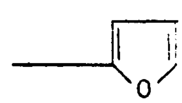 )-$CH_3$

Préparé selon le mode opératoire de l'exemple 70 avec le chlorure de l'acide furanne-2 carboxylique
Cristaux (isopropanol) F = 140-142 °C ; rendement 55 %

Exemple 90
Phénylacétate de (phényl-1 dihydro-1,2 oxo-2 naphtyridine-1,8 yl-3) propanol-2

Formule I X = Y = H, Z = $CH_2$-CH(O-CO-$CH_2$ 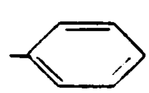 ) -$CH_3$

Préparé selon le mode opératoire de l'exemple 70 avec le chlorure de l'acide phénylacétique
Cristaux (isopropanol) F = 102-104 ° C ; rendement 50 %

Exemple 91
[(cyano-3 phényl)-1 dihydro-1,2 oxo-2 naphtyridine-1,8 yl-3] butanol-4

Formule I X = 3-CN, Y = H, Z = $CH_2-CH_2-CH_2-CH_2-OH$

Préparé selon le mode opératoire de l'exemple 7 avec la $\epsilon$-caprolactone
Cristaux (acétone) F = 160-162 ° C ; rendement 22 %

Exemple 92
Acétate de (phényl-1 dihydro-1,2 oxo-2 naphtyridine-1,8 yl-3)-2 phényl-1 éthanol

Formule I X = Y = H, Z = $CH_2-CH(O-CO-CH_3)$ 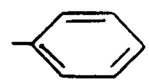

Préparé selon le mode opératoire de l'exemple 11
Cristaux (acétone) F = 19O-192 ° C ; rendement 70 %

Exemple 93
Acétate de [(fluoro-4 phényl)-1 dihydro-1,2 oxo-2 naphtyridine-1,8 yl-3] propanol-3

Formule I X = 4-F, Y = H, Z = $CH_2-CH_2-CH_2-O-CO-CH_3$

Préparé selon le mode opératoire de l'exemple 11
Cristaux (xylène) F = 154-155 ° C ; rendement 85 %

Exemple 94
Acétate de [(méthylthio-3 phényl)-1 dihydro-1,2 oxo-2 naphtyridine-1,8 yl-3] éthanol-2

Formule I X = $3-SCH_3$, Y = H, Z = $CH_2-CH_2-O-CO-CH_3$

Préparé selon le mode opératoire de l'exemple 11
Cristaux F = 118-121 ° C ; rendement 92 %

Exemple 95
(phényl-1 dihydro-1,2 oxo-2 naphtyridine-1,8 yl-3) butanol-3

Formule I X = Y = H, Z = $CH_2-CH_2-CHOH-CH_3$

Préparé selon le mode opératoire de l'exemple 40 avec la $\delta$-caprolactone
Cristaux (toluène) F = 132-135 ° C ; rendement 50 %

Exemple 96
Acétate de (phényl-1 dihydro-1,2 oxo-2 naphtyridine-1,8 yl-3) butanol-3

Formule I X = Y = H, Z = $CH_2-CH_2-CH-(O-CO-CH_3)-CH_3$

Préparé selon le mode opératoire de l'exemple 11
Cristaux F = 140-141 ° C ; rendement 82 %

Exemple 97
(phényl-1 dihydro-1,2 oxo-2 naphtyridine-1,8 yl-3) butanone-3

Formule I X = Y = H, Z = $CH_2-CH_2-CO-CH_3$

Préparé selon le mode opératoire de l'exemple 23
Cristaux (acétone) F = 157 ° C ; rendement 60 %

Exemple 98
(phényl-1 dihydro-1,2 oxo-2 naphtyridine-1,8 yl-3)-2 (pyridinyl-3)-1 éthanol

Formule I X = Y = H, Z = $CH_2$-CH(OH) 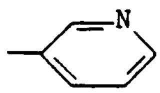

Préparé selon le mode opératoire de l'exemple 40 avec la 5-(pyridinyl-3) butyrolactone
Cristaux (acétone-éther) F = 155-157 ° C ; rendement 36 %

Exemples 99 et 100

On agite durant 7 h à température ambiante une solution de 15 g de (cyano-3 fluoro-4 phényl)amino-2 nicotinaldéhyde préparés précedemment et de 7,5 g de γ-valérolactone dans 500 ml de tétrahydrofuranne contenant une solution de méthylate de sodium préparé à partir de 1,7 g de sodium dans 50 ml de méthanol. Après un repos d'une nuit, le mélange réactionnel est additionné d'eau et de glace et extrait au chloroforme qu'on lave à l'eau, sèche sur sulfate de sodium et évapore. On obtient ainsi un mélange de 11g de deux dérivés; avec la réaction de condensation, on constate qu'une partie de dérivé fluoré a réagi avec le méthylate de sodium pour donner un groupement méthoxy. Par filtration sur une HPLC préparative, on récupère les deux dérivés suivants.

Exemple 99
[(cyano-3 fluoro-4 phényl)-1 dihydro-1,2 oxo-2 naphtyridine-1,8 yl-3] propanol-2

Formule I X = 3-CN, Y = 4-F, Z = $CH_2$-CHOH-$CH_3$

Cristaux F = 208 ° C ; quantité 3,2 g

Exemple 100
[(cyano-3 méthoxy-4 phényl)-1 dihydro-1,2 oxo-2 naphtyridine-1,8 yl-3] propanol-2

Formule I X = 3-CN, Y = 4-$OCH_3$, Z = $CH_2$-CHOH-$CH_3$

Cristaux F = 191-192 ° C ; quantité 4,3 g

Exemple 101
Phényl-1 dihydro-1,2 oxo-2 naphtyridine-1,8 éthoxyméthyl-3

Formule I X = Y = H, Z = $CH_2$-O-$C_2H_5$

Préparé selon le mode opératoire de l'exemple 38 en utilisant le β-éthoxypropionate d'éthyle
Cristaux (méthanol) F = 182 ° C ; rendement 30 %

Exemple 102
N-cyano N′-[[(phényl-1 dihydro-1,2 oxo-2 naphtyridine-1,8 yl-3) méthylthio]-2 éthyl] N″-méthylguanidine

Formule I X = Y = H, Z = $CH_2$-S-$CH_2$-$CH_2$-NH- 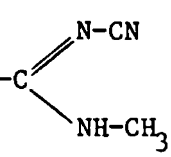

Préparé selon le mode opératoire de l'exemple 38 en utilisant l'ester méthylique de l'acide N-cyano N′-éthylthio propionique N″-méthylguanidine dans le méthanol avec le méthylate de sodium.

Cristaux (acétonitrile) F = 196-197 ° C ; rendement 24 %

Exemple 103

N-cyano N′-[[((méthylthio-3 phényl)-1 dihydro-1,2 oxo-2 naphtyridine-1,8 yl-3) méthylthio]-2 éthyl] N″-méthylguanidine

Formule I $X = 3\text{-}SCH_3$, $Y = H$, $Z =$ 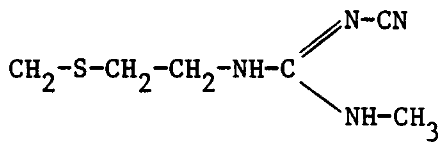

Préparé selon le mode opératoire de l'exemple 38 en utilisant l'ester méthylique de l'acide N-cyano N′-éthylthio propionique N″-méthylguanidine dans le méthanol avec le méthylate de sodium.
Cristaux (méthanol) F = 176 ° C ; rendement 20 %

Exemple 104

(Phényl-1 dihydro-1,2 oxo-2 naphtyridine-1,8 yl-3) bromo-3 propane

Formule I X = Y = H, Z = $CH_2$-$CH_2$-$CH_2$-Br

On agite à température ambiante durant 1 h 30,7 g de (phényl-1 dihydro-1,2 oxo-2 naphtyridine-1,8 yl-3) propanol-3 préparés à l'exemple 12 dans 614 ml d'acide bromhydrique 47 %. Le mélange réactionnel est ensuite porté à 90 ° C durant 6 h puis concentré sous vide, repris à l'eau et extrait au dichlorométhane qu'on lave à l'eau et sèche sur sulfate de sodium. Le dichlorométhane évaporé, on récupère 37 g de (phényl-1 dihydro-1,2 oxo-2 naphtyridine-1,8 yl-3) bromo-3 propane sous forme de cristaux de point de fusion 162 ° C.

Exemple 105

(phényl-1 dihydro-1,2 oxo-2 naphtyridine-1,8 yl-3) méthoxy-3 propane

Formule I X = Y = H, Z = $CH_2$-$CH_2$-$CH_2$-O-$CH_3$

On porte au reflux durant 3 h 10 g de (phényl-1 dihydro-1,2 oxo-2 naphtyridine-1,8 yl-3) bromo-3 propane dans une solution de méthylate de sodium préparée en faisant réagir 1 g de sodium dans 100 ml de méthanol. Le mélange réactionnel est ensuite concentré sous vide puis le résidu repris à l'eau est extrait au dichlorométhane qu'on lave à l'eau, sèche sur sulfate de sodium et qu'on évapore.

Après recristallisation des cristaux ainsi obtenus dans l'isopropanol, on récupère 4,2 g de (phényl-1 dihydro-1,2 oxo-2 naphtyridine-1,8 yl-3) méthoxy-3 propane sous forme de cristaux de point de fusion 136-137 ° C.

Exemple 106

(phényl-1 dihydro-1,2 oxo-2 naphtyridine-1,8 yl-3) méthylthio-3 propane

Formule I X = Y = H, Z = $CH_2$-$CH_2$-$CH_2$-S-$CH_3$

On agite durant 3 h une solution de 10 g de (phényl-1 dihydro-1,2 oxo-2 naphtyridine-1,8 yl-3) bromo-3 propane dans 50 ml de diméthylsulfoxyde contenant 2,5 g du sel de sodium du méthylmercaptan. On observe au début de la réaction un effet exothermique et un passage total en solution puis, lorsque le mélange réactionnel est revenu à température ambiante, la formation d'un précipité. Le mélange réactionnel est additionné ensuite d'eau et de glace et le précipité formé est essoré et soigneusement lavé à l'eau puis séché. Par recristallisation dans l'isopropanol, on récupère 7 g de (phényl-1 dihydro-1,2 oxo-2 naphtyridine-1,8 yl-3) méthylthio-3 propane sous forme de cristaux de point de fusion 135-136 ° C.

Exemple 107

(phényl-1 dihydro-1,2 oxo-2 naphtyridine-1,8 yl-3) cyano-3 propane

Formule I X = Y = H, Z = $CH_2$-$CH_2$-$CH_2$-CN

On agite à température ambiante durant 3 h une solution de 10 g de (phényl-1 dihydro-1,2 oxo-2 naphtyridine-1,8 yl-3) bromo-3 propane dans 50 ml de diméthylsulfoxyde contenant 1,5 g de cyanure de sodium. Le mélange réactionnel est ensuite additionné d'eau et de glace et le précipité formé est essoré, soigneusement lavé à l'eau et séché. Par recristallisation dans l'acétonitrile, on récupère 4,3 g de (phényl-1 dihydro-1,2 oxo-2 naphtyridine-1,8 yl-3) cyano-3 propane sous forme de cristaux de point de fusion 210-211 ° C.

Exemple 108

N-[[(phényl-1 dihydro-1,2 oxo-2 naphtyridine-1,8 yl-3) méthylthio]-2 éthyl] N′-méthylnitro-2 diamino-1,1 éthène

Formule I X = Y = H, Z = 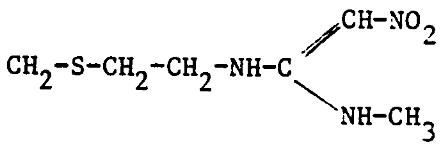

Préparé selon le mode opératoire de l'exemple 38 en utilisant l'ester méthylique de l'acide N-éthylthio propionique N′-méthylnitro-2 diamino-1,1 éthène dans le méthanol avec le méthylate de sodium.
Cristaux (diméthylformamide-eau) hydratés avec 1/3 $H_2O$ F = 207-209 ° C ; rendement 12 %

Exemple 109

Acide 5 [(trifluorométhyl-3 phényl)-1 dihydro-1,2 oxo-2 naphtyridine-1,8 yl-3] pentanoïque

Formule I X = 3-$CF_3$, Y = H, Z = $CH_2$-$CH_2$-$CH_2$-$CH_2$-COOH

On porte à 170-180 ° C durant 1 h 15 min un mélange de 18,3 g de (trifluorométhyl-3 phényl) amino-2 nicotinaldéhyde synthétisés précédemment, 16 g de pimélate de sodium et 20 g d'anhydride pimélique dans 40 ml de N-méthylpyrrolidone.

Le mélange réactionnel est ensuite refroidi, additionné d'eau et de glace puis basifié avec une solution de soude 10 %. Les produits neutres sont extraits à l'acétate d'éthyle. La phase aqueuse est ensuite acidifiée à froid par de l'acide acétique et extraite au dichlorométhane qu'on lave à l'eau et qu'on sèche sur sulfate de sodium. Après évaporation du dichlorométhane, le résidu obtenu est chromatographié sur gel de silice. Par élution avec un mélange dichlorométhane 99,5 et acide acétique 0,5, on obtient un résidu qui cristallise dans l'éther isopropylique. Les cristaux obtenus sont essorés, lavés à l'éther isopropylique et séchés. On obtient ainsi 6,3 g d'acide 5-[(trifluorométhyl-3 phényl)-1 dihydro-1,2 oxo-2 naphtyridine-1,8 yl-3] pentanoïque sous forme de cristaux de point de fusion 136-138 ° C.

Exemple 110

Acide 4 [(trifluorométhyl-3 phényl)-1 dihydro-1,2 oxo-2 naphtyridine-1,8 yl-3] butanoïque

Formule I X = 3-$CF_3$, Y = H, Z = $CH_2$-$CH_2$-$CH_2$-COOH

On porte à 170-180 ° C durant 1 h 15 min un mélange de 10 g de (trifluorométhyl-3 phényl)amino-2 nicotinaldéhyde synthétisés précédemment, 24 g d'adipate de sodium et 11,2 ml de chlorure d'adipoyle dans 60 ml de N-méthylpyrrolidone.

Le mélange réactionnel est ensuite refroidi, additionné d'eau et de glace puis basifié avec une solution de soude 10 %. Les produits neutres sont extraits à l'éther. La phase aqueuse est ensuite acidifiée à froid par de l'acide acétique et est extraite à l'éther qu'on lave à l'eau et qu'on sèche sur sulfate de sodium. Après évaporation du solvant, le résidu obtenu est chromatographié sur gel de silice. Par élution avec un mélange dichlorométhane 99,5 et acide acétique 0,5, on obtient un résidu qui cristallise dans l'éther isopropylique. Après essorage des cristaux, lavage et recristallisation dans l'acétonitrile, on obtient 4,5 g d'acide 4-[(trifluorométhyl-3 phényl)-1 dihydro-1,2 oxo-2 naphtyridine-1,8 yl-3]butanoïque sous forme de cristaux de point de fusion 165-166 ° C.

Exemple 111

Acide 5 (phényl-1 dihydro-1,2 oxo-2 naphtyridine-1,8 yl-3)pentanoïque

Formule I X = Y = H, Z = $CH_2$-$CH_2$-$CH_2$-$CH_2$-COOH

Préparé selon le mode opératoire de l'exemple 109
Cristaux (acétonitrile) F = 187-188 ° C ; rendement 23 %

Exemple 112
Acide 4 (phényl-1 dihydro-1,2 oxo-2 naphtyridine-1,8 yl-3) butanoïque

Formule I X = Y = H, Z = $CH_2$-$CH_2$-$CH_2$-COOH

Préparé selon le mode opératoire de l'exemple 110
Cristaux (acétonitrile) F = 178-179 ° C ; rendement 20 %.

Selon le mode opératoire de l'exemple 109 ont été encore synthétisés les exemples 113 à 119 suivants :

Exemple 113
Acide 6 [(trifluorométhyl-3 phényl)-1 dihydro-1,2 oxo-2 naphtyridine-1,8 yl-3] hexanoïque

Formule I X = 3-$CF_3$,Y = H, Z = $CH_2$-$CH_2$-$CH_2$-$CH_2$-$CH_2$-COOH

Cristaux F = 126-127 ° C ; rendement 28 %

Exemple 114
Acide 6 (phényl-1 dihydro-1,2 oxo-2 naphtyridine-1,8 yl-3) hexanoïque

Formule I X = Y = H, Z = $CH_2$-$CH_2$-$CH_2$-$CH_2$-$CH_2$-COOH

Cristaux (acétonitrile) F = 157-158 ° C ; rendement 28 %

Exemple 115
Acide 5 [(chloro-3 phényl)-1 dihydro-1,2 oxo-2 naphtyridine-1,8 yl-3] pentanoïque

Formule I X = 3-Cl, Y = H, Z = $CH_2$-$CH_2$-$CH_2$-$CH_2$-COOH

Cristaux (acétonitrile) F = 160-161 ° C ; rendement 20 %

Exemple 116
Acide 5 [(méthylthio-3 phényl)-1 dihydro-1,2 oxo-2 naphtyridine-1,8 yl-3] pentanoïque

Formule I X = 3-$SCH_3$, Y = H, Z = $CH_2$-$CH_2$-$CH_2$-$CH_2$-COOH

Cristaux (méthanol) F = 158-160 ° C ; rendement 20 %

Exemple 117
Acide 5 [(nitro-3 phényl)-1 dihydro-1,2 oxo-2 naphtyridine-1,8 yl-3] pentanoïque

Formule I X = 3-$NO_2$, Y = H, Z = $CH_2$-$CH_2$-$CH_2$-$CH_2$-COOH

Cristaux (éthanol) F = 183-185 ° C ; rendement 20 %

Exemple 118
Acide 5 [(fluoro-4 phényl)-1 dihydro-1,2 oxo-2 naphtyridine-1,8 yl-3] pentanoïque

Formule I X = 4-F, Y = H, Z = $CH_2$-$CH_2$-$CH_2$-$CH_2$-COOH

Cristaux (acétonitrile-acétone) F = 200-202 ° C ; rendement 21 %

Exemple 119

Acide 5 [(cyano-3 phényl)-1 dihydro-1,2 oxo-2 naphtyridine-1,8 yl-3] pentanoïque

Formule I X = 3-CN, Y = H, Z = $CH_2$-$CH_2$-$CH_2$-$CH_2$-COOH

Cristaux (diméthylformamide) F = 193-194 ° C ; rendement 13 %

Exemple 120
(phényl-1 dihydro-1,2 oxo-2 naphtyridine-1,8 yl-3)-1 [(méthoxy-2 phényl)-4 pipérazinyl-1]-3 propane

Formule I X = Y = H, Z = $CH_2-CH_2-CH_2-$ 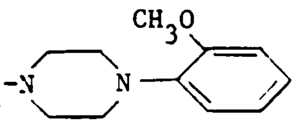

On porte au reflux durant 3 h une solution de 7 g de (phényl-1 dihydro-1,2 oxo-2 naphtyridine-1,8 yl-3) bromo-3 propane préparés à l'exemple 104 et de 3,9 g de (méthoxy-2 phényl)-1 pipérazine dans 100 ml de xylène contenant 2,8 ml de triéthylamine. Le mélange réactionnel est ensuite refroidi, additionné d'eau et de glace et extrait au chloroforme. La phase chloroformique est ensuite extraite avec une solution d'acide chlorhydrique 10 %. La phase aqueuse acide est ensuite basifiée à froid avec une solution de soude 10 %, puis extraite au dichlorométhane qu'on lave à l'eau, sèche sur sulfate de sodium et qu'on évapore. Le résidu obtenu cristallise et, après recristallisation dans l'isopropanol, on récupère 4,8 g de (phényl-1 dihydro-1,2 oxo-2 naphtyridine-1,8 yl-3)-1 [(méthoxy-2 phényl)-4 pipérazinyl-1]-3 propane sous forme de cristaux de point de fusion 112-113 ° C.

Exemple 121
(phényl-1 dihydro-1,2 oxo-2 naphtyridine-1,8 yl-3) bromo-2 éthane

Formule I X = Y = H, Z = $CH_2$-$CH_2$-Br

Préparé selon le mode opératoire de l'exemple 104
Cristaux F = 206 ° C ; rendement 90 %

Exemple 122
(phényl-1 dihydro-1,2 oxo-2 naphtyridine-1,8 yl-3)-1 [(méthoxy-2 phényl)-4 pipérazinyl-1]-2 éthane

Formule I X = Y = H, Z = $CH_2-CH_2-$ 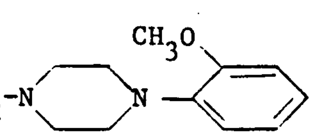

Préparé selon le mode opératoire de l'exemple 120
Cristaux (isopropanol) F = 129-131 ° C ; rendement 50 %

Exemple 123
(phényl-1 dihydro-1,2 oxo-2 naphtyridine-1,8 yl-3)-1 (imidazolyl-1)-2 éthane

Formule I X = Y = H, Z = $CH_2-CH_2$ 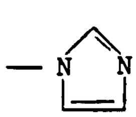

2,4 g d'imidazole sont ajoutés par petites quantités à une suspension de 0,8 g d'hydrure de sodium dans 550 ml de diméthylformamide après la fin de l'addition, le mélange est porté 1 h à 90 ° C puis laissé revenir à température ambiante pour additionner une solution de 10,7 g (phényl-1 dihydro-1,2 oxo-2

naphtyridine-1,8 yl-3) bromo-2 éthane préparés à l'exemple 121 dans 100 ml de diméthylformamide. Après la fin de l'addition, le mélange est porté 1 h à 90 ° C puis concentré sous vide. Le résidu repris à l'eau cristallise et les cristaux obtenus après essorage et lavage à l'eau sont dissous dans une solution d'acide chlorhydrique.

La phase aqueuse est lavée à l'acétate d'éthyle puis basifiée à froid par une solution de soude 10 %. Les cristaux formés sont essorés, lavés à l'eau avec un peu d'acétone et séchés. Après recristallisation dans l'acétonitrile, on récupère 3,8 g de (phényl-1 dihydro-1,2 oxo-2 naphtyridine-1,8 yl-3)-1 (imidazolyl-1)-2 éthane sous forme de cristaux de point de fusion 208-209 ° C.

Exemple 124
(phényl-1 dihydro-1,2 oxo-2 naphtyridine-1,8 yl-3) [méthyl-4 pipérazinyl-1] méthane

Formule I X = Y = H, Z = 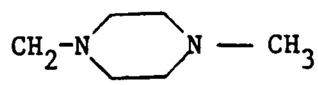

Préparé selon le mode opératoire de l'exemple 38 en utilisant le β-(méthyl-4 pipérazinyl-1) propionate d'éthyle
Cristaux (isopropanol) cristallisés avec 1 mole d'eau F = 145-146 ° C ; rendement 8 %

Exemple 125
Acide (phényl-1 dihydro-1,2 oxo-2 naphtyridine-1,8 yl-3)-3 (chloro-4 benzoyl)amino-2 propionique

Formule I X = Y = H, Z = 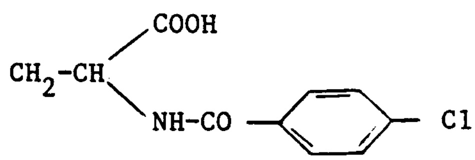

Préparé selon le mode opératoire de l'exemple 7 en utilisant l'ester diméthylique de l'acide N-(chloro-4 benzoyl)glutamique
Cristaux (acétonitrile) F = 197-199 ° C ; rendement 8 %

Exemple 126
Acide (phényl-1 dihydro-1,2 oxo-2 naphtyridine-1,8 yl-3)-2 (chloro-4 benzoyl)amino-2 acétique

Formule I X = Y = H, Z = 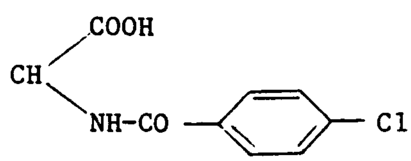

Préparé selon le mode opératoire de l'exemple 7 en utilisant l'ester diméthylique de l'acide N-(chloro-4 benzoyl)aspartique
Cristaux (acide acétique) F = 236-237 ° C ; rendement 7 %

Exemple 127
Ester éthylique de l'acide (phényl-1 dihydro-1,2 oxo-2 naphtyridine-1,8 yl-3)-5 (acétamido)-2 (éthoxycarbonyl)-2 pentanoïque

Formule I X = Y = H, Z = 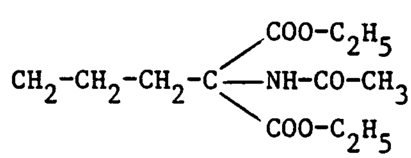

A une solution au reflux de 5,7 g d'acétamidomalonate de diéthyle préalablement métallés par 0,7 g de sodium dans 70 ml d'éthanol, on ajoute par petites quantités 8,1 g de (phényl-1 dihydro-1,2 oxo-2 naphtyridine-1,8 yl-3) bromo-3 propane préparés à l'exemple 104.

Après la fin de l'addition, le reflux est continué pendant 2 h puis le mélange réactionnel est concentré sous vide, le résidu repris par un mélange d'eau et de glace est extrait à l'acétate d'éthyle qu'on lave à l'eau puis qu'on sèche sur sulfate de sodium. Après évaporation de l'acétate d'éthyle, le résidu obtenu cristallise dans l'éther. Les cristaux essorés sont recristallisés dans l'éthanol conduisant à 4,7 g d'ester éthylique de l'acide (phényl-1 dihydro-1,2 oxo-2 naphtyridine-1,8 yl-3)-5 (acétamido)-2 (éthoxycarbonyl)-2 pentanoïque sous forme de cristaux de point de fusion 180 ° C.

Exemple 128

Chlorhydrate de l'acide (phényl-1 dihydro-1,2 oxo-2 naphtyridine-1,8 yl-3)-5 amino-2 pentanoïque

Formule I X = Y = H, Z = 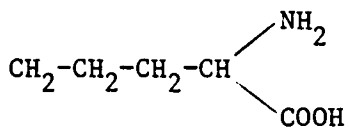

On porte au reflux durant 9 h une solution de 4,7 g d'ester éthylique de l'acide (phényl-1 dihydro-1,2 oxo-2 naphtyridine-1,8 yl-3)-5 (acétamido)-2 (éthoxycarbonyl)-2 pentanoïque préparés à l'exemple 127 dans 110 ml d'acide chlorhydrique 20 %. Le mélange réactionnel est ensuite concentré sous vide, le résidu obtenu repris à l'éther et les cristaux ainsi formés essorés puis séchés. Après recristallisation dans un mélange acétonitrile-eau 8-2, on récupère 2,4 g de chlorhydrate de l'acide (phényl-1 dihydro-1,2 oxo-2 naphtyridine-1,8 yl-3)-5 amino-2 pentanoïque sous forme de cristaux de point de fusion 203-205 ° C.

Exemple 129

(phényl-1 dihydro-1,2 oxo-2 naphtyridine-1,8 yl-3)-3 diéthylphosphonopropane

Formule I X = Y = H, Z = 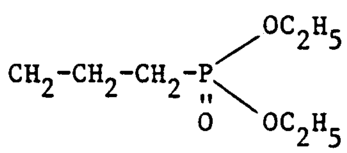

On porte à 120-130 ° C durant 6 h un mélange de 7,3 g de (phényl-1 dihydro-1,2 oxo-2 naphtyridine-1,8 yl-3) bromo-3 propane préparés à l'exemple 104 et 28 ml de triéthylphosphite. Le mélange réactionnel est ensuite évaporé à sec sous vide. Le résidu obtenu cristallise dans l'éther isopropylique. Les cristaux essorés, lavés avec l'éther isopropylique et séchés, on obtient 8 g de dérivé qui sont filtrés sur gel de silice. Avec le mélange d'éluant dichlorométhane-méthanol 9-1, on récupère 7 g de (phényl-1 dihydro-1,2 oxo-2 naphtyridine-1,8 yl-3)-3 diéthylphosphonopropane sous forme de cristaux de point de fusion 119-121 ° C.

TABLEAU I

5145-18 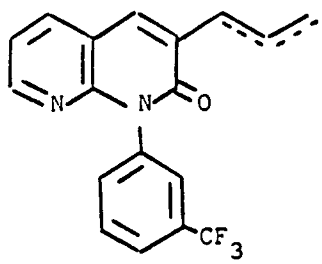 Exemple 4

5145-19 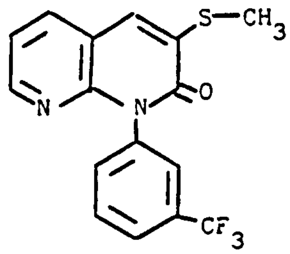 Exemple 5

5145-20 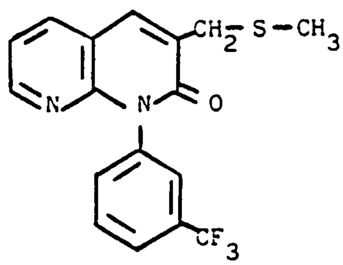 Exemple 6

5145-23 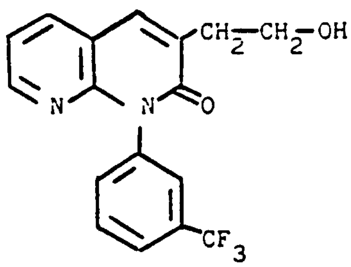 Exemple 7

5145-24 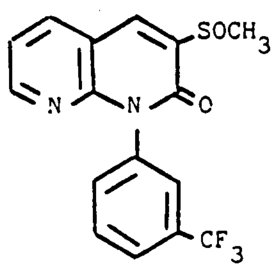 Exemple 8

5145-25

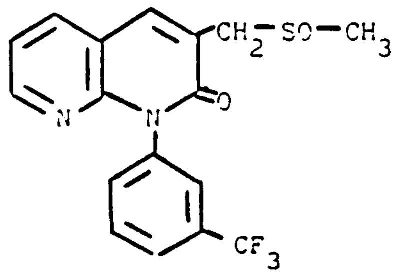

Exemple 9

5145-39

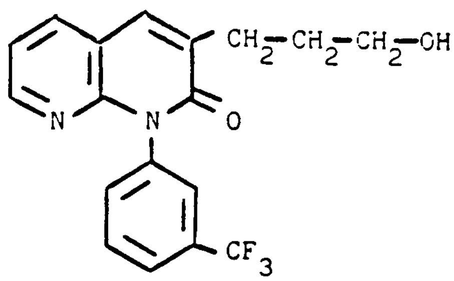

Exemple 10

5145-40

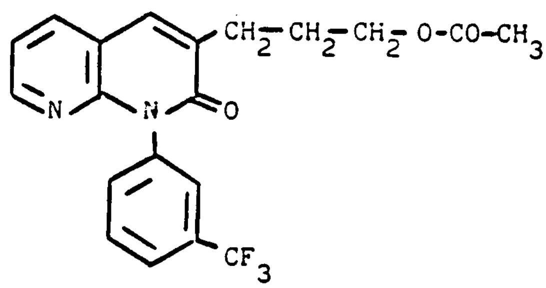

Exemple 11

5145-41

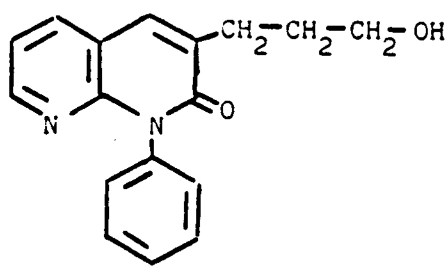

Exemple 12

5145-42

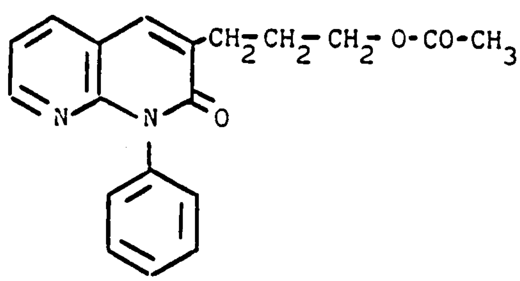

Exemple 13

5145-43

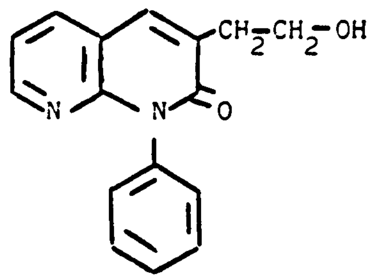

Exemple 14

5145-44

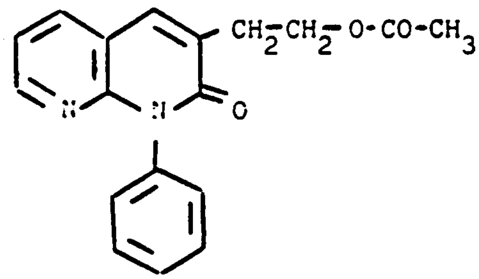

Exemple 15

5145-46

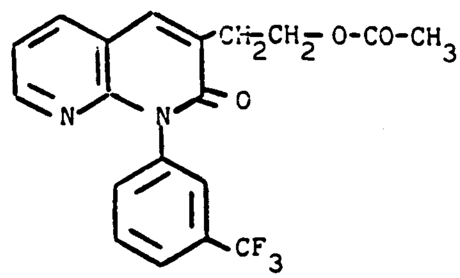

Exemple 16

5145-47

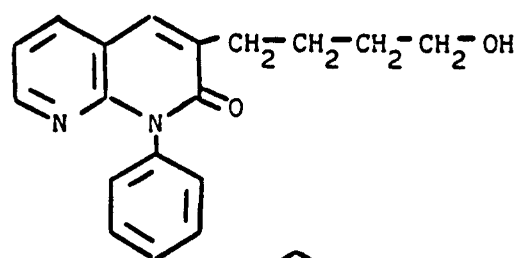

Exemple 17

5145-49

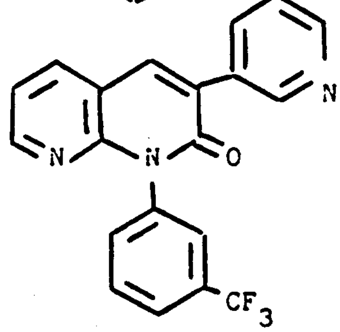

Exemple 18

5145-50

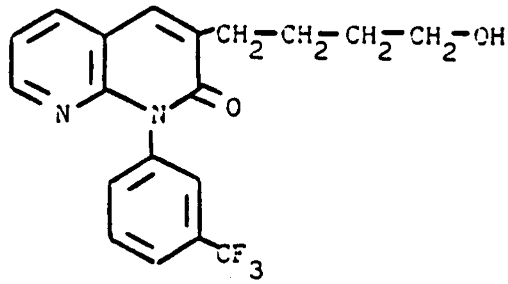

Exemple 19

5145-51

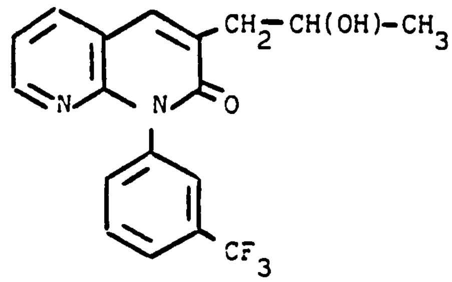

Exemple 20

5145-52

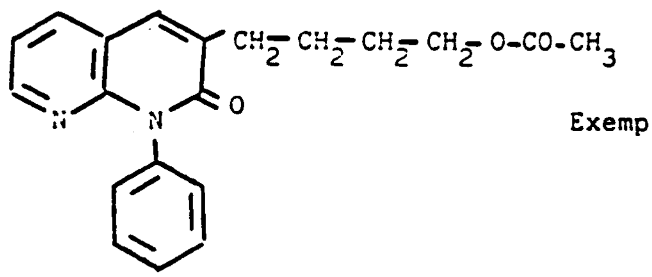

Exemple 21

5145-54

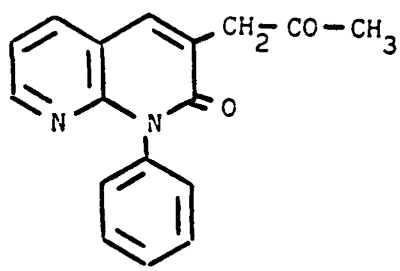

Exemple 23

5145-58

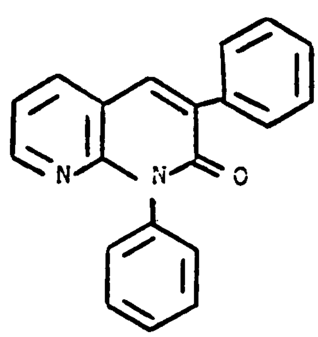

Exemple 24

5145-59

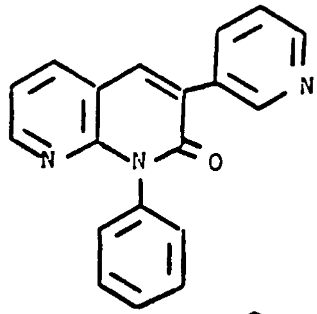

Exemple 25

5145-61

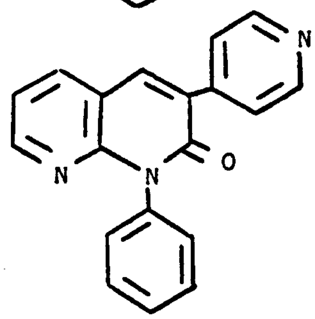

Exemple 26

5145-62

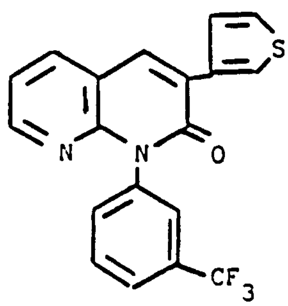

Exemple 27

5145-64

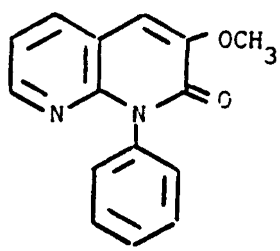

Exemple 28

5145-65

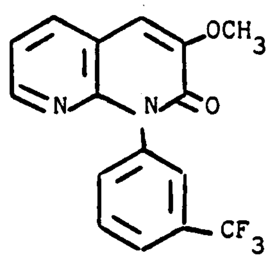

Exemple 29

5145-66

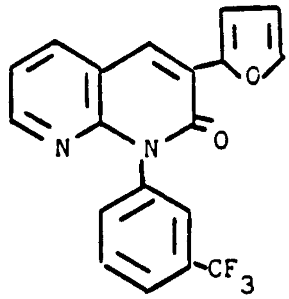

Exemple 30

5145-70

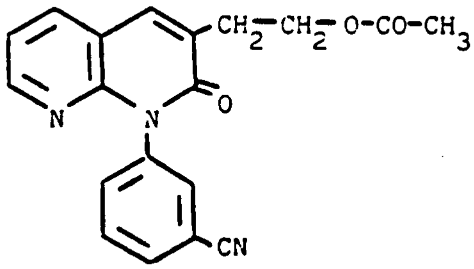

Exemple 32

5145-71

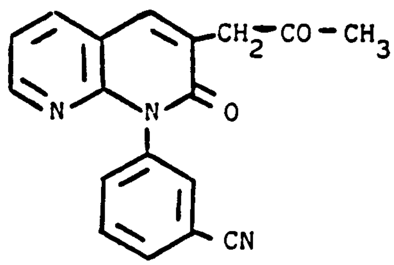

Exemple 34

5145-72

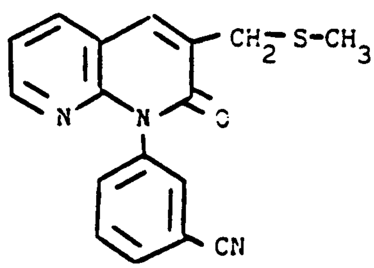

Exemple 35

5145-74

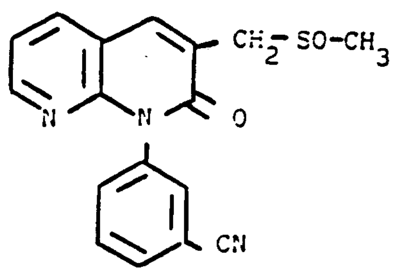

Exemple 36

5145-76

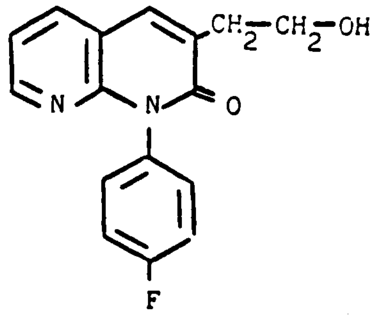

Exemple 37

5145-77

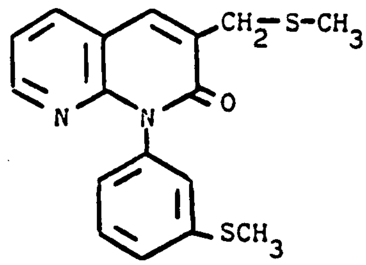

Exemple 38

5145-78

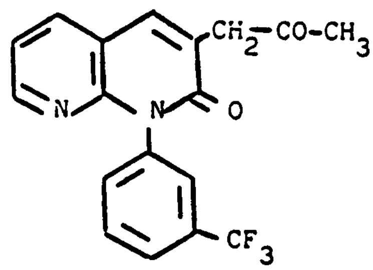

Exemple 39

5145-79

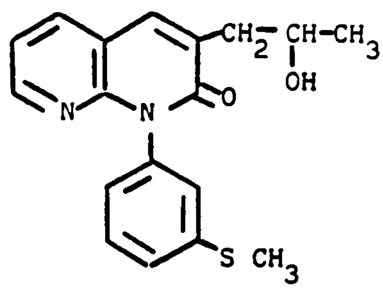

Exemple 40

5145-81

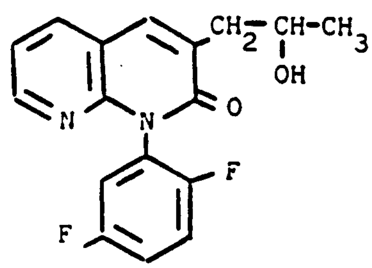

Exemple 41

5145-80

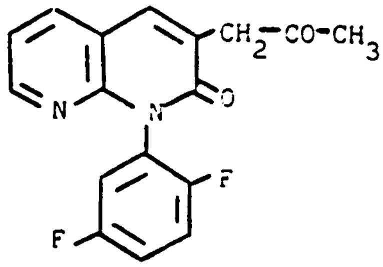

Exemple 42

5145-82

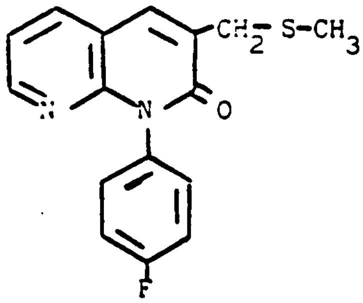

Exemple 43

5145-83

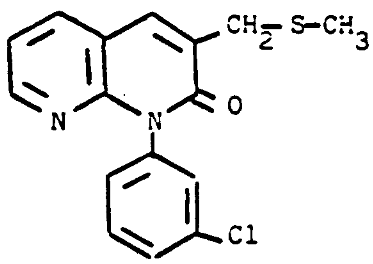

Exemple 44

5145-84

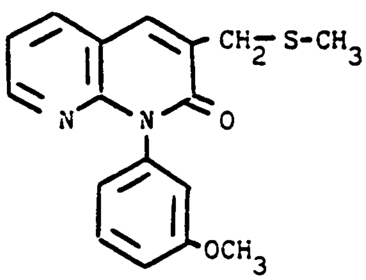

Exemple 45

5145-85

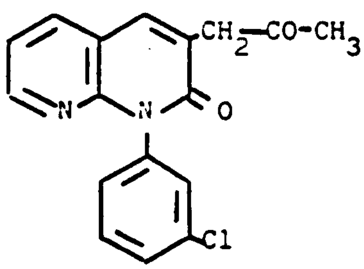

Exemple 47

5145-86

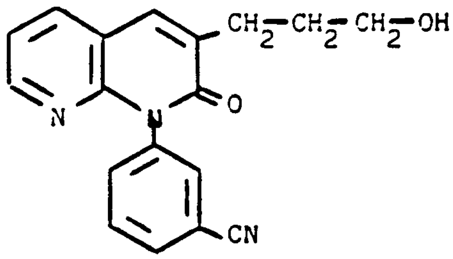

Exemple 48

5145-87

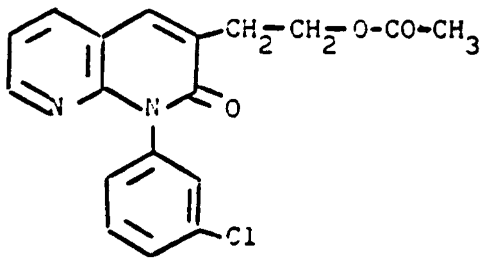

Exemple 50

5145-88

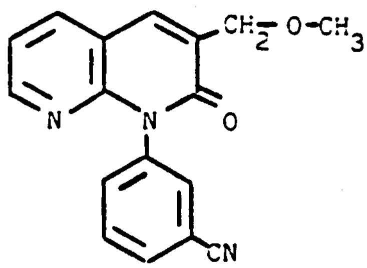

Exemple 51

5145-89

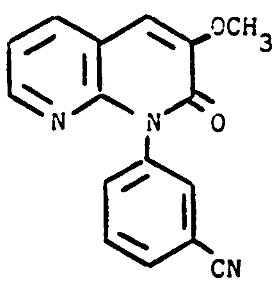

Exemple 52

5145-90

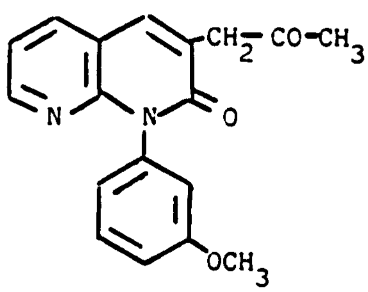

Exemple 54

5145-91

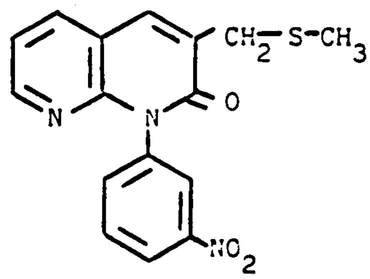

Exemple 55

5145-92

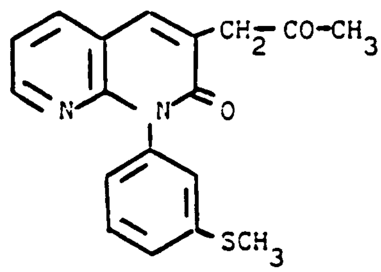

Exemple 56

5145-93

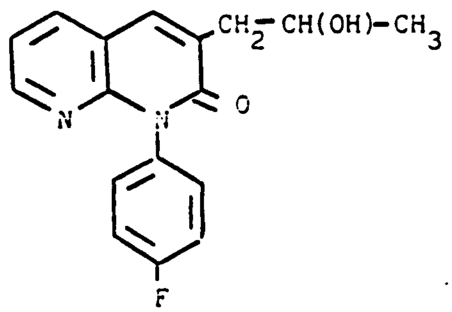

Exemple 57

5145-94

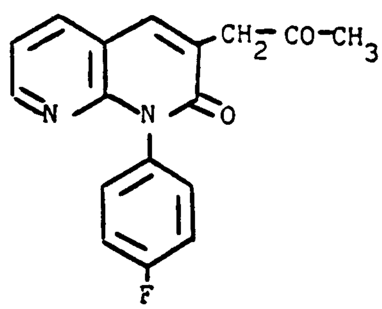

Exemple 58

5145-95

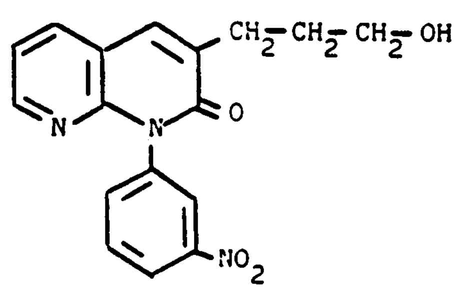

Exemple 59

5145-96

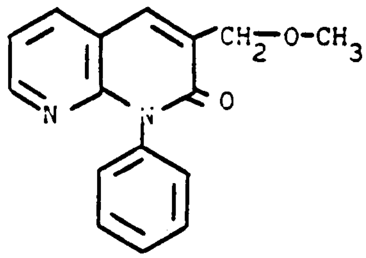

Exemple 60

5145-97

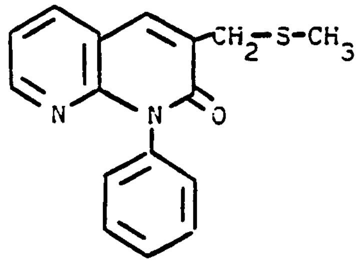

Exemple 61

5145-99

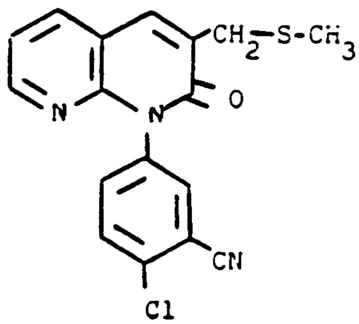

Exemple 62

5145-100

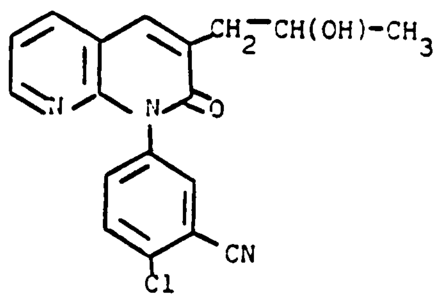

Exemple 63

5145-101

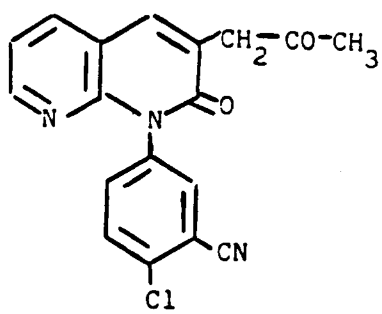

Exemple 64

5145-102

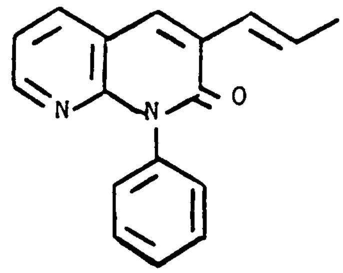

Exemple 65

5145-103

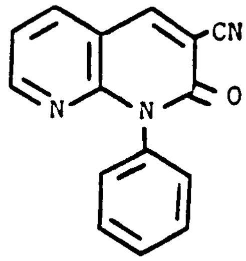

Exemple 66

5145-105

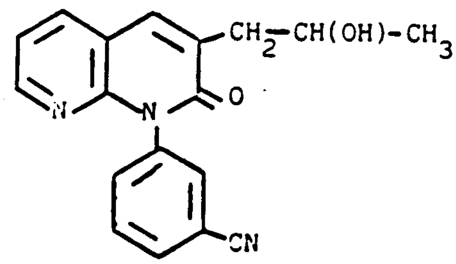

Exemple 33

5145-106

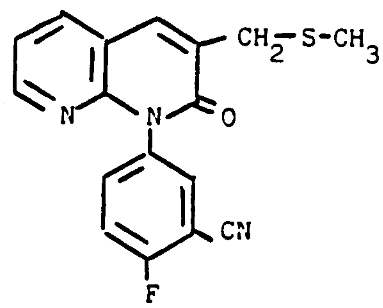

Exemple 67

5145-107

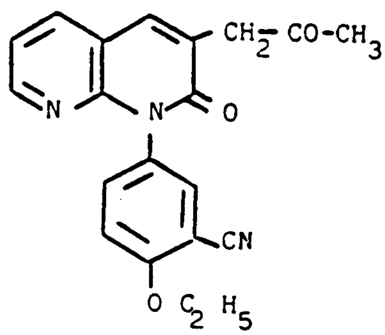

Exemple 68

5145-108

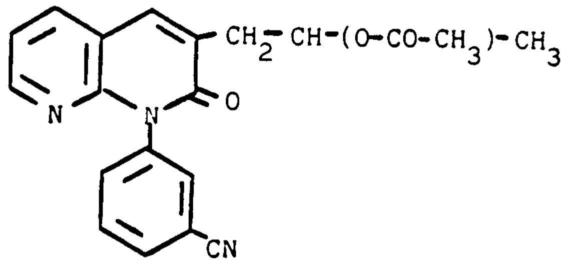

Exemple 69

5145-109

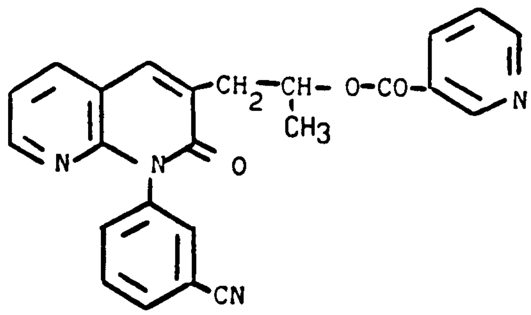

Exemple 70

5145-110

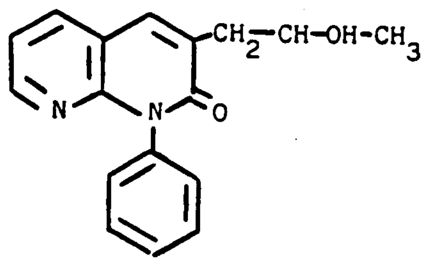

Exemple 22

5145-111

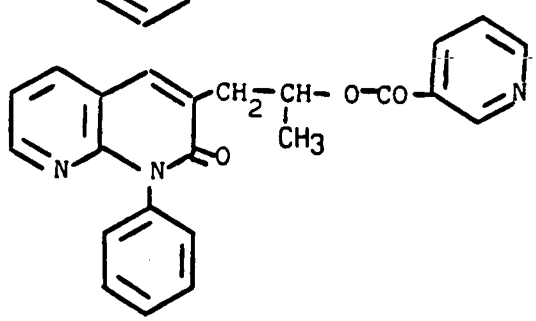

Exemple 71

5145-112

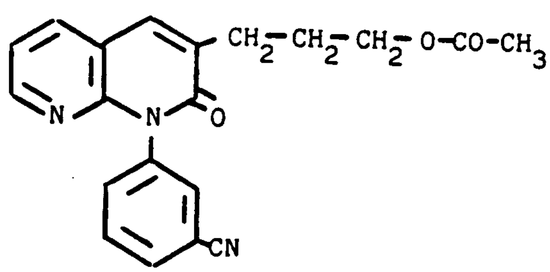

Exemple 72

5145-113

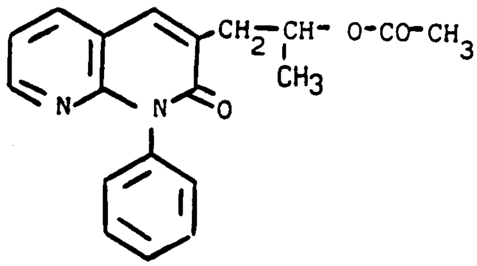

Exemple 73

5145-114

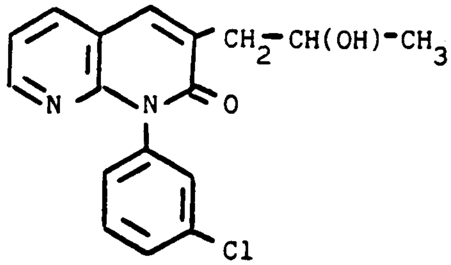

Exemple 46

5145-115

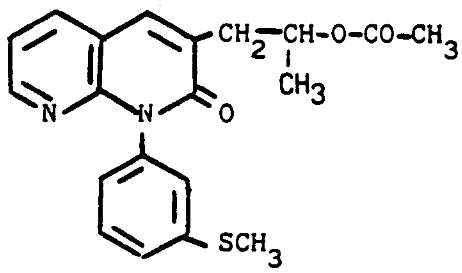

Exemple 74

5145-116

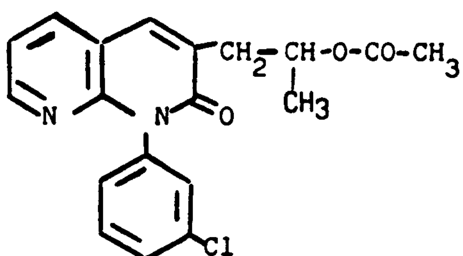

Exemple 75

5145-117

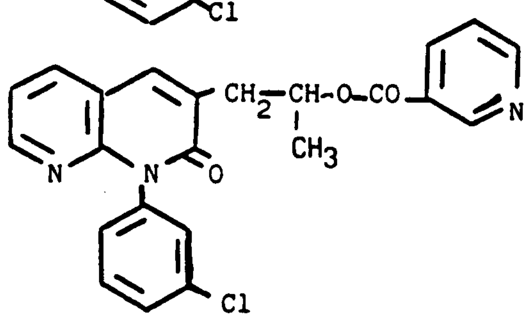

Exemple 76

5145-118

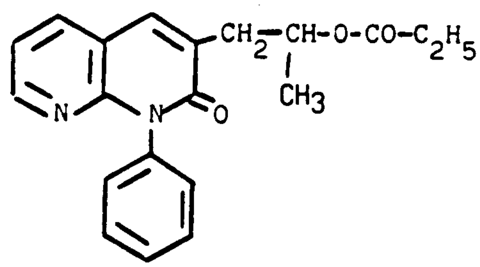

Exemple 77

5145-119

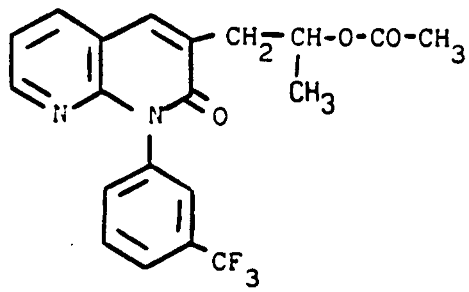

Exemple 78

5145-121

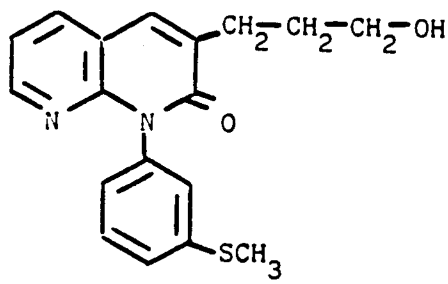

Exemple 79

5145-122

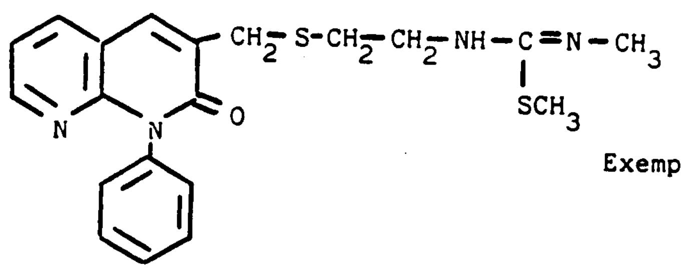

Exemple 80

5145-123

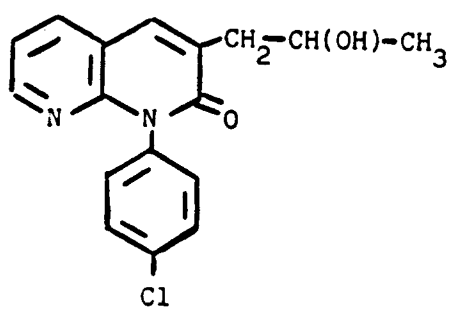

Exemple 81

5145-124

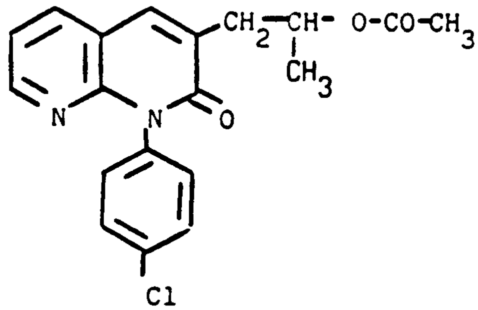

Exemple 82

5145-125

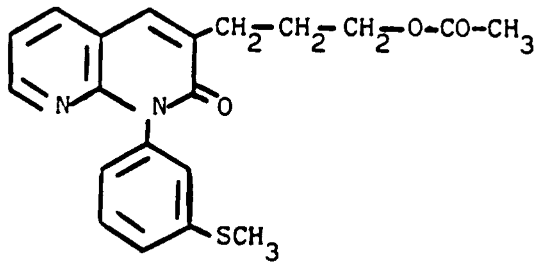

Exemple 83

5145-126

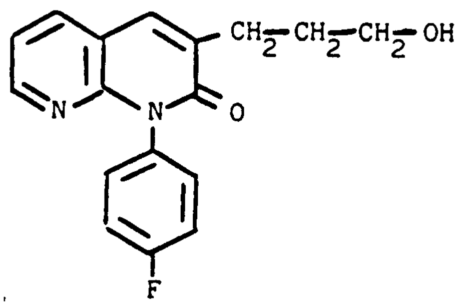

Exemple 84

5145-127

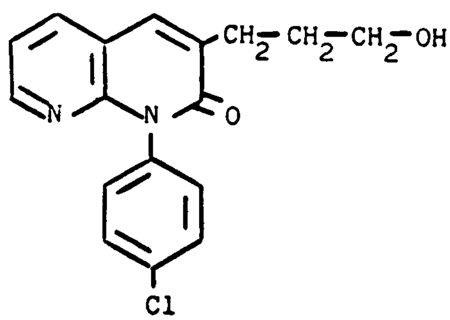

Exemple 85

5145-128

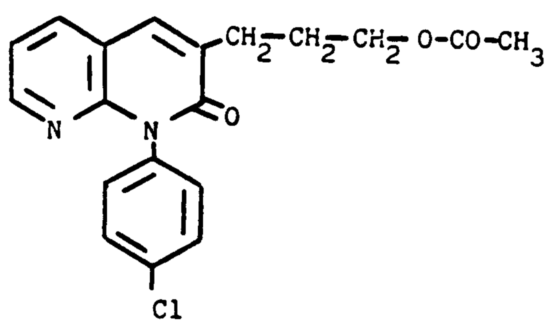

Exemple 86

5145-129

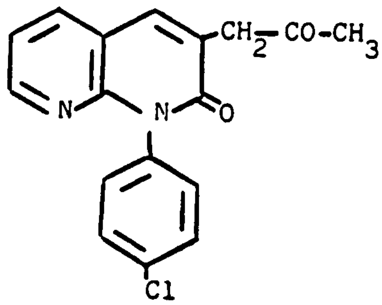

Exemple 87

5145-130

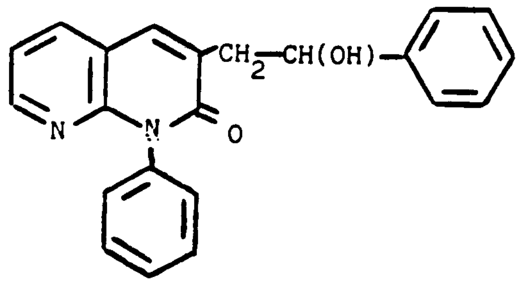

Exemple 88

5145-131

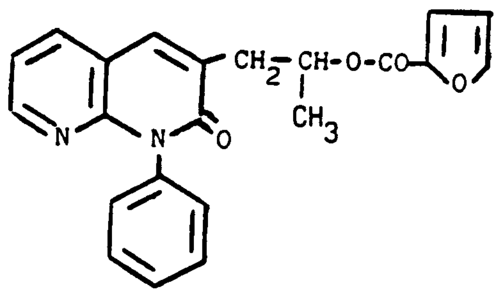

Exemple 89

5145-132

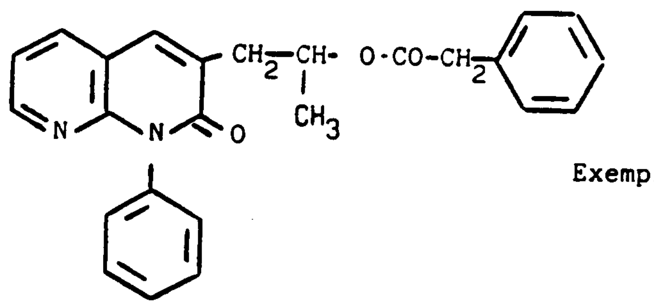

Exemple 90

5145-133

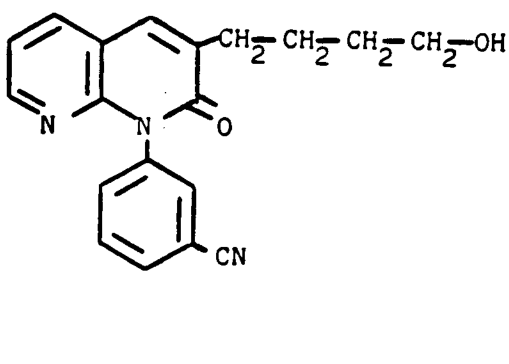

Exemple 91

5145-134

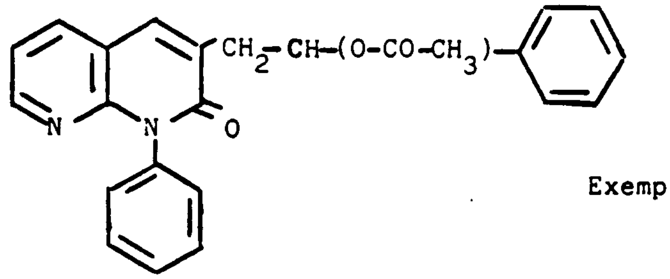

Exemple 92

5145-135

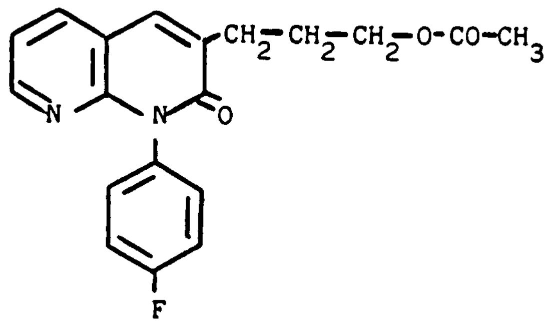

Exemple 93

5145-136

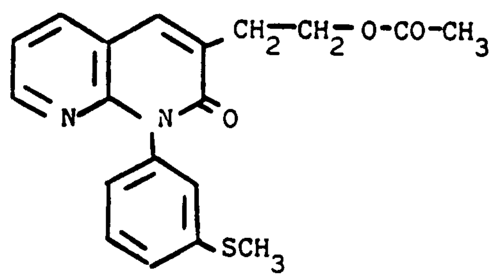

Exemple 94

5145-137

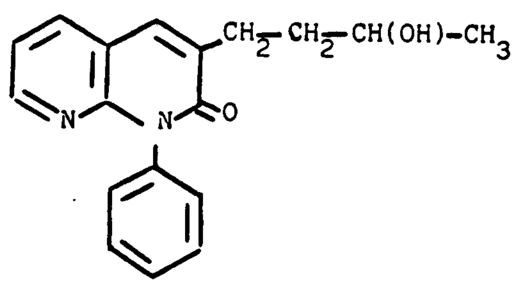

Exemple 95

5145-139

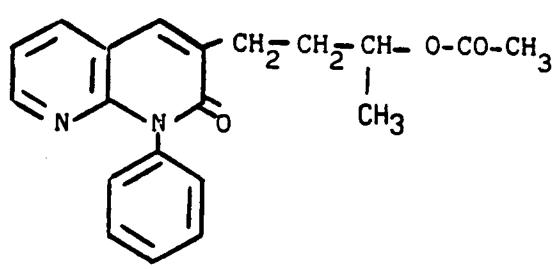

Exemple 96

5145-140

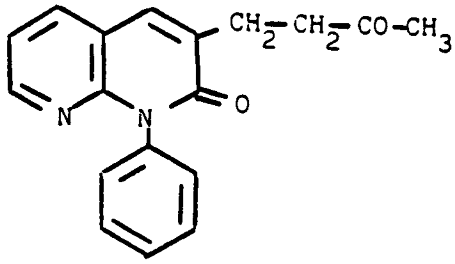

Exemple 97

5145-141

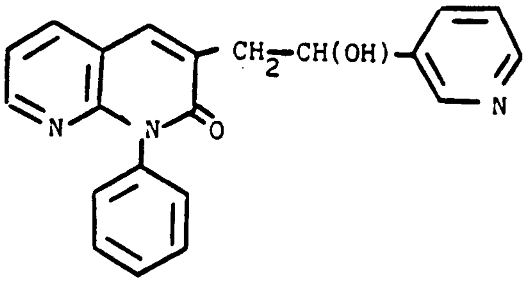

Exemple 98

5145-142

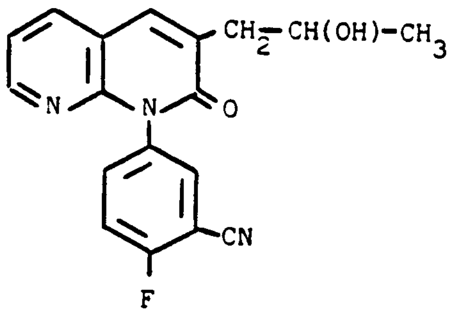

Exemple 99

5145-143

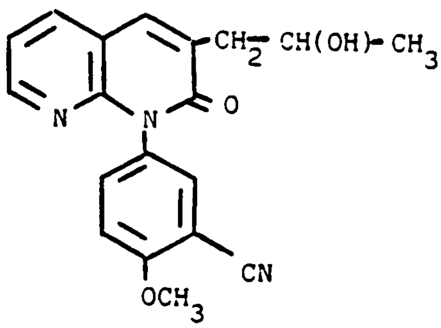

Exemple 100

5145-144

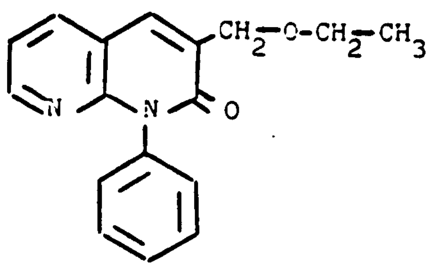

Exemple 101

5145-145

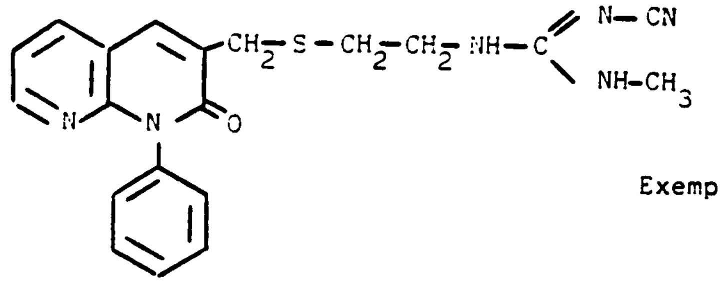

Exemple 102

5145-146

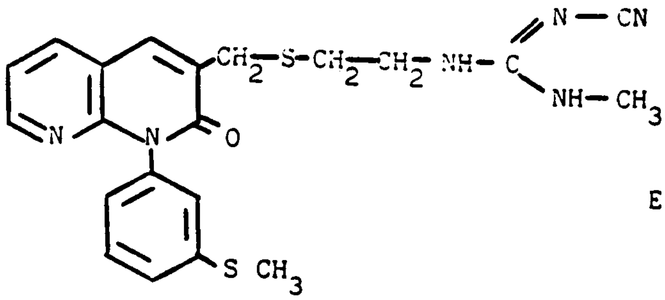

Exemple 103

5145-147

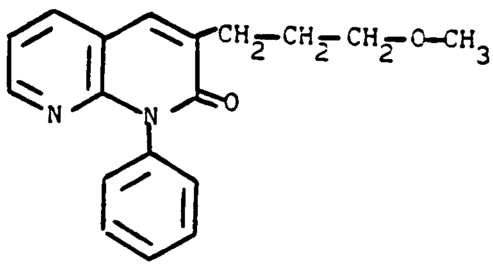

Exemple 105

5145-148

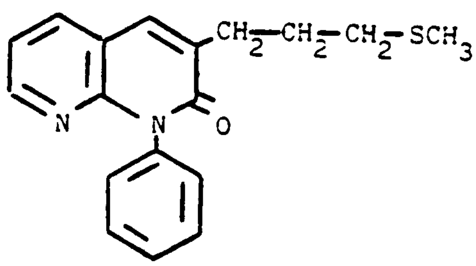

Exemple 106

5145-149

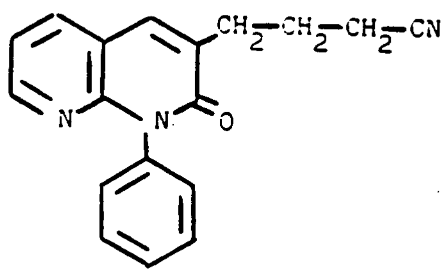

Exemple 107

5145-151

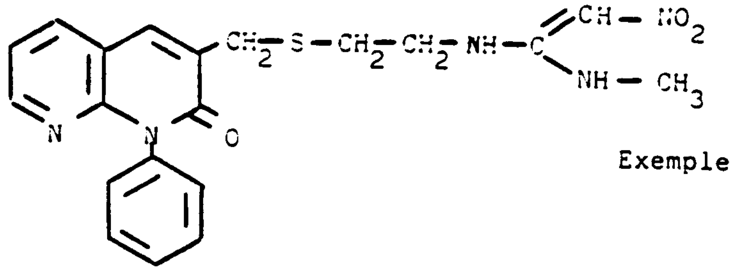

Exemple 108

5196-05

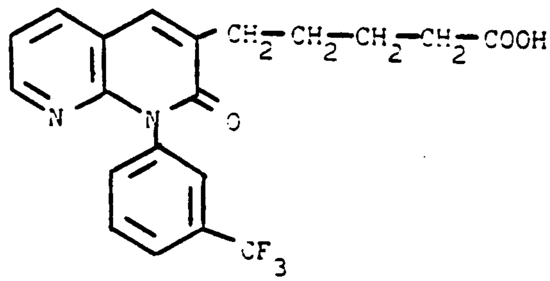

Exemple 109

5196-06

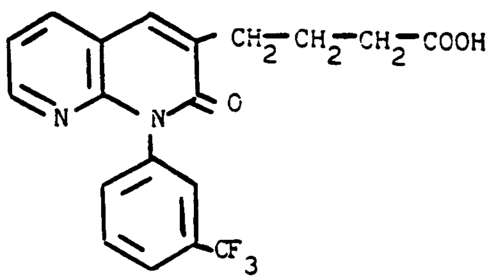

Exemple 110

5196-07

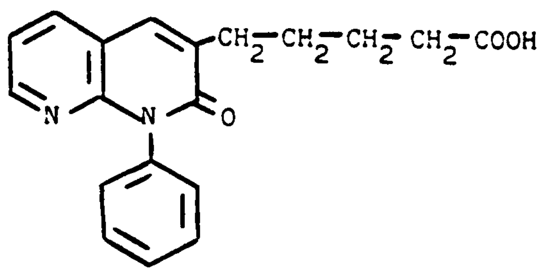

Exemple 111

5196-08

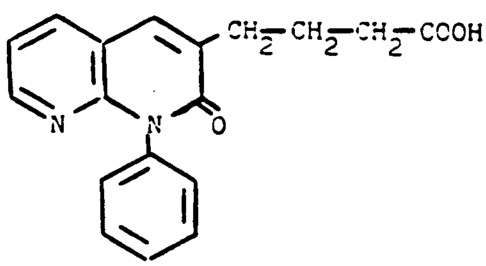

Exemple 112

5196-09

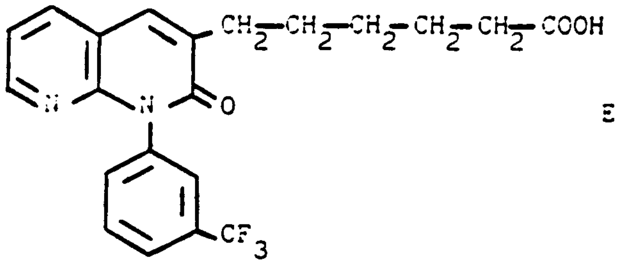

Exemple 113

5196-10

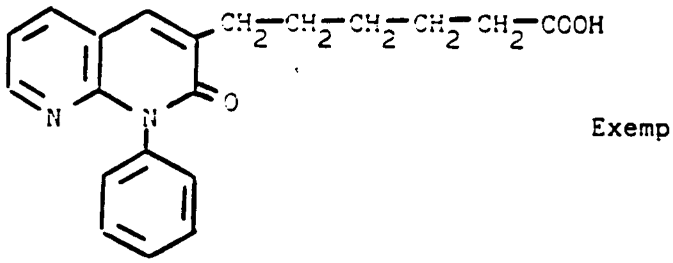

Exemple 114

5196-13

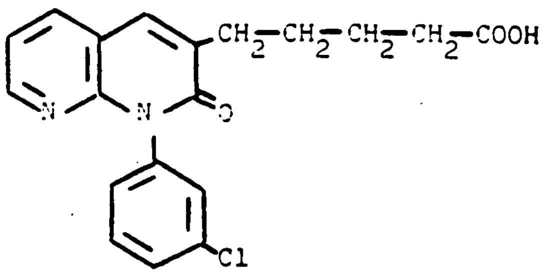

Exemple 115

5196-14

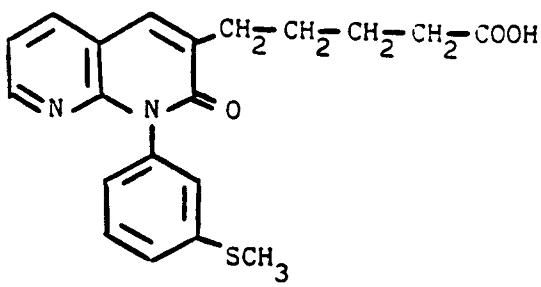

Exemple 116

5196-15

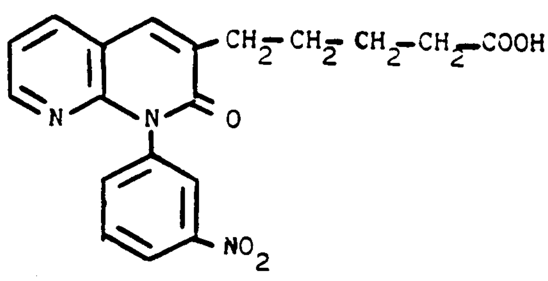

Exemple 117

5196-16

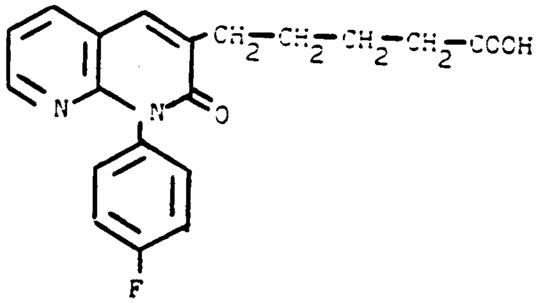

Exemple 118

5196-17

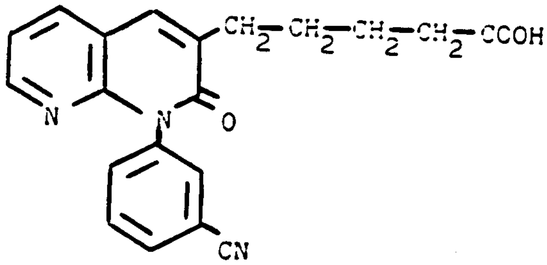

Exemple 119

37-1

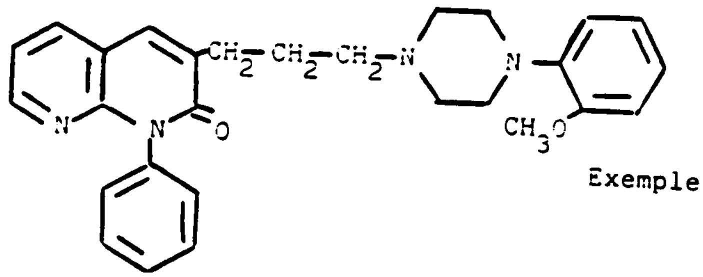

Exemple 120

37-3

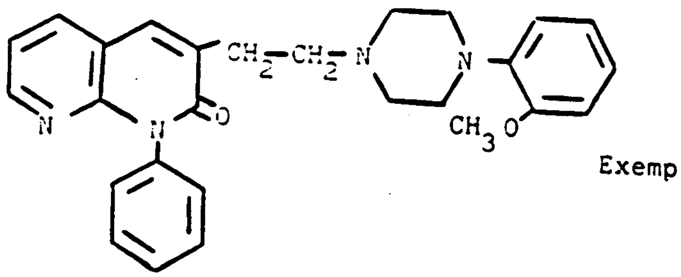

Exemple 122

37-4

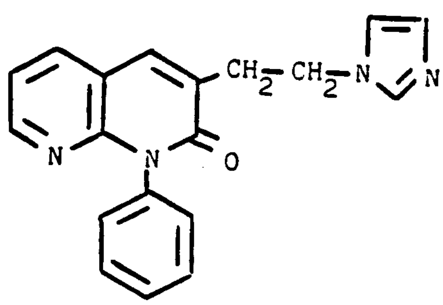

Exemple 123

37-5

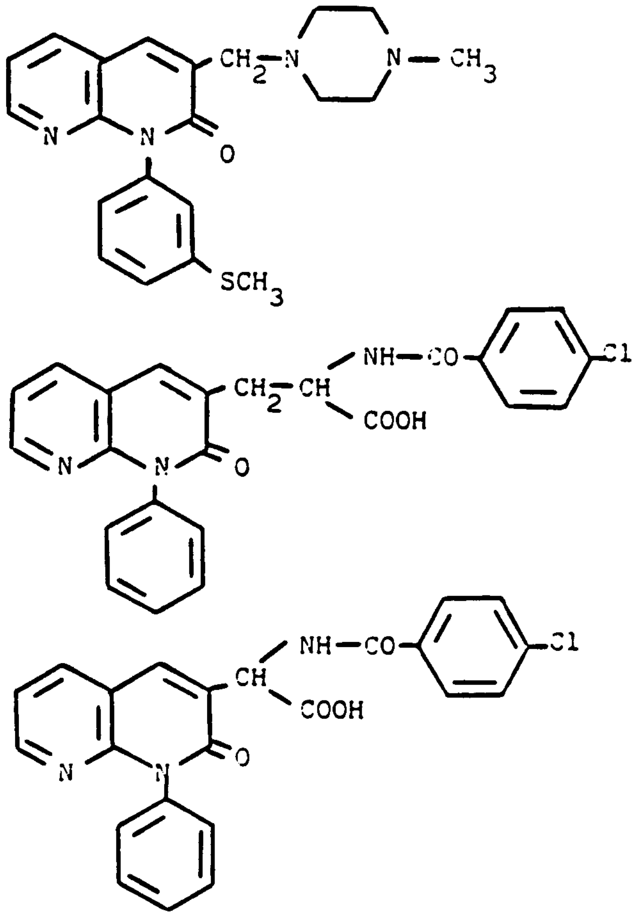

Exemple 124

63-1

Exemple 125

63-2

Exemple 126

63-3

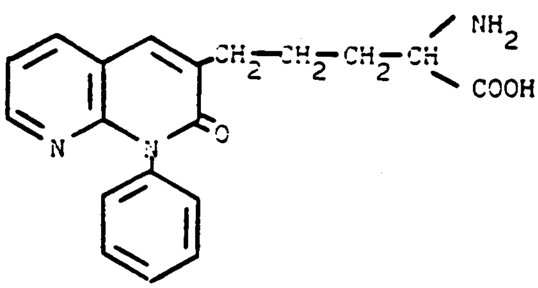

Exemple 128

63-4

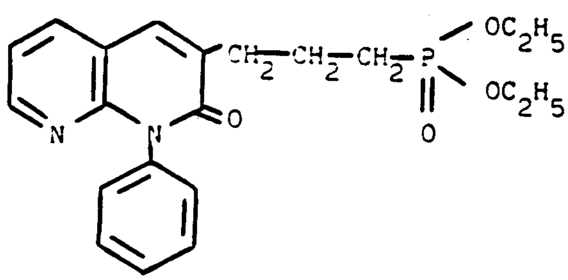

Exemple 129

PHARMACOLOGIE

ACTIVITE ANTI-ULCERE

1. Méthode

Des lots de 10 à 30 rats mâles de souche OFA (provenance IFFA CREDO, France) pesant 160-180 g

sont mis à la diète hydrique 24 h avant l'administration orale du produit à étudier.

Trente minutes après le gavage, un agent ulcérigène médicamenteux ou un agent nécrosant (éthanol absolu) est administré par voie orale à des doses, qui entraînent chez les animaux témoins, une ulcération gastrique maximale. Les estomacs sont ensuite prélevés selon le test, respectivement 6 h et 1 h après le dernier traitement. Les lésions gastriques sont alors évaluées macroscopiquement (cotation et mesure de l'ampleur des ulcères).

2. Résultats

Les résultats sont exprimés sous forme de doses actives 50 (dose inhibant de 50 % les lésions provoquées) déterminées graphiquement à partir de la droite exprimant la relation entre les pourcentages d'inhibition et les doses utilisées.

DOSE ACTIVE 50

(exprimée en mg.kg-1, administration par voie orale)

| | 1) Administration d'un agent médicamenteux ulcérigène | 2) Administration d'un agent nécrosant |
|---|---|---|
| Exemple 6 | 1,4 | 0,09 |
| Exemple 7 | 2,95 | 0,180 |
| Exemple 9 | 0,105 | 0,44 |
| Exemple 10 | 16 | 0,35 |
| Exemple 12 | 3 | 0,13 |
| Exemple 13 | 3,8 | 0,18 |
| Exemple 14 | 1,1 | 0,09 |
| Exemple 15 | 1 | 0,65 |
| Exemple 16 | 4,5 | 0,29 |
| Exemple 17 | 7,5 | 0,22 |
| Exemple 19 | 4,5 | 0,07 |
| Exemple 20 | 0,9 | 0,018 |
| Exemple 21 | 7,2 | 0,8 |
| Exemple 22 | 0,120 | 0,008 |

DOSE ACTIVE 50

(exprimée en mg.kg-1, administration par voie orale)

| | 1) Administration d'un agent médicamenteux ulcérigène | 2) Administration d'un agent nécrosant |
|---|---|---|
| Exemple 23 | 0,150 | 0,002 |
| Exemple 24 | 1,9 | 2,4 |
| Exemple 25 | 3,4 | 0,036 |
| Exemple 26 | 1,6 | 0,35 |
| Exemple 32 | 0,9 | 0,015 |
| Exemple 33 | 0,019 | 0,008 |
| Exemple 36 | 0,820 | 0,075 |
| Exemple 38 | 0,240 | 0,016 |
| Exemple 39 | 1 | 0,115 |
| Exemple 40 | 0,850 | 0,027 |
| Exemple 41 | 1,4 | 0,070 |
| Exemple 42 | 2 | 0,090 |
| Exemple 43 | 1,5 | 0,033 |
| Exemple 44 | 0,280 | 0,110 |
| Exemple 45 | 0,940 | 0,035 |
| Exemple 47 | 0,145 | 0,007 |
| Exemple 48 | 1,65 | 0,090 |
| Exemple 50 | 0,550 | 0,025 |
| Exemple 51 | 0,280 | 0,062 |
| Exemple 52 | 0,350 | 0,080 |
| Exemple 54 | 0,900 | 0,085 |
| Exemple 56 | 0,890 | 0,030 |
| Exemple 57 | 0,080 | 0,005 |
| Exemple 58 | 0,045 | 0,009 |
| Exemple 59 | 0,4 | 0,013 |
| Exemple 60 | 0,9 | 0,115 |
| Exemple 61 | 0,190 | 0,028 |
| Exemple 62 | 4 | 2 |
| Exemple 64 | 2,8 | 0,080 |
| Exemple 66 | 3,8 | 0,750 |
| Exemple 67 | 0,525 | 0,016 |

DOSE ACTIVE 50

(exprimée en mg.kg-1, administration par voie orale)

| | 1) Administration d'un agent médicamenteux ulcérigène | 2) Administration d'un agent nécrosant |
|---|---|---|
| Exemple 69 | 0,125 | 0,006 |
| Exemple 70 | 0,200 | 0,010 |
| Exemple 71 | 0,330 | 0,070 |
| Exemple 72 | 3 | 0,090 |
| Exemple 73 | 0,200 | 0,032 |
| Exemple 46 | 0,024 | 0,018 |
| Exemple 74 | 5,25 | 0,225 |
| Exemple 75 | 0,078 | 0,005 |
| Exemple 76 | 0,061 | 0,029 |
| Exemple 77 | 0,175 | 0,017 |
| Exemple 78 | 1,9 | 0,140 |
| Exemple 79 | 0,600 | 0,021 |
| Exemple 81 | 0,8 | 0,150 |
| Exemple 87 | 4,7 | 0,062 |
| Exemple 90 | 0,600 | 0,140 |
| Exemple 91 | 3,4 | 0,140 |
| Exemple 94 | 4,5 | 0,087 |
| Exemple 95 | 0,34 | 0,014 |
| Exemple 109 | 0,195 | 0,250 |
| Exemple 110 | 2,4 | 3,5 |
| Exemple 111 | 2,5 | 0,370 |
| Exemple 112 | 13 | 3,5 |
| Exemple 113 | 3,6 | 2 |
| Exemple 116 | 0,080 | 0,130 |
| Exemple 117 | 0,021 | 0,120 |
| Exemple 118 | 1 | 0,100 |

1) agent anti-inflammatoire non stéroïdien

2) éthanol

ACTIVITE ANTISECRETOIRE

1. Méthode

Une ligature au niveau du pylore est pratiquée sous anesthésie à l'éther chez des lots de 5 rats mâles de souche OFA (provenance IFFA CREDO, France) pesant 180-200 g.

Le produit à étudier est administré par voie sous-cutanée au temps de la ligature. Les animaux sont sacrifiés 4 h plus tard et le liquide gastrique est recueilli puis son acidité titrée par la soude 0,1 N à pH 4 et 7.

2. Résultats

Les résultats sont exprimés en doses actives 50 (dose provoquant une inhibition de 50 % de la sécrétion acide totale) déterminées graphiquement à partir de la droite exprimant le pourcentage d'inhibition de la sécrétion acide totale en fonction des doses utilisées.

DOSE ACTIVE 50

(exprimée en mg.kg-1, administration par voie sous-cutanée)

| | |
|---|---|
| Exemple 6 | 0,070 |
| Exemple 7 | 2,6 |
| Exemple 9 | 0,32 |
| Exemple 10 | 1,2 |
| Exemple 12 | 0,09 |
| Exemple 13 | 0,05 |
| Exemple 14 | 0,46 |
| Exemple 15 | 0,35 |
| Exemple 16 | 0,28 |
| Exemple 17 | 0,2 |
| Exemple 19 | 0,17 |
| Exemple 20 | 0,06 |
| Exemple 21 | 0,8 |
| Exemple 23 | 0,013 |
| Exemple 24 | 30 |
| Exemple 25 | 1,5 |
| Exemple 26 | 0,125 |
| Exemple 32 | 0,075 |
| Exemple 36 | 0,130 |

DOSE ACTIVE 50

(exprimée en mg.kg-1, administration par voie sous-cutanée)

| | |
|---|---|
| Exemple 38 | 0,030 |
| Exemple 39 | 0,750 |
| Exemple 40 | 0,170 |
| Exemple 41 | 0,850 |
| Exemple 42 | 0,320 |
| Exemple 43 | 0,04 |
| Exemple 44 | 0,010 |
| Exemple 45 | 0,095 |
| Exemple 47 | 0,110 |
| Exemple 48 | 2,4 |
| Exemple 50 | 0,065 |
| Exemple 51 | 0,200 |
| Exemple 52 | 0,036 |
| Exemple 54 | 1,50 |
| Exemple 56 | 0,170 |
| Exemple 57 | 0,020 |
| Exemple 58 | 0,170 |
| Exemple 59 | 0,165 |
| Exemple 60 | 1,2 |
| Exemple 61 | 0,052 |
| Exemple 62 | 0,650 |
| Exemple 64 | 0,042 |
| Exemple 66 | 2,5 |
| Exemple 33 | 0,014 |
| Exemple 67 | 0,033 |
| Exemple 69 | 0,035 |
| Exemple 70 | 0,021 |
| Exemple 22 | 0,027 |
| Exemple 71 | 0,150 |
| Exemple 72 | 0,460 |
| Exemple 73 | 0,100 |
| Exemple 46 | 0,022 |
| Exemple 74 | 0,300 |

DOSE ACTIVE 50

(exprimée en mg.kg-1, administration par voie sous-cutanée)

| | |
|---|---|
| Exemple 75 | 0,100 |
| Exemple 76 | 0,019 |
| Exemple 77 | 0,200 |
| Exemple 78 | 0,200 |
| Exemple 79 | 0,020 |
| Exemple 81 | 0,490 |
| Exemple 87 | 0,475 |
| Exemple 90 | 0,110 |
| Exemple 91 | 0,170 |
| Exemple 94 | 0,170 |
| Exemple 95 | 0,140 |
| Exemple 109 | 0,090 |
| Exemple 110 | 0,550 |
| Exemple 111 | 0,300 |
| Exemple 112 | 1,5 |
| Exemple 113 | 1,2 |
| Exemple 116 | 0,018 |
| Exemple 117 | 0,024 |
| Exemple 118 | 0,140 |

TOXICOLOGIE

Les premières études toxicologiques réalisées chez le rat à jeun Sprague Dawley après administration par voie orale ont permis de montrer que les $DL_{50}$ pour la plupart des produits exemplifiés sont supérieures ou égales à 300 mg.kg-1. Seuls les produits des exemples 26 et 36 possèdent une dose létale 50 comprise entre 100 et 300 mg.kg-1.

CONCLUSION

Les produits selon l'invention et leurs sels d'addition d'acides non toxiques décrits dans la présente demande semblent particulièrement intéressants ; leur activité anti-ulcère et leur activité antisécrétoire sont extrêmement puissantes en particulier pour les produits des exemples 22, 33, 34, 55, 57, 58, 70, 75, 97.

Ils pourront être utilisés par voie orale ou injectable dans le traitement des ulcères gastro-duodénaux, sous forme d'ampoules injectables ou de gelules dosées de 5 à 100 mg.

**Revendications**

**Revendications pour les Etats contractants suivants : BE, CH, DE, FR, GB, IT, LI, LU, NL, SE**

**1.** Composés phénylnaphthyridines fonctionnalisés en position 3 de formule :

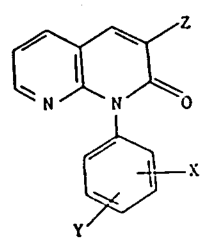

(I)

dans laquelle :

X et Y représentent l'atome d'hydrogène, un halogène, un groupement alkyle inférieur de 1 à 5 atomes de carbone un trifluorométhyle, un alcoxy, un méthylthio, un nitro, un cyano et peuvent être en position ortho, métha, para sur le cycle aromatique,

Z est un groupement fonctionnel choisi parmi :

a) un propényle ou propynyle,

b) une chaîne $-(CH_2)_n-S-(CH_2)_m-R$ ou $-(CH_2)_n-SO-(CH_2)_m-R$, dans laquelle n et m sont identiques ou différents et sont des nombres compris entre 0 et 5, et R est un méthyle, ou une fonction du type

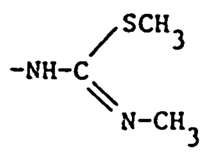

,

du type

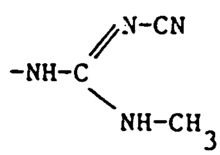

ou du type

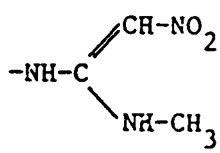

,

c) une chaîne $-(CH_2)_n-O-(CH_2)_m-CH_3$ (n et m étant définis comme ci-dessus)

d) une chaîne $-(CH_2)_n-OH$ ou un de ses esters choisis parmi le groupe consistant d'un acétate, propionate, nicotinate, furanne-2-carboxylate, et phényl acétate, n étant défini comme ci-dessus ;

e) une chaîne $-(CH_2)_n-CHOH-(CH_2)_m-R'$ ou un de ses esters choisis parmi le groupe consistant d'un acétate, propionate, nicotinate, furanne-2-carboxylate, et phényl acétate, n et m étant définis comme ci-dessus, R' étant un méthyle ou un groupe phényle ou pyridyle;

f) une chaîne $-(CH_2)_n-CO-(CH_2)_m-CH_3$ (n et m étant définis comme ci-dessus) ;

g) une chaîne $-(CH_2)_n-COOH$ (n étant défini comme ci-dessus), principalement n étant au moins égal à 3 et optimalement n = 4 ;

h) une chaîne $-(CH_2)_n-Hal$ (n étant défini comme ci-dessus et Hal étant un halogène) ;

i) une chaîne $-(CH_2)_n-CN$ (n étant défini comme ci-dessus) ;

j) une chaîne

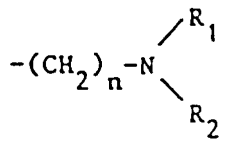

n étant défini comme ci-dessus, et $NR_1R_2$ forme un cycle pypérazine substitué par un groupe méthyle, un groupe phényle ou un groupe phényle substitué par un méthoxy ou alternativement $NR_1R_2$ forme un cycle imidazole ;
k) une chaîne

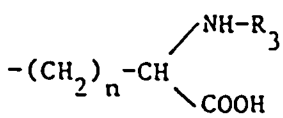

n étant défini comme ci-dessus,
$R_3$ étant un hydrogène ou un groupe para-chlorobenzoyl ;
l) une chaîne

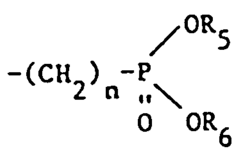

n étant défini comme ci-dessus,
$R_5$ et $R_6$ étant un groupe éthyle, et
m) un noyau phényle, pyridyle, thiophène ou furanne, ainsi qu'éventuellement les sels d'addition d'acide non toxiques.

**2.** Nouveaux composés selon la revendication 1, caractérisés en ce que X et Y sont en position métha ou para.

**3.** Nouveaux composés selon la revendication 1 ou 2, caractérisés en ce que X = Y = H.

**4.** Nouveaux composés selon la revendication 1 ou 2, caractérisés en ce que X = 3-CN, Y = H.

**5.** Nouveaux composés selon la revendication 1 ou 2, caractérisés en ce que X = 4-F, Y = H.

**6.** Nouveaux composés selon la revendication 1 ou 2, caractérisés en ce que X = 3-$NO_2$, Y = H.

**7.** Nouveaux composés selon la revendication 1 ou 2, caractérisés en ce que X = 3-Cl, Y = H.

**8.** Nouveaux composés selon la revendication 1 ou 2, caractérisés en ce que Z comporte une fonction alcool de formule $-(CH_2)_n-OH$ ou un ester de celui-ci.

**9.** Nouveaux composés selon la revendication 1 ou 2, caractérisés en ce que Z = $-CH_2-CHOH-CH_3$ ou un ester de cet alcool secondaire.

**10.** Nouveaux composés selon la revendication 1 ou 2, caractérisés en ce que Z = $- CH_2-CO-CH_3$.

**11.** Nouveaux composés selon la revendication 1 ou 2, caractérisés en ce que Z = $-(CH_2)_n-CO-(CH_2)_m-CH_3$.

**12.** Nouveaux composés selon la revendication 1 ou 2, caractérisés en ce que Z = méthylthiométhyle.

**13.** Nouveaux composés selon la revendication 1 ou 2, caractérisés en ce qu'il est choisi parmi les suivants :

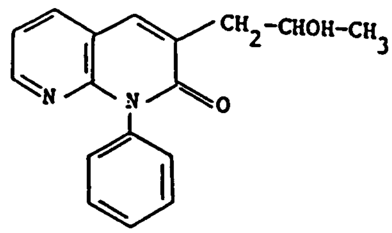

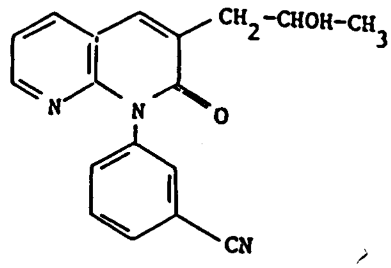

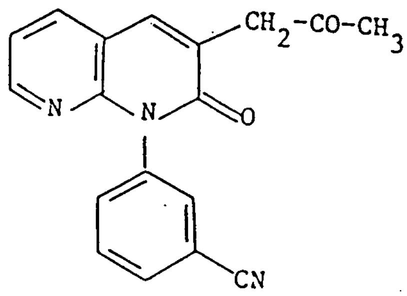

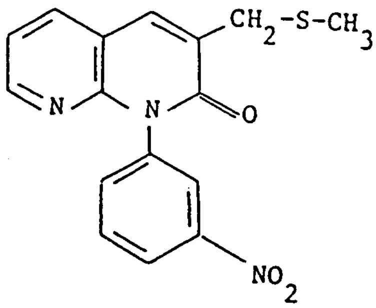

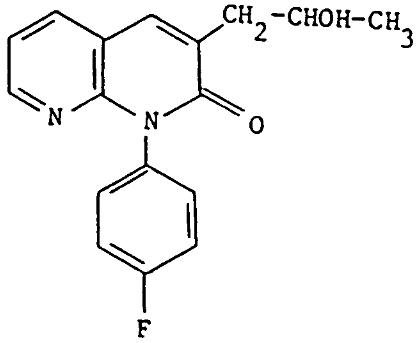

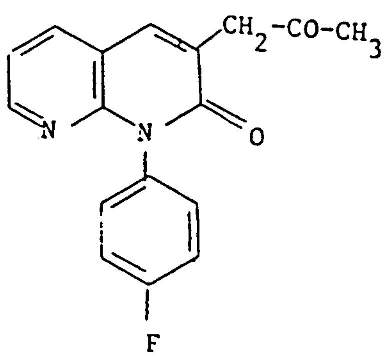

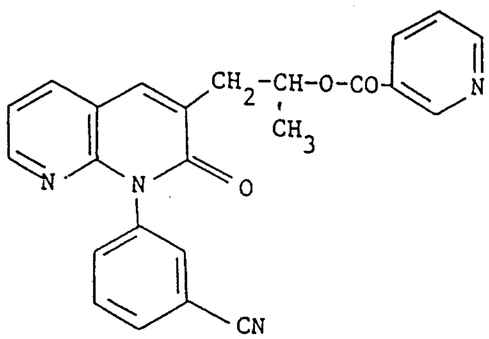

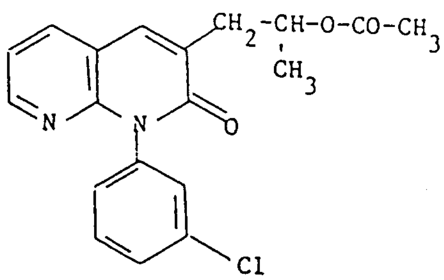

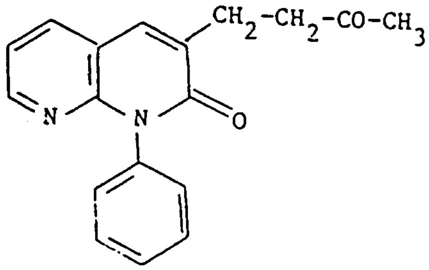

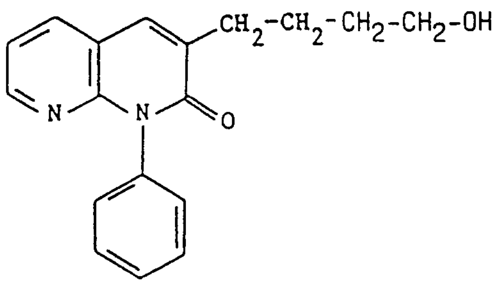

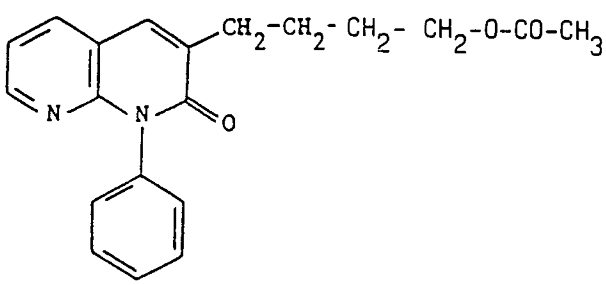

**14.** Médicaments utiles notamment pour leur activité anti-ulcère et anti-secrétoire, caractérisés en ce qu'ils contiennent au moins un composé selon l'une quelconque des revendications 1 à 13 ou éventuellement un de ses sels d'addition d'acide non toxiques.

15. Procédé de préparation des composés de formule (I) selon l'une quelconque des revendications 1 à 13, caractérisés en ce qu'ils sont préparés par une méthode de réaction de condensation choisie parmi celle de Stobbe, de Perkin, de Claisen ou de Knoevenagel, sur un aldéhyde de formule (II) :

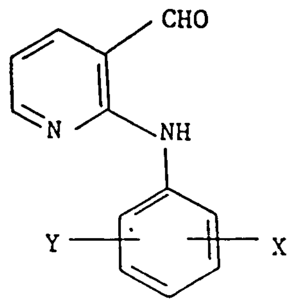

(II)

dans la formule (II), X et Y sont tels que définis précédemment.

16. Procédé selon la revendication 15, caractérisé en ce que les aldéhydes de formule (II) sont obtenus par oxydation, à l'aide d'un oxydant doux, par exemple $MNO_2$, dans un solvant organique, par exemple le dichlorométhane ou le chloroforme, à une température comprise entre 20 et 50 ° C d'un alcool de formule (III) :

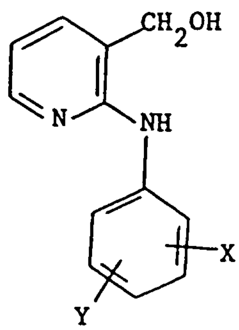

(III)

dans la formule (III) X et Y sont tels que définis précédemment.

17. Procédé selon la revendication 16, caractérisé en ce que les alcools de formule (III) sont obtenus par réduction à l'aide d'un réducteur classique, par exemple l'hydrure double d'aluminium et de lithium dans un solvant organique par exemple le tétrahydrofuranne ou l'éther éthylique d'un acide ou d'un de ses esters de formule (IV) ; dans le cas où le noyau phényle sera porteur d'une substitution sensible à certains réducteurs tels que par exemple nitro ou cyano, on choisira la réduction de l'ester par un réducteur respectant cette substitution par exemple le borohydrure de lithium préparé "in situ" a partir du borohydrure de potassium et du chlorure de lithium :

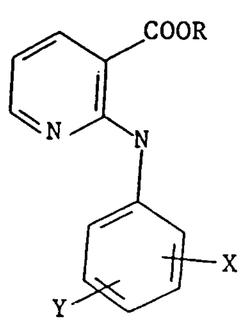

(IV)

dans la formule (IV), X et Y sont tels que définis précédemment, et R est l'atome d'hydrogène ou un

alkyle.

**18.** Procédé de préparation des composés de formule (I) selon l'une quelconque des revendications 1 à 13, caractérisé en ce que l'on fait réagir les aldéhydes de formule (II), définie à la revendication 14 avec :

a) des anhydrides d'acides de formule

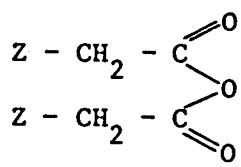

Z étant défini comme ci-dessus en présence du sel de sodium, de l'acide $Z-CH_2-COOH$

b) des anhydrides d'acides de formule

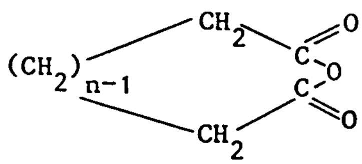

ou son chlorure

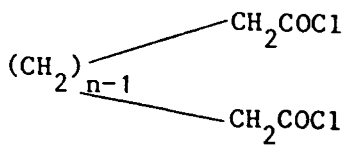

en présence du sel de sodium de l'acide correspondant, n étant défini comme ci-dessus

c) des esters d'acides de formule

$Z - CH_2 - COOR''$

Z étant défini comme ci-dessus, R" étant un alkyle, en présence d'alcoolate de sodium ou de potassium ou d'hydrure de sodium ou de sodium métal, ou

d) des lactones de formule :

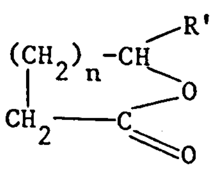

n étant défini comme ci-dessus, R' étant un méthyle ou un noyau aromatique ou hétéroatomatique en présence d'alcoolate de sodium ou de potassium ou d'hydrure de sodium ou de sodium métal, en présence ou en l'absence d'un solvant organique tel qu'un alcool, le benzène, le toluène, ou la N-méthylpyrrolidone, à une température comprise entre 20 et 200 ° C environ.

**19.** Procédé selon l'une des revendications 17 ou 18, caractérisé en ce que :

a) les dérivés S→O s'obtiennent par oxydation de composés -S- à l'aide par exemple d'un peracide, l'acide métachloroperbenzoïque pouvant être utilisé, dans un solvant tel que le chloroforme ou le dichlorométhane ;

b) les dérivés ester d'alcool seront obtenus de façon classique par estérification des alcools correspondants par un chlorure d'acide ou un anhydride d'acide ;

c) les dérivés cétoniques sont synthétisés par oxydation des alcools secondaires à l'aide d'oxydants connus tels que le réactif de Jones ou le réactif de Sarett dans un solvant organique tel que l'acétone, le chloroforme ou le dichlorométhane ;

d) les dérivés halogénés sont préparés par action du chlorure de thionyle ou de l'acide bromhydrique sur les alcools primaires correspondants, ces dérivés peuvent être utilisés en particulier pour la synthèse des dérivés aminés par action de l'amine correspondante, imidazolyle par action de l'imidazole métallé, pour préparer certains dérivés cyanés, alcoxy ou alkylthio par action du sel de sodium ou de potassium du composé correspondant, pour la préparation de certains amino et amidoacides par synthèse malonique en condensant l'acétamidomalonate de dialkyle préalablement métallé ou encore par la synthèse de composés phosphorés par action de trialkylphosphites.

**Revendications pour les Etats contractants suivants : AT, ES, GR**

**1.** Utilisation des phénylnaphthyridines fonctionnalisées en position 3 de formule :

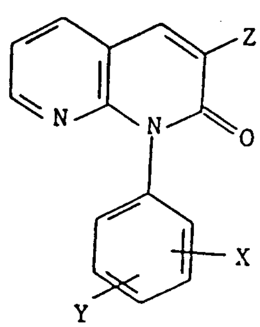

(I)

dans laquelle :

X et Y représentent l'atome d'hydrogène, un halogène, un groupement alkyle inférieur de 1 à 5 atomes de carbone, un trifluorométhyle, un alcoxy, un méthylthio, un nitro, un cyano et peuvent être en psoition ortho, métha, para sur le cycle aromatique,

Z est un groupement fonctionnel choisi parmi :

a) un propényle ou propynyle,

b) une chaîne $-(CH_2)_n-S-(CH_2)_m-R$ ou $-(CH_2)_n-SO-(CH_2)_m-R$, dans laquelle n et m sont identiques ou différents et sont des nombres compris entre 0 et 5, et R est un méthyle, ou une fonction du type

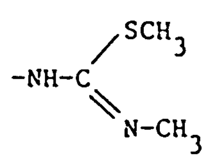

,

du type

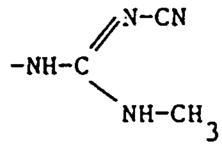

ou du type

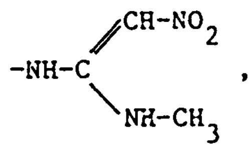

c) une chaîne $-(CH_2)_n-O-(CH_2)_m-CH_3$ (n et m étant définis comme ci-dessus)

d) une chaîne $-(CH_2)_n-OH$ ou un de ses esters choisis parmi le groupe consistant d'un acétate, propionate, nicotinate, furanne-2-carboxylate, et phényl acétate, n étant défini comme ci-dessus ;

e) une chaîne $-(CH_2)_n-CHOH-(CH_2)_m-R'$ ou un de ses esters choisis parmi le groupe consistant d'un acétate, propionate, nicotinate, furanne-2-carboxylate, et phényl acétate, n et m étant définis comme ci-dessus, R' étant un méthyle ou un groupe phényle ou pyridyle;

f) une chaîne $-(CH_2)_n-CO-(CH_2)_m-CH_3$ (n et m étant définis comme ci-dessus) ;

g) une chaîne $-(CH_2)_n-COOH$ (n étant défini comme ci-dessus), principalement n étant au moins égal à 3 et optimalement n = 4 ;

h) une chaîne $-(CH_2)_n-Hal$ (n étant défini comme ci-dessus et Hal étant un halogène) ;

i) une chaîne $-(CH_2)_n-CN$ (n étant défini comme ci-dessus) ;

j) une chaîne

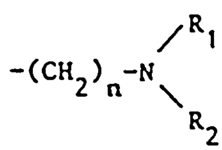

n étant défini comme ci-dessus, et $NR_1R_2$ forme un cycle pypérazine substitué par un groupe méthyle, un groupe phényle ou un groupe phényle substitué par un méthoxy ou alternativement $NR_1R_2$ forme un cycle imidazole ;

k) une chaîne

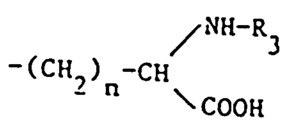

n étant défini comme ci-dessus,

$R_3$ étant un hydrogène ou un groupe para-chlorobenzoyl ;

l) une chaîne

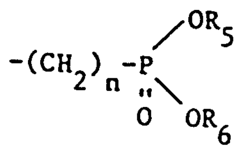

n étant défini comme ci-dessus,
$R_5$ et $R_6$ étant un groupe éthyle, et
m) un noyau phényle, pyridyle, thiophène ou furanne, ainsi qu'éventuellement les sels d'addition d'acide non toxiques, pour la préparation d'une composition pharmaceutique utile notamment pour son activité anti-ulcère et anti-secrétoire.

**2.** Utilisation selon la revendication 1, caractérisée en ce que X et Y sont en position métha ou para.

**3.** Utilisation selon la revendication 1 ou 2, caractérisée en ce que X = Y = H.

**4.** Utilisation selon la revendication 1 ou 2, caractérisée en ce que X = 3-CH, Y = H.

**5.** Utilisation selon la revendication 1 ou 2, caractérisée en ce que X = 4-F, Y = H.

**6.** Utilisation selon la revendication 1 ou 2, caractérisée en ce que X = 3-$NO_2$, Y = H.

**7.** Utilisation selon la revendication 1 ou 2, caractérisée en ce que X = 3-Cl, Y = H.

**8.** Utilisation selon la revendication 1 ou 2, caractérisée en ce qui Z comporte une fonction alcool de formule $-(CH_2)_n-OH$ ou un ester de celui-ci.

**9.** Utilisation selon la revendication 1 ou 2, caractérisée en ce que Z = $-CH_2-CHOH-CH_3$ ou un ester de cet alcool secondaire.

**10.** Utilisation selon la revendication 1 ou 2, caractérisée en ce que Z = $- CH_2-CO-CH_3$.

**11.** Utilisation selon la revendication 1 ou 2, caractérisée en ce que Z = $-(CH_2)_n-CO-(CH_2)_m-CH_3$.

**12.** Utilisation selon la revendication 1 ou 2, caractérisée en ce que Z = méthylthiométhyle.

**13.** Utilisation selon la revendication 1 ou 2, caractérisée en ce qu'il est choisi parmi les suivants :

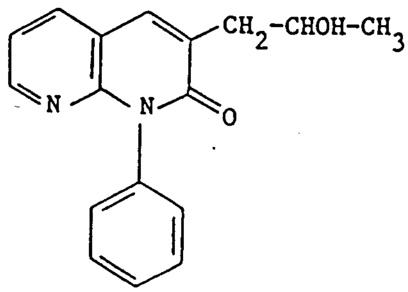

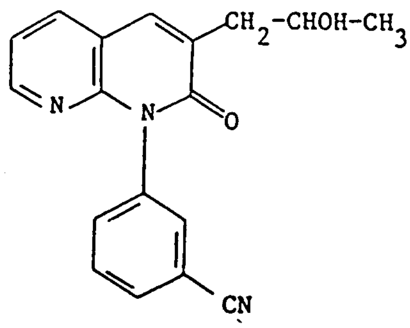

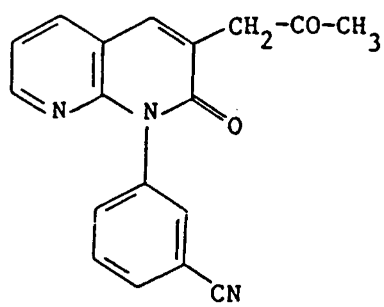

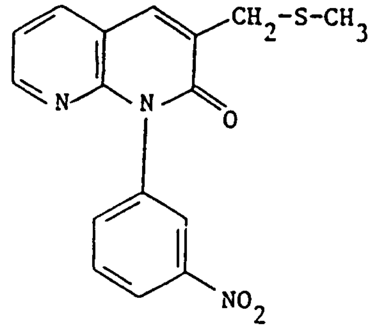

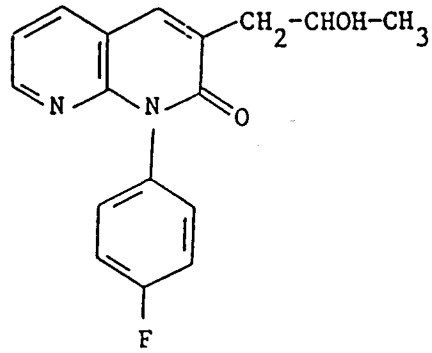

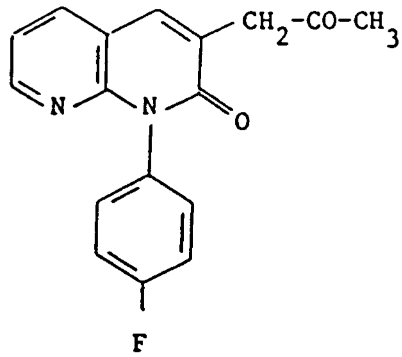

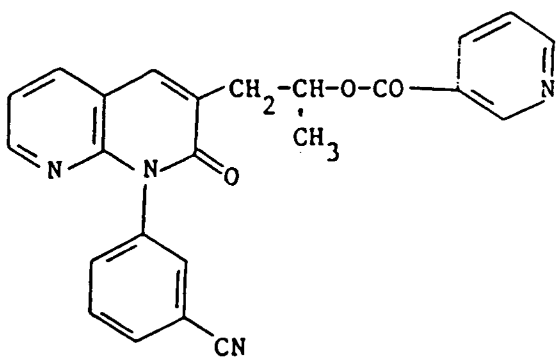

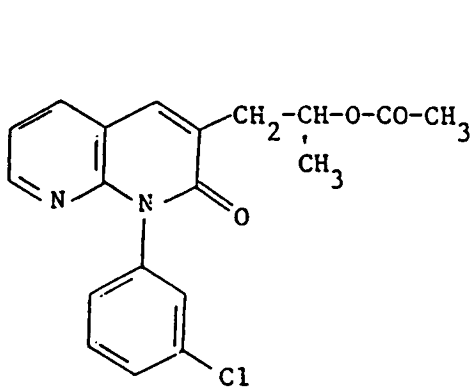

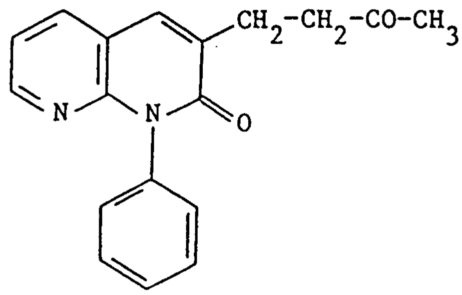

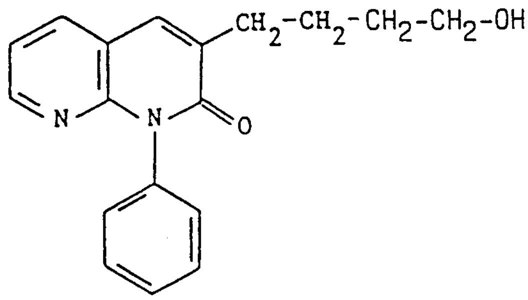

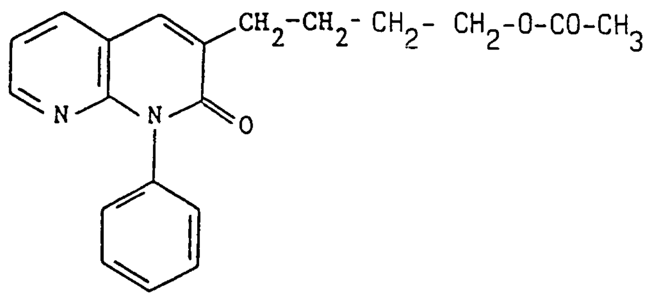

**14.** Procédé de préparation d'un médicament utile notamment pour son activité anti-ulcère et anti-secrétoire, caractérisé en ce qu'on mélange, à titre d'ingrédient actif, au moins un composé selon l'une quelconque des revendications 1 à 13, ou éventuellement un de ses sels d'addition d'acide non toxiques avec un excipient ou véhicule pharmaceutiquement acceptable.

**15.** Procédé de préparation des composés de formule (I) selon l'une quelconque des revendications 1 à 13, caractérisés en ce qu'ils sont préparés par une méthode de réaction de condensation choisie parmi celle de Stobbe, de Perkin, de Claisen ou de Knoevenagel, sur un aldéhyde de formule (II) :

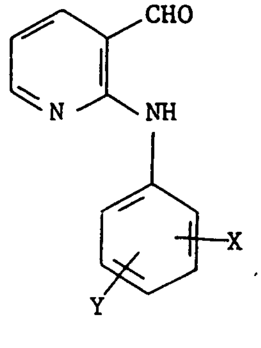

(II)

dans la formule (II), X et Y sont tels que définis précédemment.

**16.** Procédé selon la revendication 15, caractérisé en ce que les aldéhydes de formule (II) sont obtenus par oxydation, à l'aide d'un oxydant doux,par exemple $MNO_2$, dans un solvant organique, par exemple le dichlorométhane ou le chloroforme, à une température comprise entre 20 et 50 °C d'un alcool de formule (III) :

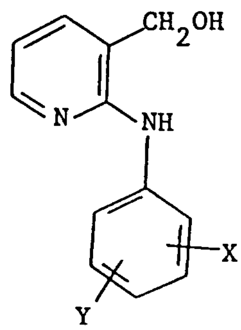

(III)

dans la formule (III) X et Y sont tels que définis précédemment.

**17.** Procédé selon la revendication 16, caractérisé en ce que les alcools de formule (III) sont obtenus par réduction à l'aide d'un réducteur classique, par exemple l'hydrure double d'aluminium et de lithium dans un solvant organique par exemple le tétrahydrofuranne ou l'éther éthylique d'un acide ou d'un de ses esters de formule (IV) ; dans le cas où le noyau phényle sera porteur d'une substitution sensible à certains réducteurs tels que par exemple nitro ou cyano, on choisira la réduction de l'ester par un réducteur respectant cette substitution par exemple le borohydrure de lithium préparé "in situ" à partir du borohydrure de potassium et du chlorure de lithium :

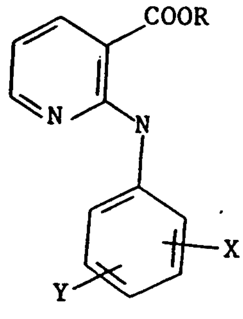

(IV)

dans la formule (IV), X et Y sont tels que définis précédemment, et R est l'atome d'hydrogène ou un alkyle.

**18.** Procédé de préparation des composés de formule (I) selon l'une quelconque des revendications 1 à 13, caractérisé en ce que l'on fait réagir les aldéhydes de formule (II), définie à la revendication 14 avec :

a) des anhydrides d'acides de formule

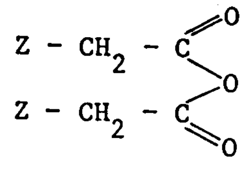

Z étant défini comme ci-dessus en présence du sel de sodium, de l'acide $Z\text{-}CH_2\text{-}COOH$

b) des anhydrides d'acides de formule

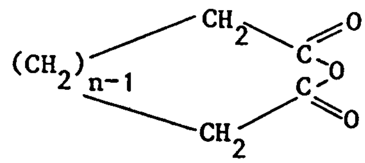

ou son chlorure

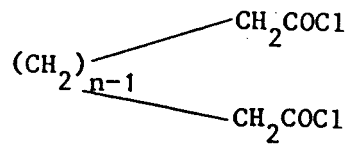

en présence du sel de sodium de l'acide correspondant, n étant défini come ci-dessus
c) des esters d'acides de formule

Z - $CH_2$ - COOR"

Z étant défini comme ci-dessus, R" étant un alkyle, en présence d'alcoolate de sodium ou de potassium ou d'hydrure de sodium ou de sodium métal, ou
d) des lactones de formule :

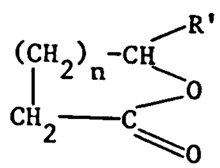

n étant défini comme ci-dessus, R' étant un méthyle ou un noyau aromatique ou hétéroatomatique en présence d'alcoolate de sodium ou de potassium ou d'hydrure de sodium ou de sodium métal, en présence ou en l'absence d'un solvant organique tel qu'un alcool, le benzène, le toluène, ou la N-méthylpyrrolidone, à une température comprise entre 20 et 200° C environ.

**19.** Procédé selon l'une des revendications 17 ou 18, caractérisé en ce que :
a) les dérivés S→O s'obtiennent par oxydation de composés -S- à l'aide par exemple d'un peracide, l'acide métachloroperbenzoïque pouvant être utilisé, dans un solvant tel que le chloroforme ou le dichlorométhane ;
b) les dérivés ester d'alcool seront obtenus de façon classique par estérification des alcools correspondants par un chlorure d'acide ou un anhydride d'acide ;
c) les dérivés cétoniques sont synthétisés par oxydation des alcools secondaires à l'aide d'oxydants connus tels que le réactif de Jones ou le réactif de Sarett dans un solvant organique tel que l'acétone, le chloroforme ou le dichlorométhane ;
d) les dérivés halogénés sont préparés par action du chlorure de thionyle ou de l'acide bromhydrique sur les alcools primaires correspondants, ces dérivés peuvent être utilisés en particulier pour la synthèse des dérivés aminés par action de l'amine correspondante, imidazolyle par action de l'imidazole métallé, pour préparer certains dérivés cyanés, alcoxy ou alkylthio par action du sel de sodium ou de potassium du composé correspondant, pour la préparation de certains amino et amidoacides par synthèse malonique en condensant l'acétamidomalonate de dialkyle préalablement métallé ou encore par la synthèse de composés phosphorés par action de trialkylphosphites.

## Claims

### Claims for the following Contracting States : BE, CH, DE, FR, GB, IT, LI, LU, NL, SE

**1.** Phenylnaphthyridine compounds carrying functional groups in the 3-position, of the formula :

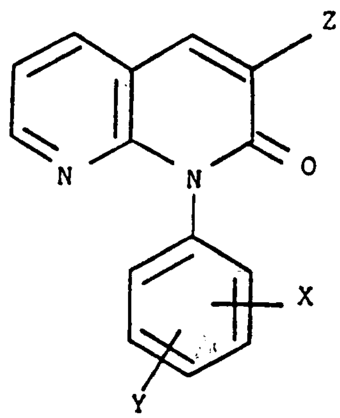

(I)

in which:
X and Y represent the hydrogen atom, a halogen, a lower alkyl group containing 1 to 5 carbon atoms, a trifluoromethyl, an alkoxy, a methylthio, a nitro or a cyano and can be in the ortho, meta or para position on the aromatic ring,
Z represents a functional group selected from:
a) a propenyl or propynyl
b) a chain $-(CH_2)_n-S-(CH_2)_m-R$ or $-(CH_2)_n-SO-(CH_2)_m-R$, in which n and m are identical or different and are numbers between 0 and 5, and R is a methyl or a functional group of the type:

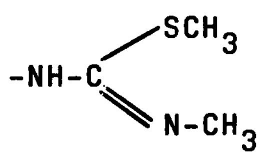

,

of the type

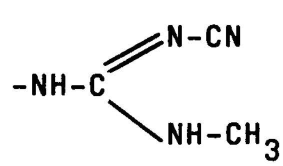

or of the type

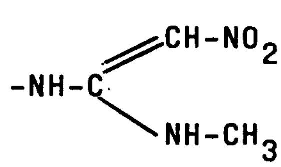

,

c) a chain $-(CH_2)_n-O-(CH_2)_m-CH_3$ (n and m being defined as above)
d) a chain $-(CH_2)_n-OH$ or one of its esters selected from the group consisting in an acetate, propionate, nicotinate, furan-2-carboxylate, and phenylacetate, n being defined as above;
e) a chain $-(CH_2)_n-CHOH-(CH_2)_m-R'$ or one of its esters selected from the group consisting in an acetate, propionete, nicotinate, furan-2-carboxylate, and phenylacetate, n and m being defined as above, R' being a methyl or a phenyl or pyridyl group;
f) a chain $-(CH_2)_n-CO-(CH_2)_m-CH_3$ (n and m being defined as above);
g) a chain $-(CH_2)_n-COOH$ (n being defined as above), n being principally equal to at least 3 and optimally equal to 4;
h) a chain $-(CH_2)_n-Hal$ (n being defined as above and Hal being a halogen);
i) a chain $-(CH_2)_n-CN$ (n being defined as above);
j) a chain

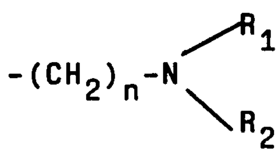

n being defined as above, and $HR_1R_2$ forms a piperazine ring substituted by a methyl group, a phenyl group or a phenyl group substituted by a methoxy or alternatively $NR_1R_2$ forms an imidazole ring;
k) a chain

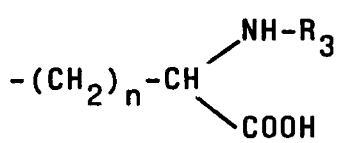

n being defined as above,
$R_3$ being a hydrogen or a parachlorobenzoyl group;
l) a chain

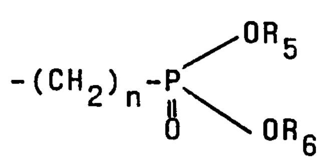

n being defined as above,
$R_5$ and $R_6$ being an ethyl group, and
m) a phenyl, pyridyl, thiophene or furan nucleus, and if appropriate non-toxic acid addition salts.

**2.** New compound according to claim 1, characterized in that X and Y are in the mate or para position.

**3.** New compound according to claim 1 or 2, characterized in that X = Y = H.

**4.** New compound according to claim 1 or 2, characterized in that X = 3-CN and Y = H.

**5.** New compound according to claim 1 or 2, characterized in that X = 4-F and Y = H.

**6.** New compound according to claim 1 or 2, characterized in that X = $3-NO_2$ and Y = H.

**7.** New compound according to claim 1 or 2, characterized in that X = 3-Cl and Y = H.

**8.** New compound according to claim 1 or 2, characterized in that Z contains an alcohol functional group of formula $-(CH_2)_n-OH$ or an ester thereof.

**9.** New compound according to claim 1 or 2, characterized in that Z = $-CH_2-CHOH-CH_3$ or an ester of this secondary alcohol.

**10.** New compound according to claim 1 or 2, characterized in that Z = $-CH_2-CO-CH_3$.

**11.** New compound according to claim 1 or 2, characterized in that Z = $(CH_2)_n-CO-(CH_2)_m-CH_3$.

**12.** New compound according to claim 1 or 2, characterized in that Z = a methylthiomethyl.

**13.** New compound according to claim 1 or 2, characterized in that it is selected from the following:

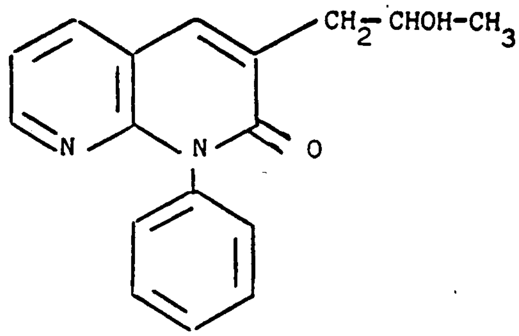

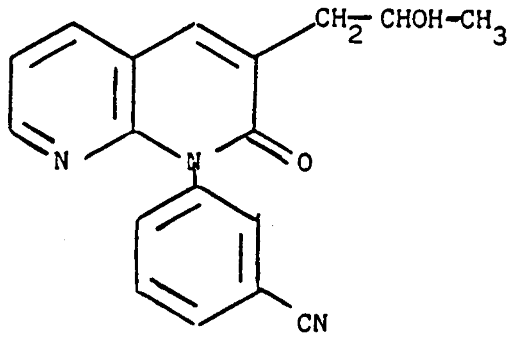

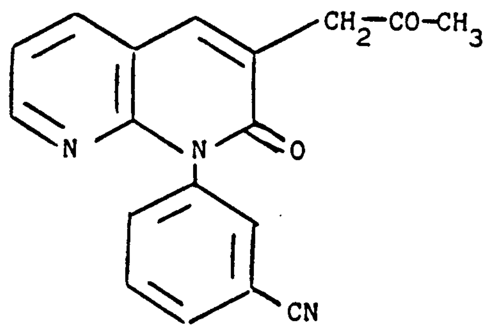

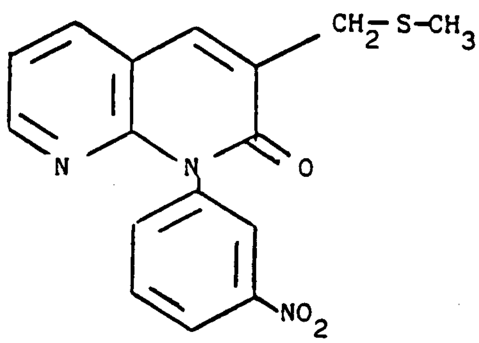

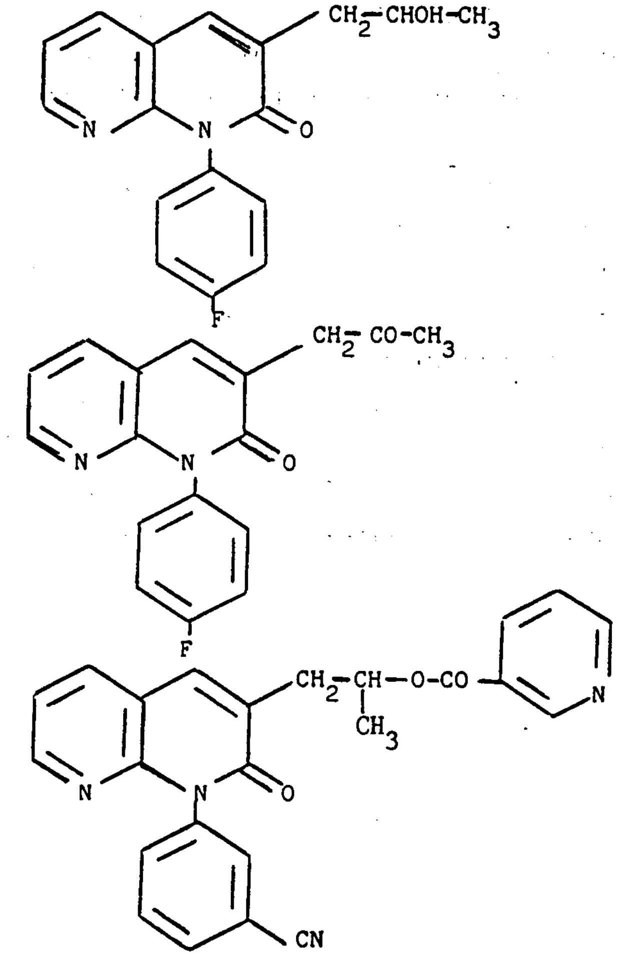

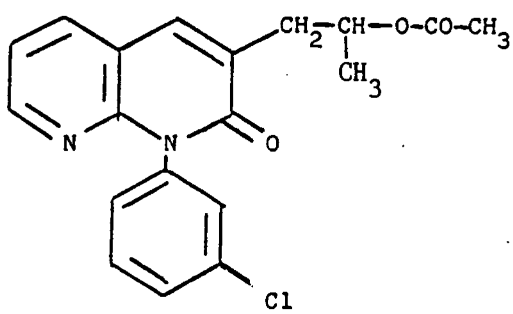

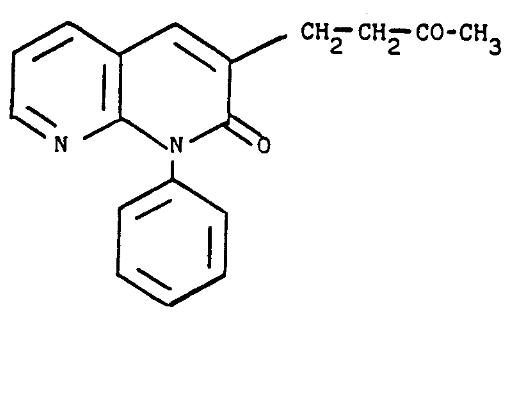

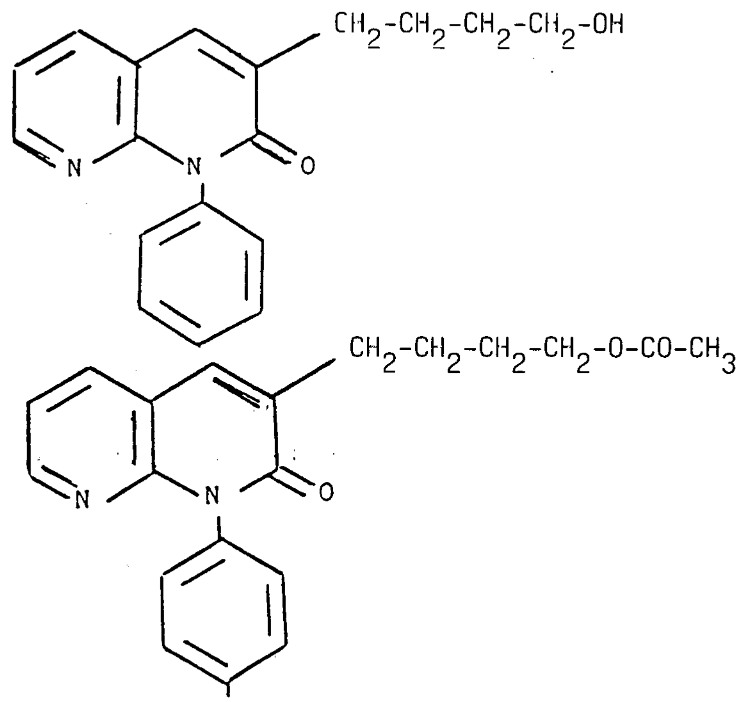

**14.** Drug useful in particular for its ulcer-inhibiting and anti-secretory activity, characterized in that they contain at least one compound according to any one of claims 1 to 13 or if appropriate one of its non-toxic acid addition salts.

**15.** Process for the preparation of the compounds of the formula (I) according to any one of claims 1 to 13, characterized in that they are prepared by a method involving a condensation reaction, selected from those of Stobbe, Perkin, Claisen or Knoevenagel, with an aldehyde of the formula (II):

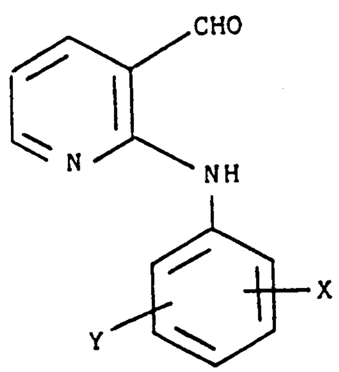

(II)

in the formula (II), X and Y are as defined above.

**16.** Process according to claim 15, characterized in that the aldehydes of the formula (II) are obtained by the oxidation of an alcohol of the formula (III) with a mild oxidizing agent, for example $MnO_2$, in an organic solvent, for example methylene chloride or chloroform, at a temperature of between 20 and 50 ° C:

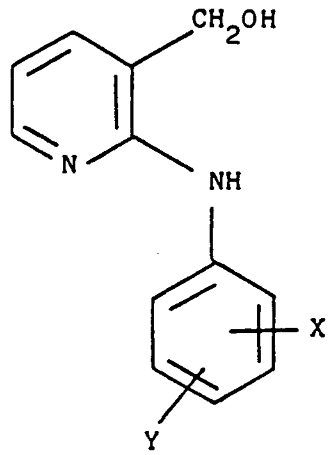

(III)

in the formula (III), X and Y are as defined above.

**17.** Process according to claim 16, characterized in that the alcohols of the formula (III) are obtained by the reduction of an acid or one of its esters of the formula (IV) with a conventional reducing agent, for example lithium aluminium hydride, in an organic solvent, for example tetrahydrofuran or ethyl ether; in the case where the phenyl nucleus carries a substituent which is sensitive to certain reducing agents, for example a nitro or cyano substituent, the reducing agent for reducing the ester will be chosen so as not to affect this substituent, an example being lithium borohydride prepared "in situ" from potassium borohydride and lithium chloride:

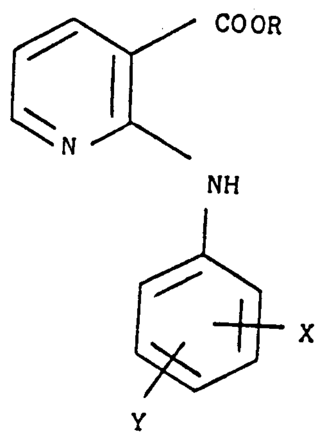

(IV)

in the formula (IV), X and Y are as defined above and R is the hydrogen atom or an alkyl.

**18.** Process for the preparation of the compounds of the formula (I) according to any one of claims 1 to 13, characterized in that reacting the aldehydes of the formula (II), defined in claim 14, is performed with:

a) acid anhydrides of the formula:

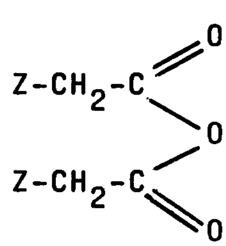

Z being defined as above, in the presence of the sodium salt of the acid $Z-CH_2-COOH$;
b) acid anhydrides of the formula:

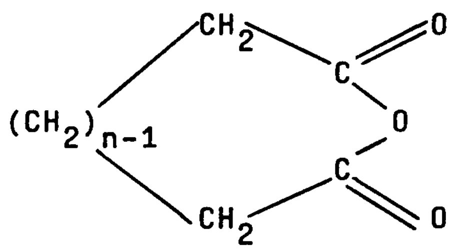

or its chloride

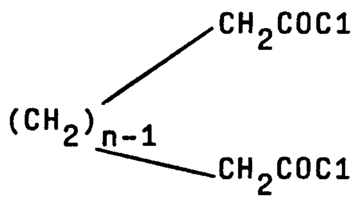

in the presence of the sodium salt of the corresponding acid, n being defined as above;
c) acid esters of the formula:

$Z-CH_2-COOR''$

Z being defined as above and R" being an alkyl, in the presence of a sodium or potassium alcoholate, sodium hydride or sodium metal; or
d) lactones of the formula:

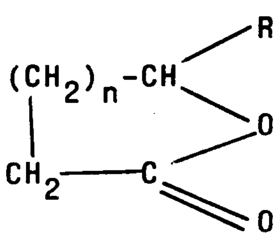

n being defined as above and R' being a methyl or an aromatic or heteroaromatic nucleus, in the presence of a sodium or potassium alcoholate, sodium hydride or sodium metal, and in the presence or absence of an organic solvent such as an alcohol, benzene, toluene or N-methylpyrrolidone, at a temperature of between about 20 and 200 ° C.

**19.** Process according to one of claims 17 or 18, characterized in that:
a) the S→O derivatives are obtained by oxidizing the -S- compounds, for example with a peracid, it being possible to use metachloroperbenzoic acid, in a solvent such as chloroform or methylene chloride;
b) the ester derivatives of alcohols will be obtained in the conventional manner by esterifying the corresponding alcohols with an acid chloride or an acid anhydride;
c) the ketone derivatives are synthesized by oxidizing the secondary alcohols with known oxidizing agents such as Jones' reagent or Serett's reagent, in an organic solvent such as acetone, chloroform

or methylene chloride; and

d) the halogen derivatives are prepared by reacting thionyl chloride or hydrobromic acid with the corresponding primary alcohols; these derivatives can be used in particular to synthesize amino derivatives by reaction with the corresponding amine, or imidazolyl derivatives by reaction with metallated imidazolyl, or to prepare certain cyano, alkoxy or alkylthio derivatives by reaction with the sodium or potassium salt of the corresponding compound, or to prepare certain amino and amido acids via malonic synthesis by condensation with the previously metallated dialkyl acetamidomalonate, or alternatively via the synthesis of phosphorus compounds by reaction with trialkyl phosphites.

**Claims for the following Contracting States : AT, ES, GR**

**1.** Use of the phenylnaphthyridines carrying functional groups in the 3-position, of the formula :

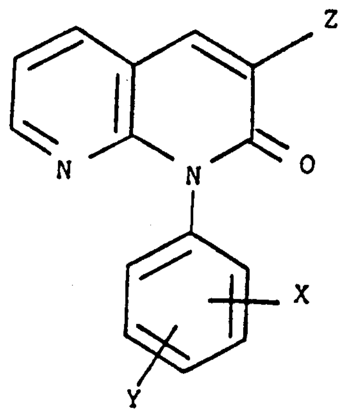

(I)

in which:

X and Y represent the hydrogen atom, a halogen, a lower alkyl group containing 1 to 5 carbon atoms, a trifluoromethyl, an alkoxy, a methylthio, a nitro or a cyano and can be in the ortho, meta or para position on the aromatic ring,

Z represents a functional group selected from:

a) a propenyl or propynyl

b) a chain $-(CH_2)_n-S-(CH_2)_m-R$ or $-(CH_2)_n-SO-(CH_2)_m-R$, in which n end m are identical or different and are numbers between 0 and 5, and R is a methyl or a functional group of the type:

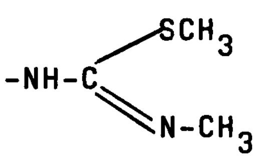

,

of the type

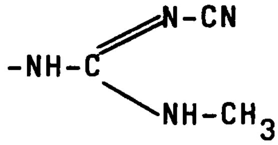

or of the type

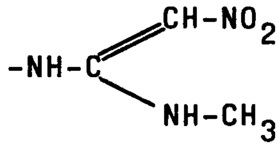
,

c) a chain $-(CH_2)_n-O-(CH_2)_m-CH_3$ (n and m being defined as above)

d) a chain $-(CH_2)_n-OH$ or one of its esters selected from the group consisting in an acetate, propionate, nicotinate, furan-2-carboxylate, and phenylacetate, n being defined as above;

e) a chain $-(CH_2)_n-CHOH-(CH_2)_m-R'$ or one of its esters selected from the group consisting in an acetate, propionate, nicotinate, furan-2-carboxylate, and phenylacetate, n and m being defined as above, R' being a methyl or a phenyl or pyridyl group;

f) a chain $-(CH_2)_n-CO-(CH_2)_m-CH_3$ (n and m being defined as above);

g) a chain $-(CH_2)_n-COOH$ (n being defined as above), n being principally equal to at least 3 and optimally equal to 4;

h) a chain $-(CH_2)_n-Hal$ (n being defined as above and Hal being a halogen);

i) a chain $-(CH_2)_n-CN$ (n being defined as above);

j) a chain

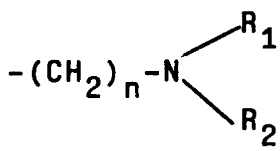

n being defined as above, and $NR_1R_2$ forms a piperazine ring substituted by a methyl group, a phenyl group or a phenyl group substituted by a methoxy or alternatively $NR_1R_2$ forms an imidazole ring;

k) a chain

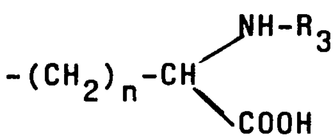

n being defined as above,

$R_3$ being a hydrogen or a parachlorobenzoyl group;

l) a chain

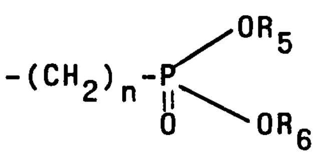

n being defined as above,

$R_5$ and $R_6$ being an ethyl group, and

m) a phenyl, pyridyl, thiophene or furan nucleus, and if appropriate non-toxic acid addition salts, for the preparation of a pharmaceutical composition in particular useful for its ulcer-inhibiting and anti-secretory activity.

**2.** Use according to claim 1, characterized in that X and Y are in the meta or para position.

3. Use according to claim 1 or 2, characterized in that X = Y = H.

4. Use according to claim 1 or 2, characterized in that X = 3-CH and Y = H.

5. Use according to claim 1 or 2, characterized in that X = 4-F and Y = H.

6. Use according to claim 1 or 2, characterized in that X = 3-$NO_2$ and Y = H.

7. Use according to claim 1 or 2, characterized in that X = 3-Cl and Y = H.

8. Use according to claim 1 or 2, characterized in that Z contains an alcohol functional group of formula $-(CH_2)_n$ -OH or an ester thereof.

9. Use according to claim 1 or 2, characterized in that Z = $-CH_2-CHOH-CH_3$ or an ester of this secondary alcohol.

10. Use according to claim 1 or 2, characterized in that Z = $-CH_2-CO-CH_3$.

11. Use according to claim 1 or 2, characterized in that Z = $-(CH_2)_n-CO-(CH_2)_m-CH_3$.

12. Use according to claim 1 or 2, characterized in that Z = a methylthiomethyl.

13. Use according to claim 1 or 2, characterized in that it is selected from the following:

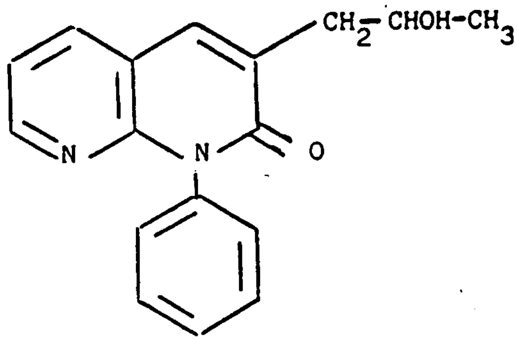

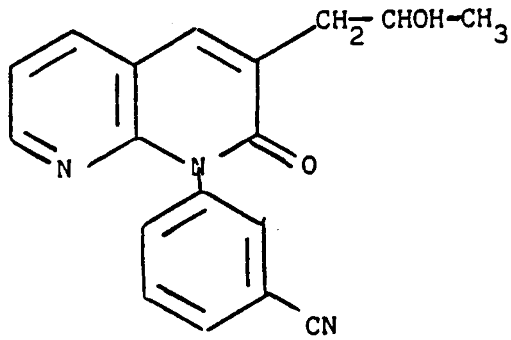

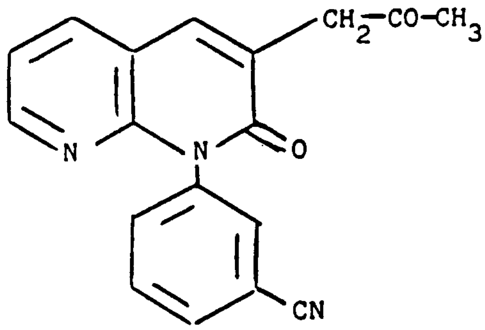

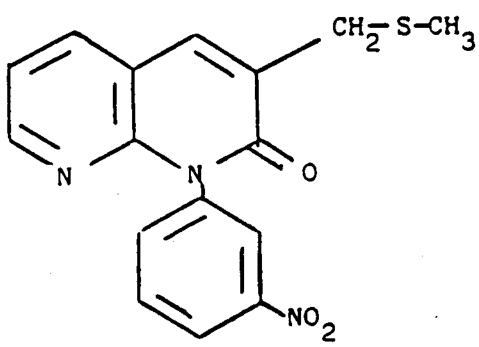

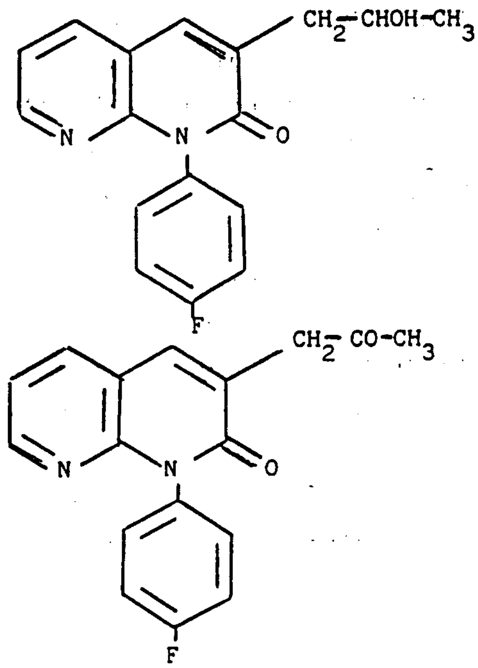

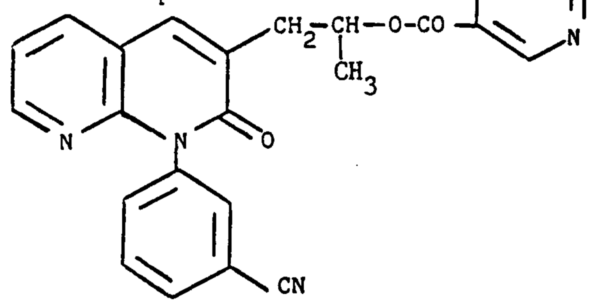

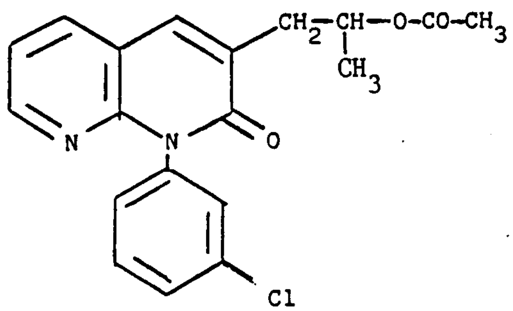

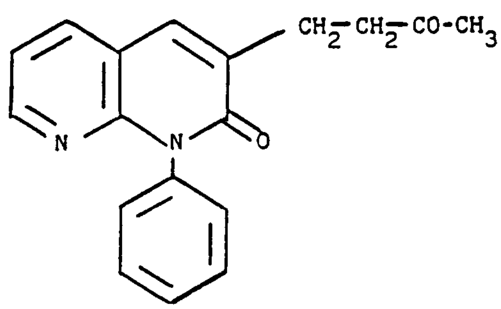

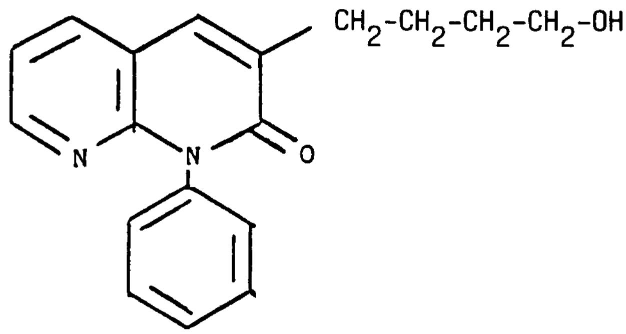

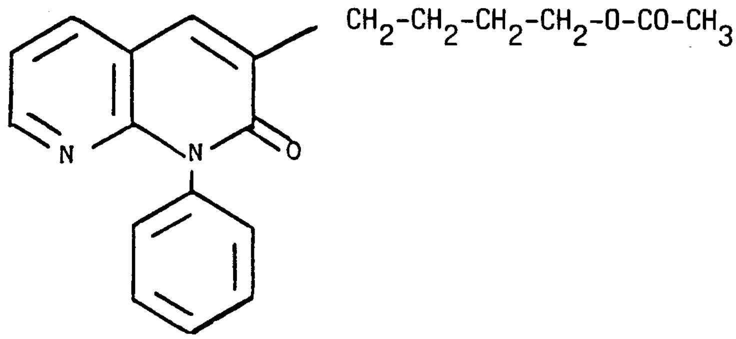

**14.** Process for the preparation of a drug useful in particular for its ulcer-inhibiting and antisecretory activity, characterized in that mixing of at least one compound is performed according to any one of claims 1 to 13 or if appropriate one of its non-toxic acid addition salts, as the active ingredient, with a pharmaceutically acceptable excipient or vehicle.

**15.** Process for the preparation of the compounds of the formula (I) according to any one of claims 1 to 13, characterized in that they are prepared by a method involving a condensation reaction, selected from those of Stobbe, Perkin, Claisen or Knoevenagel, with an aldehyde of the formula (II):

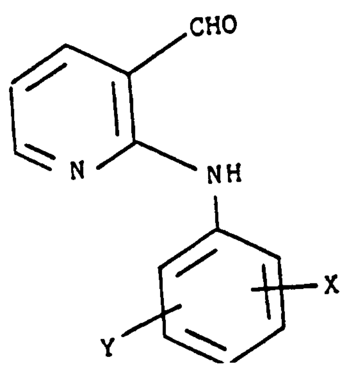

(II)

in the formula (II), X and Y are as defined above.

**16.** Process according to claim 15, characterized in that the aldehydes of the formula (II) are obtained by the oxidation of an alcohol of the formula (III) with a mild oxidizing agent, for example $MnO_2$, in an organic solvent, for example methylene chloride or chloroform, at a temperature of between 20 and 50 ° C:

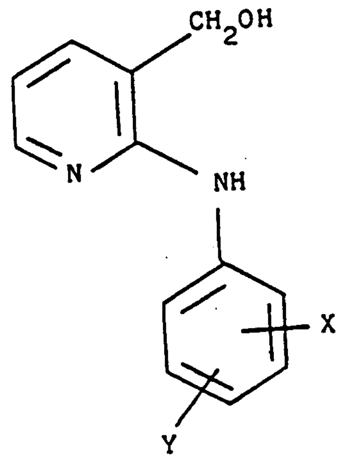

(III)

in the formula (III), X and Y are as defined above.

**17.** Process according to claim 16, characterized in that the alcohols of the formula (III) are obtained by the reduction of an acid or one of its eaters of the formula (IV) with a conventional reducing agent, for example lithium aluminium hydride, in an organic solvent, for example tetrahydrofuran or ethyl ether; in the case where the phenyl nucleus carries a substituent which is sensitive to certain reducing agents, for example a nitro or cyano substituent, the reducing agent for reducing the ester will be chosen so as not to affect this substituent, an example being lithium borohydride prepared "in situ" from potassium borohydride and lithium chloride:

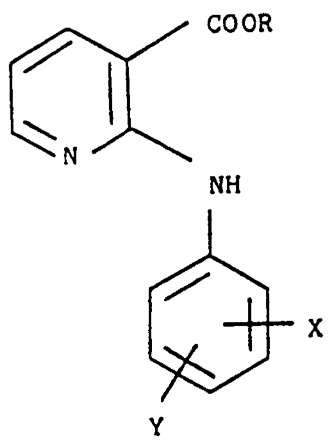

(IV)

in the formula (IV), X and Y are as defined above and R is the hydrogen atom or an alkyl.

**18.** Process for the preparation of the compounds of the formula (I) according to any one of claims 1 to 13, characterized in that reacting the aldehydes of the formula (II), defined in claim 14, is performed with:
a) acid anhydrides of the formula:

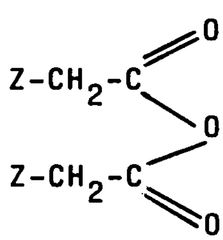

Z being defined as above, in the presence of the sodium salt of the acid Z-$CH_2$-COOH;

b) acid anhydrides of the formula:

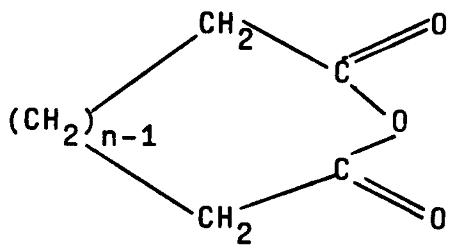

or its chloride

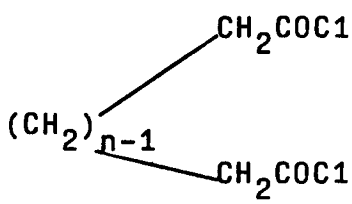

in the presence of the sodium salt of the corresponding acid, n being defined as above;
c) acid eaters of the formula:

Z-$CH_2$-COOR"

Z being defined as above and R" being an alkyl, in the presence of a sodium or potassium alcoholate, sodium hydride or sodium metal; or
d) lactones of the formula:

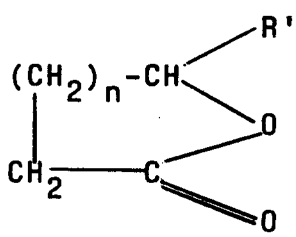

n being defined as above and R' being a methyl or an aromatic or heteroaromatic nucleus, in the presence of a sodium or potassium alcoholate, sodium hydride or sodium metal, and in the presence or absence of an organic solvent such as an alcohol, benzene, toluene or N-methylpyrrolidone, at a temperature of between about 20 and 200 ° C.

**19.** Process according to one of claims 17 or 18, characterized in that:
a) the S--->O derivatives are obtained by oxidizing the -S- compounds, for example with a peracid, it being possible to use metachloroperbenzoic acid, in a solvent such as chloroform or methylene chloride;
b) the ester derivatives of alcohols will be obtained in the conventional manner by esterifying the corresponding alcohols with an acid chloride or an acid anhydride;
c) the ketone derivatives are synthesized by oxidizing the secondary alcohols with known oxidizing agents such as Jones' reagent or Sarett's reagent, in an organic solvent such as acetone, chloroform or methylene chloride; and

d) the halogen derivatives are prepared by reacting thionyl chloride or hydrobromic acid with the corresponding primary alcohols; these derivatives can be used in particular to synthesize amino derivatives by reaction with the corresponding amine, or imidazolyl derivatives by reaction with metallated imidazolyl, or to prepare certain cyano, alkoxy or alkylthio derivatives by reaction with the sodium or potassium salt of the corresponding compound, or to prepare certain amino and amido acids via malonic synthesis by condensation with the previously metallated dialkyl acetamidomalonate, or alternatively via the synthesis of phosphorus compounds by reaction with trialkyl phosphites.

## Patentansprüche

**Patentansprüche für folgende Vertragsstaaten : BE, CH, DE, FR, GB, IT, LI, LU, NL, SE**

**1.** In Stellung 3 funktionalisierte Phenylnaphthyridinverbindungen der Formel

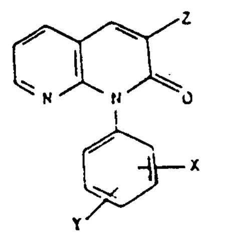

(I)

worin:

- X und Y Wasserstoff, ein Halogen, eine nied.Alkylgruppe mit 1 bis 5 Kohlenstoffatomen, Trifluormethyl, Alkoxy, Methylthio, Nitro oder Cyano darstellen und sich in der ortho-, meta- oder para-Position am aromatischen Ring befinden können,
- Z eine funktionelle Gruppe ist, ausgewählt aus:
  a) Propenyl oder Propinyl,
  b) einer Kette $-(CH_2)_n-S-(CH_2)_m-R$ oder $-(CH_2)_n-SO-(CH_2)_m-R$, worin n und m gleich oder verschieden und Zahlen zwischen 0 und 5 sind und R Methyl ist oder eine Funktion des Typs

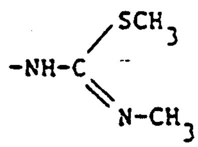

,

des Typs

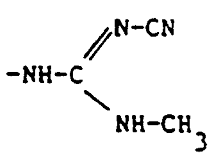

oder des Typs

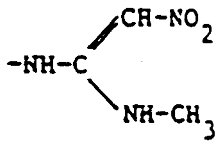

,

c) einer Kette $-(CH_2)_n-O-(CH_2)_m-CH_3$ (worin n und m wie oben definiert sind)
d) einer Kette $-(CH_2)_n-OH$ oder einem ihrer Ester, ausgewählt aus der Gruppe bestehend aus einem Acetat, Propionat, Nicotinat, Furan-2-carboxylat und Phenylacetat worin n wie oben definiert ist;
e) einer Kette $-(CH_2)_n-CHOH-(CH_2)_m-R'$ oder einem ihrer Ester, ausgewählt aus der Gruppe bestehend aus einem Acetat, Propionat, Nicotinat, Furan-2-carboxylat und Phenylacetat, worin n und m wie oben definiert sind und R' Methyl oder eine Phenyl- oder Pyridylgruppe ist;
f) einer Kette $-(CH_2)_n-CO-(CH_2)_m-CH_3$ (worin n und m wie oben definiert sind;
g) einer Kette $-(CH_2)_n-COOH$ (worin n wie oben definiert ist), wobei n insbesondere mindestens gleich 3 und Vorzugsweise 4 ist;
h) einer Kette $-(CH_2)_n-Hal$ (worin n wie oben definiert und Hal ein Halogen ist);
i) einer Kette $-(CH_2)_n-CN$ (worin n wie oben definiert ist);
j) einer Kette

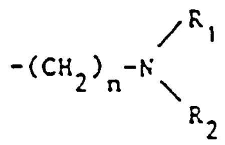

worin n wie oben definiert ist, und $NR_1R_2$ einen Piperazinring bildet, der durch eine Methyl-, Phenyl- oder eine durch Methoxy substituierte Phenylgruppe substituiert ist, oder alternativ $NR_1R_2$ einen Imidazolring bildet;
k) einer Kette

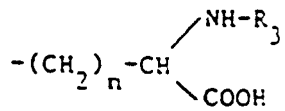

worin n wie oben definiert ist und $R_3$ Wasserstoff oder eine para-Chlorbenzoylgruppe ist;;
l) einer Kette

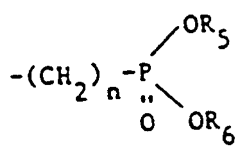

worin n wie oben definiert ist und $R_5$ und $R_6$ eine Ethylgruppe sind; und
m) einem Phenyl-, Pyridyl-, Thiophen- oder Furankern; sowie gegebenenfalls die nicht toxischen Säureadditionssalze.

**2.** Neue Verbindungen nach Anspruch 1, dadurch gekennzeichnet, daß X und Y in der meta- oder para-Position sind.

**3.** Neue Verbindungen nach Anspruch 1 oder 2, dadurch gekennzeichnet, daß X = Y = H.

**4.** Neue Verbindungen nach Anspruch 1 oder 2, dadurch gekennzeichnet, daß X = 3-CN, Y = H.

**5.** Neue Verbindungen nach Anspruch 1 oder 2, dadurch gekennzeichnet, daß X = 4-F, Y = H.

**6.** Neue Verbindungen nach Anspruch 1 oder 2, dadurch gekennzeichnet, daß X = 3-$NO_2$, Y = H.

**7.** Neue Verbindungen nach Anspruch 1 oder 2, dadurch gekennzeichnet, daß X = 3-Cl, Y = H.

**8.** Neue Verbindungen nach Anspruch 1 oder 2, dadurch gekennzeichnet, daß Z eine Alkoholfunktion der Formel $-(CH_2)_n-OH$ oder einen Ester derselben aufweist.

9. Neue Verbindungen nach Anspruch 1 oder 2, dadurch gekennzeichnet, daß Z $-(CH_2)_n-CHOH-CH_3$ oder ein Ester dieses sekundären Alkohols ist.

10. Neue Verbindungen nach Anspruch 1 oder 2, dadurch gekennzeichnet, daß Z = $-CH_2-CO-CH_3$.

11. Neue Verbindungen nach Anspruch 1 oder 2, dadurch gekennzeichnet, daß Z = $-(CH_2)_n-CO-(CH_2)_m-CH_3$.

12. Neue Verbindungen nach Anspruch 1 oder 2, dadurch gekennzeichnet, daß Z = Methylthiomethyl.

13. Neue Verbindungen nach Anspruch 1 oder 2, dadurch gekennzeichnet, daß sie ausgewählt sind aus:

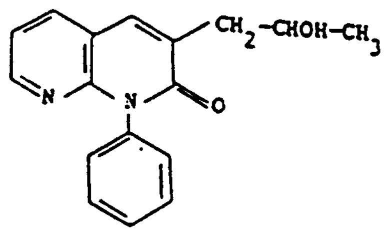

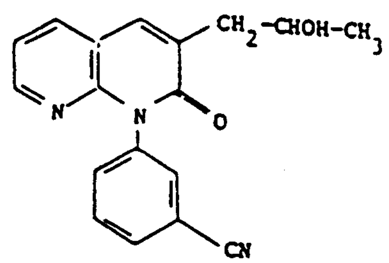

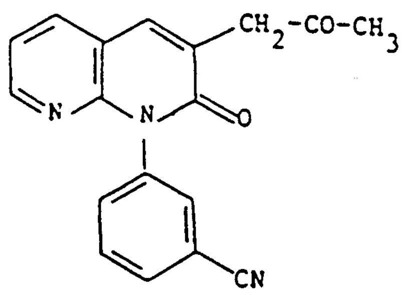

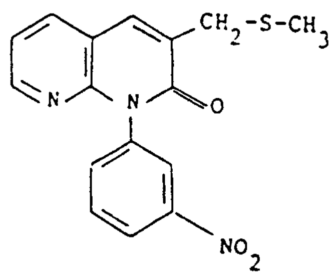

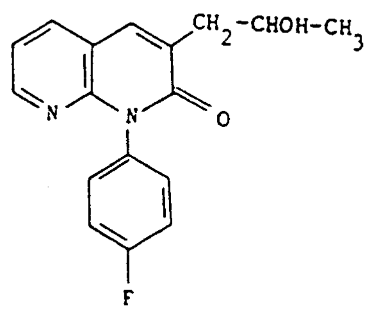

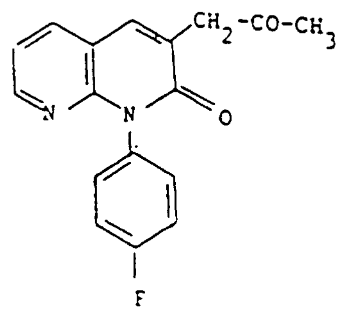

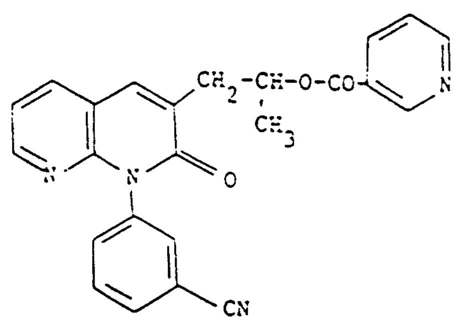

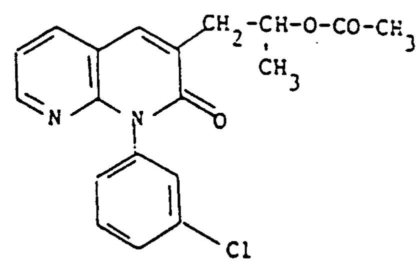

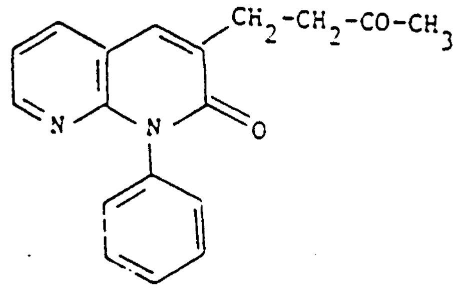

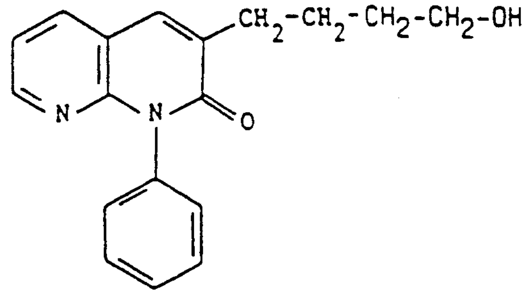

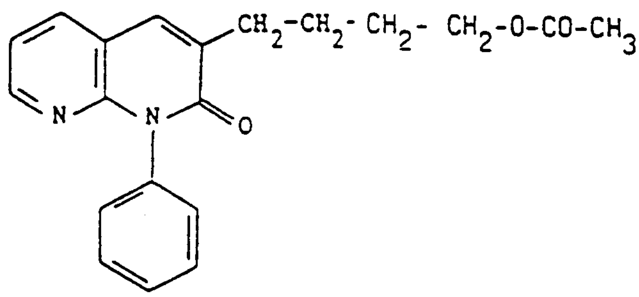

14. Medikamente, die insbesondere wegen ihrer geschwür- und sekretionshemmenden Aktivität nützlich sind, dadurch gekennzeichnet, daß sie mindestens eine Verbindung nach einem der Ansprüche 1 bis 13 oder gegebenenfalls eines ihrer nicht toxischen Säureadditionssalze enthalten.

15. Verfahren zur Herstellung der Verbindungen der Formel (I) nach einem der Ansprüche 1 bis 13, dadurch gekennzeichnet, daß sie durch eine Kondensationsreaktion, ausgewählt aus den Verfahren nach Stobbe, Perkin, Claisen oder Knoevenagel, hergestellt werden , u.zw. mit einem Aldehyd der Formel (II)

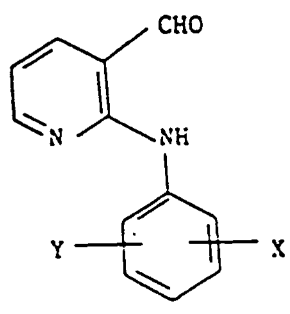

(II)

in welcher Formel (II) X und Y wie zuvor definiert sind.

16. Verfahren nach Anspruch 15, dadurch gekennzeichnet, daß die Aldehyde der Formel (II) durch Oxidation eines Alkohols der Formel (III)

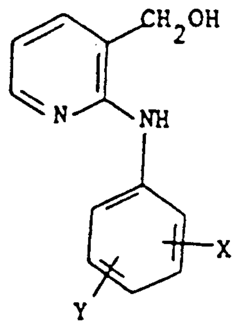

(III)

in welcher Formel (III) X und Y wie zuvor definiert sind, mit einem milden Oxidationsmittel, z.B. $MnO_2$, in einem organischen Lösungsmittel, z.B. Dichlormethan oder Chloroform, bei einer Temperatur zwischen 20 und 50 ° C erhalten werden.

**17.** Verfahren nach Anspruch 16, dadurch gekennzeichnet, daß die Alkohole der Formel (III) durch Reduktion einer Säure oder eines ihrer Ester der Formel (IV)

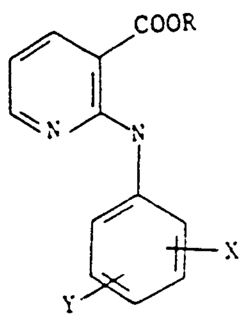

(IV)

in welcher Formel (IV) X und Y wie zuvor definiert sind und R das Wasserstoffatom oder ein Alkyl ist, mit einem klassischen Reduktionsmittel, z.B. Lithiumaluminiumhydrid, in einem organischen Lösungsmittel, z.B. Tetrahydrofuran oder Ethylether, erhalten werden; für den Fall, daß der Phenylkern Träger eines für gewisse Reduktionsmittel empfindlichen Substituenten, wie z.B. Nitro oder Cyano, ist, wählt man die Reduktion des Esters mit einem Reduktionsmittel, das diesem Substituenten Rechnung trägt, z.B. "in situ" aus Kaliumborhydrid und Lithiumchlorid hergestelltem Lithiumborhydrid.

**18.** Verfahren zur Herstellung der Verbindungen der Formel (I) nach einem der Ansprüche 1 bis 13, dadurch gekennzeichnet, daß die Aldehyde der im Anspruch 14 definierten Formel II mit

a) Säureanhydriden der Formel

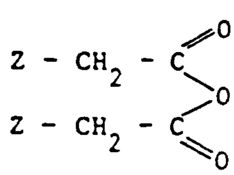

worin Z wie oben definiert ist, in Gegenwart des Natriumsalzes der Säure $Z\text{-}CH_2\text{-}COOH$,

b) Säureanhydriden der Formel

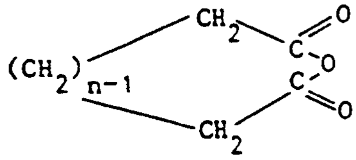

oder deren Chlorid

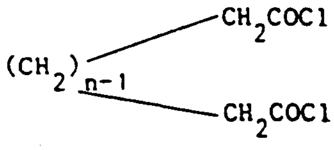

in Gegenwart des Natriumsalzes der entsprechenden Säure, worin n wie oben definiert ist,
c) Estern von Säuren der Formel

Z - $CH_2$ - COOR"

worin Z wie oben definiert ist und R" Alkyl ist, in Gegenwart eines Natrium- oder Kaliumalkoholats oder Natriumhydrids oder Natriummetalls, oder
d) Lactonen der Formel

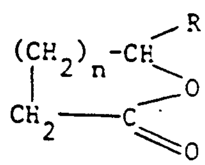

worin n wie oben definiert ist und R' Methyl oder ein aromatischer oder heteroaromatischer Kern ist, in Gegenwart eines Natrium- oder Kaliumalkoholats oder eines Natriumhydrids oder Natriummetalls,
in Gegenwart oder in Abwesenheit eines organischen Lösungsmittels, wie eines Alkohols, Benzol, Toluol oder N-Methylpyrrolidon, und bei einer Temperatur zwischen etwa 20 und 200 °C reagieren gelassen werden.

**19.** Verfahren nach einem der Ansprüche 17 oder 18, dadurch gekennzeichnet, daß
a) die Derivate S→O durch Oxidation von Verbindungen -S- mit z.B. einer Persäure, wobei Metachlorperbenzoesäure verwendet werden kann, in einem Lösungsmittel, wie Chloroform oder Dichlormethan erhalten werden;
b) die Esterderivate des Alkohols auf klassische Weise durch Verestern der entsprechenden Alkohole mit einem Säurechlorid oder einem Säureanhydrid erhalten werden;
c) die Ketonderivate durch Oxidation der sekundären Alkohole mit bekannten Oxidationsmitteln, wie dem Jones-Reagens oder dem Sarett-Reagens, in einem organischen Lösungsmittel, wie Aceton, Chloroform oder Dichlormethan, synthetisiert werden;
d) die Halogenderivate durch Umsetzung von Thionylchlorid oder von Bromwasserstoffsäure mit den entsprechenden primären Alkoholen hergestellt werden, welche Derivate insbesondere verwendet werden können zur Synthese von Aminoderivaten durch Umsetzung des entsprechenden Amins und von Imidazolyl durch Umsetzung des Metallimidazols, zur Herstellung bestimmter Cyano- Alkoxy- oder Alkylthioderivate durch Umsetzung des Natrium- oder Kaliumsalzes der entsprechenden Verbindung und zur Herstellung bestimmter Amino- und Amidosäuren durch Malonsäuresynthese unter Kondensieren des zuvor metallisierten Dialkylacetamidomalonats oder auch durch Synthese von Phosphorverbindungen durch Umsetzung mit Trialkylphosphiten.

**Patentansprüche für folgende Vertragsstaaten : AT, ES, GR**

**1.** Verwendung der in Stellung 3 funktionalisierten Phenylnaphthyridine der Formel

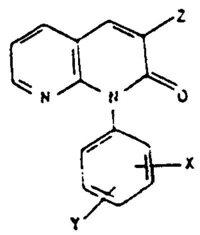

(I)

worin:

- X und Y Wasserstoff, ein Halogen, eine nied.Alkylgruppe mit 1 bis 5 Kohlenstoffatomen, Trifluormethyl, Alkoxy, Methylthio, Nitro oder Cyano darstellen und sich in der ortho-, meta- oder para-Position am aromatischen Ring befinden können,
- Z eine funktionelle Gruppe ist, ausgewählt aus:

a) Propenyl oder Propinyl,

b) einer Kette $-(CH_2)_n-S-(CH_2)_m-R$ oder $-(CH_2)_n-SO-(CH_2)_m-R$, worin n und m gleich oder verschieden und Zahlen zwischen 0 und 5 sind und R Methyl ist oder eine Funktion des Typs

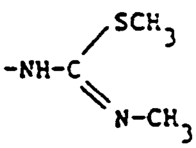

,

des Typs oder

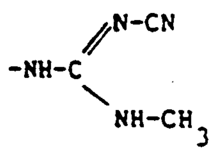

des Typs

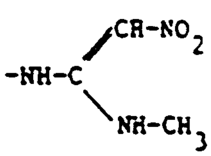

,

c) einer Kette $-(CH_2)_n-O-(CH_2)_m-CH_3$ (worin n und m wie oben definiert sind)

d) einer Kette $-(CH_2)_n-OH$ oder einem ihrer Ester, ausgewählt aus der Gruppe bestehend aus einem Acetat, Propionat, Nicotinat, Furan-2-carboxylat und Phenylacetat, worin n wie oben definiert ist;

e) einer Kette $-(CH_2)_n-CHOH-(CH_2)_m-R'$ oder einem ihrer Ester, ausgewählt aus der Gruppe bestehend aus einem Acetat, Propionat, Nicotinat, Furan-2-carboxylat und Phenylacetat, worin n und m wie oben definiert sind und R' Methyl oder eine Phenyl- oder Pyridylgruppe ist;

f) einer Kette $-(CH_2)_n-CO-(CH_2)_m-CH_3$ (worin n und m wie oben definiert sind;

g) einer Kette $-(CH_2)_n-COOH$ (worin n wie oben definiert ist), wobei n insbesondere mindestens gleich 3 und vorzugsweise 4 ist;

h) einer Kette $-(CH_2)_n-Hal$ (worin n wie oben definiert und Hal ein Halogen ist);

i) einer Kette $-(CH_2)_n-CN$ (worin n wie oben definiert ist);

j) einer Kette

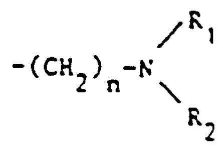

worin n wie oben definiert ist, und $NR_1R_2$ einen Piperazinring bildet, der durch eine Methyl-, Phenyl oder eine durch Methoxy substituierte Phenylgruppe substituiert ist, oder alternativ $NR_1R_2$ einen Imidazolring bildet;
k) einer Kette

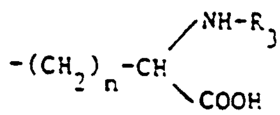

worin n wie oben definiert ist und $R_3$ Wasserstoff oder eine para-Chlorbenzoylgruppe ist;;
l) einer Kette

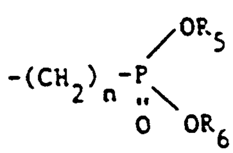

worin n wie oben definiert ist und $R_5$ und $R_6$ eine Ethylgruppe sind; und
m) einem Phenyl-, Pyridyl-, Thiophen- oder Furankern; sowie gegebenenfalls der nicht toxischen Säureadditionssalze zur Herstellung einer Pharmazeutischen Zusammensetzung, die insbesondere wegen ihrer geschwür- und sekretionshemmenden Aktivität nützlich ist.

**2.** Verwendung nach Anspruch 1, dadurch gekennzeichnet, daß X und Y in der meta- oder para-Position sind.

**3.** Verwendung nach Anspruch 1 oder 2, dadurch gekennzeichnet, daß X = Y = H.

**4.** Verwendung nach Anspruch 1 oder 2, dadurch gekennzeichnet, daß X = 3-CH, Y = H.

**5.** Verwendung nach Anspruch 1 oder 2, dadurch gekennzeichnet, daß X = 4-F, Y = H.

**6.** Verwendung nach Anspruch 1 oder 2, dadurch gekennzeichnet, daß X = 3-$NO_2$, Y = H.

**7.** Verwendung nach Anspruch 1 oder 2, dadurch gekennzeichnet, daß X = 3-Cl, Y = H.

**8.** Verwendung nach Anspruch 1 oder 2, dadurch gekennzeichnet, daß Z eine Alkoholfunktion der Formel $-(CH_2)_n-OH$ oder einen Ester derselben aufweist.

**9.** Verwendung nach Anspruch 1 oder 2, dadurch gekennzeichnet, daß Z $-(CH_2)_n-CHOH-CH_3$ oder ein Ester dieses sekundären Alkohols ist.

**10.** Verwendung nach Anspruch 1 oder 2, dadurch gekennzeichnet, daß Z = $-CH_2-CO-CH_3$.

**11.** Verwendung nach Anspruch 1 oder 2, dadurch gekennzeichnet, daß Z = $-(CH_2)_n-CO-(CH_2)_m-CH_3$.

**12.** Verwendung nach Anspruch 1 oder 2, dadurch gekennzeichnet, daß Z = Methylthiomethyl.

**13.** Verwendung nach Anspruch 1 oder 2, dadurch gekennzeichnet, daß sie ausgewählt sind aus:

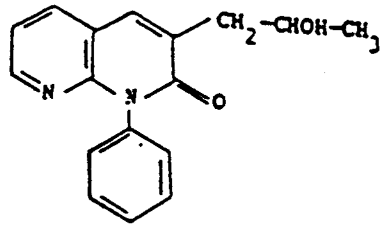

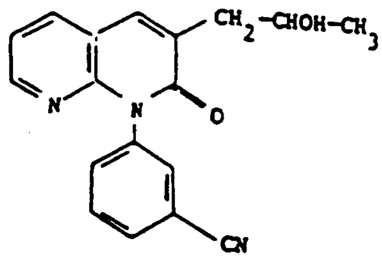

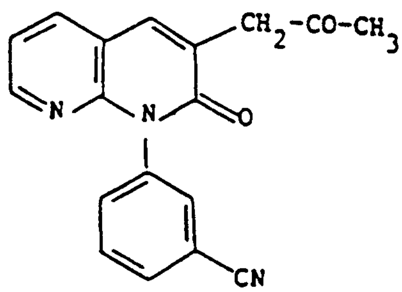

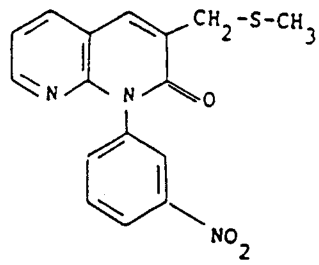

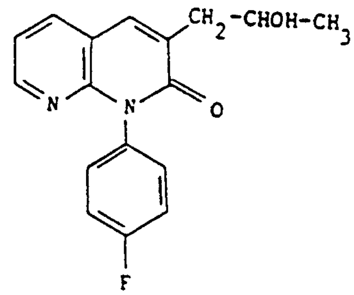

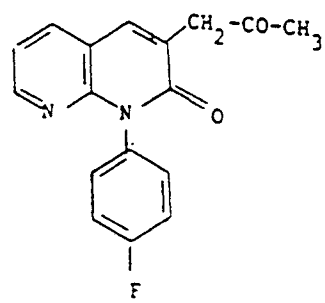

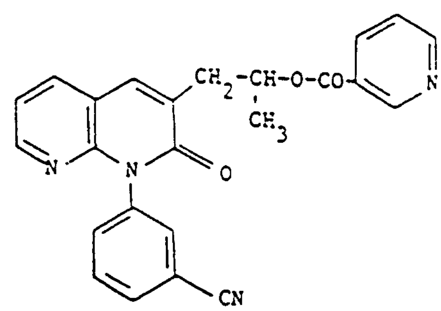

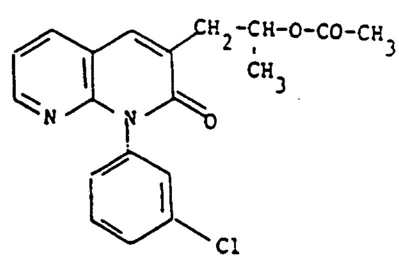

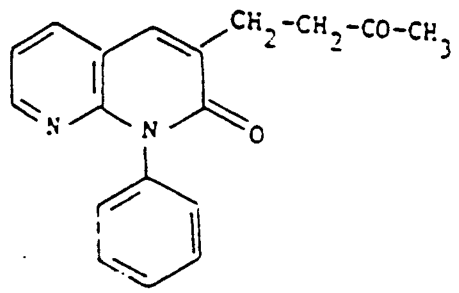

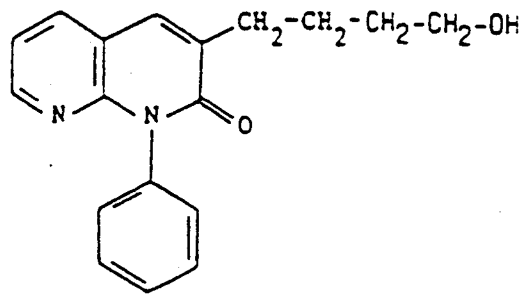

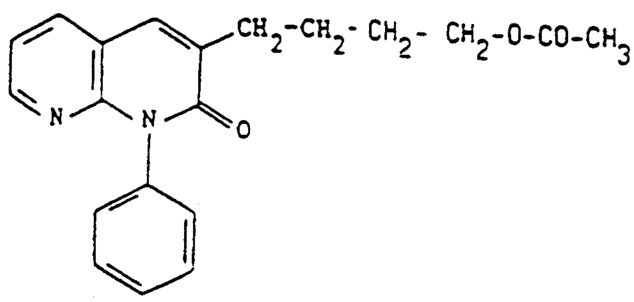

**14.** Verfahren zur Herstellung eines Medikaments, das insbesondere wegen seiner geschwür- und sekretionshemmenden Aktivität nützlich ist, dadurch gekennzeichnet, daß als aktives Ingrediens mindestens eine Verbindung nach einem der Ansprüche 1 bis 13 oder gegebenenfalls eines ihrer nicht toxischen Säureadditionssalze mit einem pharmazeutisch akzeptablen Exzipienten oder Träger vermischt wird.

**15.** Verfahren zur Herstellung der Verbindungen der Formel (I) nach einem der Ansprüche 1 bis 13, dadurch gekennzeichnet, daß sie durch eine Kondensationsreaktion, ausgewählt aus den Verfahren nach Stobbe, Perkin, Claisen oder Knoevenagel, hergestellt werden , u.zw. mit einem Aldehyd der Formel (II)

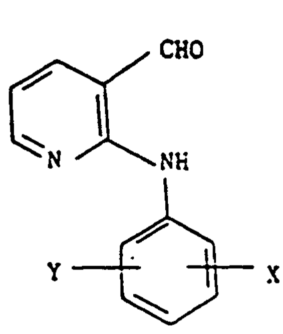

(II)

in welcher Formel (II) X und Y wie zuvor definiert sind.

**16.** Verfahren nach Anspruch 15, dadurch gekennzeichnet, daß die Aldehyde der Formel (II) durch

Oxidation eines Alkohols der Formel (III)

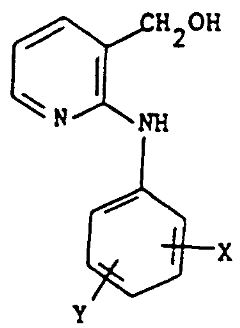

(III)

in welcher Formel (III) X und Y wie zuvor definiert sind, mit einem milden Oxidationsmittel, z.B. $MnO_2$, in einem organischen Lösungsmittel, z.B. Dichlormethan oder Chloroform, bei einer Temperatur zwischen 20 und 50 ° C erhalten werden.

**17.** Verfahren nach Anspruch 16, dadurch gekennzeichnet, daß die Alkohole der Formel (III) durch Reduktion einer Säure oder eines ihrer Ester der Formel (IV)

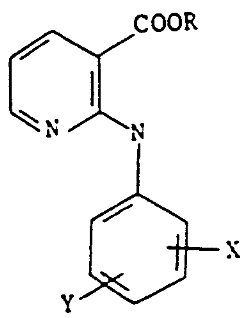

(IV)

in welcher Formel (IV) X und Y wie zuvor definiert sind und R das Wasserstoffatom oder ein Alkyl ist, mit einem klassischen Reduktionsmittel, z.B. Lithiumaluminiumhydrid, in einem organischen Lösungsmittel, z.B. Tetrahydrofuran oder Ethylether, erhalten werden; für den Fall, daß der Phenylkern Träger eines für gewisse Reduktionsmittel empfindlichen Substituenten, wie z.B. Nitro oder Cyano, ist, wählt man die Reduktion des Esters mit einem Reduktionsmittel, das diesem Substituenten Rechnung trägt, z.B. "in situ" aus Kaliumborhydrid und Lithiumchlorid hergestelltem Lithiumborhydrid.

**18.** Verfahren zur Herstellung der Verbindungen der Formel (I) nach einem der Ansprüche 1 bis 13, dadurch gekennzeichnet, daß die Aldehyde der im Anspruch 14 definierten Formel II mit

a) Säureanhydriden der Formel

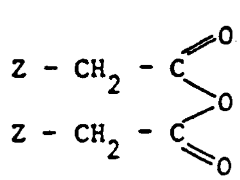

worin Z wie oben definiert ist, in Gegenwart des Natriumsalzes der Säure $Z\text{-}CH_2\text{-}COOH$,

b) Säureanhydriden der Formel

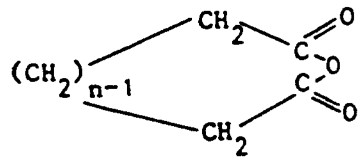

oder deren Chlorid

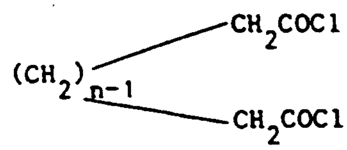

in Gegenwart des Natriumsalzes der entsprechenden Säure, worin n wie oben definiert ist,
c) Estern von Säuren der Formel

Z - $CH_2$ - COOR"

worin Z wie oben definiert ist und R" Alkyl ist, in Gegenwart eines Natrium- oder Kaliumalkoholats oder Natriumhydrids oder Natriummetalls, oder
d) Lactonen der Formel

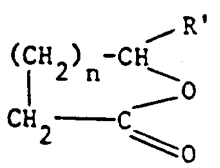

worin n wie oben definiert ist und R' Methyl oder ein aromatischer oder heteroaromatischer Kern ist, in Gegenwart eines Natrium- oder Kaliumalkoholats oder eines Natriumhydrids oder Natriummetalls,
in Gegenwart oder in Abwesenheit eines organischen Lösungsmittels, wie eines Alkohols, Benzol, Toluol oder N-Methylpyrrolidon, und bei einer Temperatur zwischen etwa 20 und 200°C reagieren gelassen werden.

**19.** Verfahren nach einem der Ansprüche 17 oder 18, dadurch gekennzeichnet, daß
a) die Derivate S→O durch Oxidation von Verbindungen -S- mit z.B. einer Persäure, wobei Metachlorperbenzoesäure verwendet werden kann, in einem Lösungsmittel, wie Chloroform oder Dichlormethan erhalten werden;
b) die Esterderivate des Alkohols auf klassische Weise durch Verestern der entsprechenden Alkohole mit einem Säurechlorid oder einem Säureanhydrid erhalten werden;
c) die Ketonderivate durch Oxidation der sekundären Alkohole mit bekannten Oxidationsmitteln, wie dem Jones-Reagens oder dem Sarett-Reagens, in einem organischen Lösungsmittel, wie Aceton, Chloroform oder Dichlormethan, synthetisiert werden;
d) die Halogenderivate durch Umsetzung von Thionylchlorid oder von Bromwasserstoffsäure mit den entsprechenden primären Alkoholen hergestellt werden, welche Derivate insbesondere verwendet werden können zur Synthese von Aminoderivaten durch Umsetzung des entsprechenden Amins und von Imidazolyl durch Umsetzung des Metallimidazols, zur Herstellung bestimmter Cyano- Alkoxy- oder Alkylthioderivate durch Umsetzung des Natrium- oder Kaliumsalzes der entsprechenden Verbindung und zur Herstellung bestimmter Amino- und Amidosäuren durch Malonsäuresynthese unter Kondensieren des zuvor metallisierten Dialkylacetamidomalonats oder auch durch Synthese von Phosphorverbindungen durch Umsetzung mit Trialkylphosphiten.